(19) Europäisches Patentamt
European Patent Office
Office européen des brevets

(11) EP 2 066 628 B1

# (12) EUROPEAN PATENT SPECIFICATION

(45) Date of publication and mention of the grant of the patent:
**20.10.2010 Bulletin 2010/42**

(21) Application number: **07799729.4**

(22) Date of filing: **20.07.2007**

(51) Int Cl.:
***C07D 209/18*** *(2006.01)* ***A61K 31/404*** *(2006.01)*
***A61P 11/06*** *(2006.01)* ***A61P 27/14*** *(2006.01)*
***A61P 37/06*** *(2006.01)* ***A61P 37/08*** *(2006.01)*

(86) International application number:
**PCT/US2007/073945**

(87) International publication number:
**WO 2008/014186 (31.01.2008 Gazette 2008/05)**

## (54) 2-PHENYL-INDOLES AS PROSTAGLANDIN D2 RECEPTOR ANTAGONISTS

2-PHENYLINDOLE ALS ANTAGONISTEN DES PROSTAGLANDIN-D2-REZEPTORS

2-PHÉNYL-INDOLES EN TANT QU'ANTAGONISTES DU RÉCEPTEUR DE LA PROSTAGLANDINE D2

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **25.07.2006 US 820301 P**
**25.07.2006 US 820302 P**
**25.07.2006 US 820299 P**

(43) Date of publication of application:
**10.06.2009 Bulletin 2009/24**

(73) Proprietor: **Sanofi-Aventis**
**75013 Paris (FR)**

(72) Inventors:
- **YANG, Zhaoxia**
**Bridgewater, New Jersey 08807 (US)**
- **REILING, Stephan**
**Bridgewater, New Jersey 08807 (US)**
- **NIEDUZAK, Thaddeus R.**
**Bridgewater, New Jersey 08807 (US)**
- **MATHEW, Rose M.**
**Bridgewater, New Jersey 08807 (US)**
- **JACKSON, Sharon**
**Bridgewater, New Jersey 08807 (US)**
- **HARRIS, Keith J.**
**Bridgewater, New Jersey 08807 (US)**

(74) Representative: **Ori, Janos**
**Chinoin Gyogyszer és Vegyészeti Termékek Gyara Zrt.**
**To u. 1-5.**
**H-1045 Budapest (HU)**

(56) References cited:
**WO-A-2006/081343**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention is directed to 2-phenyl-indole compounds, their preparation, pharmaceutical compositions containing these compounds, and their pharmaceutical use in the treatment of disease states capable of being modulated by the inhibition of the prostaglandin D2 receptor.

### BACKGROUND OF THE INVENTION

Local allergen challenge in patients with allergic rhinitis, bronchial asthma, allergic conjunctivitis and atopic dermatitis has been shown to result in rapid elevation of prostaglandin D2 "(PGD2)" levels in nasal and bronchial lavage fluids, tears and skin chamber fluids. PGD2 has many inflammatory actions, such as increasing vascular permeability in the conjunctiva and skin, increasing nasal airway resistance, airway narrowing and eosinophil infiltration into the conjunctiva and trachea.

PGD2 is the major cyclooxygenase product of arachidonic acid produced from mast cells on immunological challenge [Lewis, RA, Soter NA, Diamond PT, Austen KF, Oates JA, Roberts LJ II, prostaglandin D2 generation after activation of rat and human mast cells with anti-IgE, J.Immunol. 129, 1627-1631, 1982]. Activated mast cells, a major source of PGD2, are one of the key players in driving the allergic response in conditions such as asthma, allergic rhinitis, allergic conjunctivitis, allergic dermatitis and other diseases [Brightling CE, Bradding P, Pavord ID, Wardlaw AJ, New Insights into the role of the mast cell in asthma, Clin Exp Allergy 33, 550-556, 2003].

Many of the actions of PGD2 are mediated through its action on the D-type prostaglandin ("DP") receptor, a G protein-coupled receptor expressed on epithelium and smooth muscle.

In asthma, the respiratory epithelium has long been recognized as a key source of inflammatory cytokines and chemokines that drive the progression of the disease [Holgate S, Lackie P, Wilson S, Roche W, Davies D, Bronchial Epithelium as a Key Regulator of Airway Allergen Sensitzation and Remodeling in Asthma, Am J Respir Crit Care Med. 162, 113-117, 2000]. In an experimental murine model of asthma, the DP receptor is dramatically up-regulated on airway epithelium on antigen challenge [Matsuoka T, Hirata M, Tanaka H, Takahashi Y, Murata T, Kabashima K, Sugimoto Y, Kobayashi T, Ushikubi F, Aze Y, Eguchi N, Urade Y, Yoshida N, Kimura K, Mizoguchi A, Honda Y, Nagai H, Narumiya S, Prostaglandin D2 as a mediator of allergic asthma, Science 287, 2013-2017, 2000]. In knockout mice, lacking the DP receptor, there is a marked reduction in airway hypereactivity and chronic inflammation [Matsuoka T, Hirata M, Tanaka H, Takahashi Y, Murata T, Kabashima K, Sugimoto Y, Kobayashi T, Ushikubi F, Aze Y, Eguchi N, Urade Y, Yoshida N, Kimura K, Mizoguchi A, Honda Y, Nagai H, Narumiya S, Prostaglandin D2 as a mediator of allergic asthma, Science 287, 2013-2017, 2000]; two of the cardinal features of human asthma.

The DP receptor is also thought to be involved in human allergic rhinitis, a frequent allergic disease that is characterized by the symptoms of sneezing, itching, rhinorea and nasal congestion. Local administration of PGD2 to the nose causes a dose dependent increase in nasal congestion [Doyle WJ, Bochm S, Skoner DP, Physiologic responses to intranasal dose-response challenges with histamine, methacholine, bradykinin, and prostaglandin in adult volunteers with and without nasal allergy, J Allergy Clin Immunol. 86(6 Pt 1), 924-35, 1990].

DP receptor antagonists have been shown to reduce airway inflammation in a guinea pig experimental asthma model [Arimura A, Yasui K, Kishino J, Asanuma F, Hasegawa H, Kakudo S, Ohtani M, Arita H (2001), Prevention of allergic inflammation by a novel prostaglandin receptor antagonist, S-5751, J Pharmacol Exp Ther. 298(2), 411-9, 2001]. PGD2, therefore, appears to act on the DP receptor and plays an important role in elicitation of certain key features of allergic asthma.

DP antagonists have been shown to be effective at alleviating the symptoms of allergic rhinitis in multiple species, and more specifically have been shown to inhibit the antigen-induced nasal congestion, the most manifest symptom of allergic rhinitis [Jones, T. R., Savoie, C., Robichaud, A., Sturino, C., Scheigetz, J., Lachance, N., Roy, B., Boyd, M., Abraham, W., Studies with a DP receptor antagonist in sheep and guinea pig models of allergic rhinitis, Am. J. Resp. Crit. Care Med. 167, A218, 2003; and Arimura A, Yasui K, Kishino J, Asanuma F, Hasegawa H, Kakudo S, Ohtani M, Arita H Prevention of allergic inflammation by a novel prostaglandin receptor antagonist, S-5751. J Pharmacol Exp Ther. 298(2), 411-9, 2001].

DP antagonists are also effective in experimental models of allergic conjunctivitis and allergic dermatitis [Arimura A, Yasui K, Kishino J, Asanuma F, Hasegawa H, Kakudo S, Ohtani M, Arita H, Prevention of allergic inflammation by a novel prostaglandin receptor antagonist, S-5751. J Pharmacol Exp Ther. 298(2), 411-9, 2001; and Torisu K, Kobayashi K, Iwahashi M, Nakai Y, Onoda T, Nagase T, Sugimoto I, Okada Y, Matsumoto R, Nanbu F, Ohuchida S, Nakai H, Toda M, Discovery of a new class of potent, selective, and orally active prostaglandin D2 receptor antagonists, Bioorg. & Med Chem. 12, 5361-5378, 2004]. WO2006/081343 discloses 2-phenyl-indole derivatives, their preparation, pharmaceutical compositions containing these compounds and their pharmaceutical use in treatment of disease states capable of being

modulated by the inhibition of the prostaglandin D2 receptor.

## SUMMARY OF THE INVENTION

The present invention is direct to a compound of formula (A),

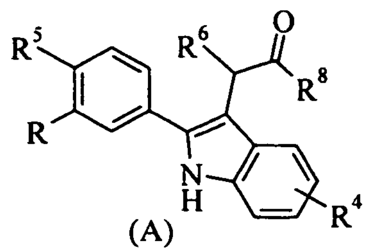

wherein:

| | |
|---|---|
| R is | $R^1CH_2SO_2$-, $R^2CH_2SO_2NH$-, or $R^3NHSO_2$-, |
| $R^1$ is | phenyl optionally substituted with halo, |
| $R^2$ is | phenyl substituted with halo, |
| $R^3$ is | 2,6-dichloro-benzyl, 3,5-dichloro-benzyl, 2,4-dichloro-phenylethyl, 2-methoxy- phenylethyl, 3-methoxy-phenylethyl, 4-methoxy-phenylethyl, 2-trifluoromethyl- phenylethyl, phenylethyl or 3-phenyl-n-propyl, |
| $R^4$ is | hydrogen, |
| $R^5$ is | chloro, |
| $R^6$ is | hydrogen and |
| $R^8$ is | hydroxy; or |

| | |
|---|---|
| R is | cyclohexylaminosulfonyl, |
| $R^4$ is | 4-chloro, 4-fluoro, 4-methyl or 7-chloro, |
| $R^5$ is | chloro or ethyl, |
| $R^6$ is | hydrogen or methyl, and |
| $R^8$ is | hydroxy; or |

| | |
|---|---|
| R is | cyclohexylaminosulfonyl, |
| $R^4$ is | hydrogen, |
| $R^5$ is | chloro, |
| $R^6$ is | hydrogen, |
| $R^8$ is | -$NHR^7$, and |
| $R^7$ is | methyl, methylsulfonyl, ethylsulfonyl, haloalkylsulfonyl or tetrazolyl; |

or a pharmaceutically acceptable salt, hydrate, or solvate thereof, a pharmaceutically acceptable ester prodrug thereof, or a pharmaceutically acceptable salt, hydrate or solvate of the ester prodrug.

Another aspect of the present invention is a pharmaceutical composition comprising, a pharmaceutically effective amount of one or more compounds of the invention, or a pharmaceutically acceptable salt, hydrate, or solvate thereof, a pharmaceutically acceptable ester prodrug thereof, or a pharmaceutically acceptable salt, hydrate or solvate of the ester prodrug, in admixture with a pharmaceutically acceptable carrier.

## DETAILED DESCRIPTION OF THE INVENTION

Definition of the Terms

As used above, and throughout the description of the invention, the following terms, unless otherwise indicated, shall be understood to have the following meanings:

"Compounds of the present invention", and equivalent expressions, are meant to embrace compounds of Formulae (A), (I), (II) or (III) as hereinbefore described, which expression includes the prodrugs, the pharmaceutically acceptable salts, and the solvates, e.g., hydrates, where the context so permits. Similarly, reference to intermediates,

whether or not they themselves are claimed, is meant to embrace their salts, and solvates, where the context so permits.

"Haloalkyl" means alkyl substituted by one to three halo groups. Particular haloalkyl are loweralkyl substituted by one to three halogens. Most particular haloalkyl are loweralkyl substituted by one halogen.

"Haloalkylsulfonyl" means haloalkyl-$SO_2$-. Example includes $CF_3$-$SO_2$-.

"Patient" includes human and other mammals.

"Pharmaceutically acceptable prodrugs" as used herein refers to those prodrugs of the compounds of the present invention which are, within the scope of sound medical judgment, suitable for use in contact with the tissues of patients with undue toxicity, irritation, allergic response commensurate with a reasonable benefit/risk ratio, and effective for their intended use of the compounds of the invention. The term "prodrug" means a compound that is transformed *in vivo* to yield a compound of the invention or a pharmaceutically acceptable salt, hydrate or solvate of the compound. The transformation may occur by various mechanisms, such as through hydrolysis in blood. The compounds bearing metabolically cleavable groups have the advantage that they may exhibit improved bioavailability as a result of enhanced solubility and/or rate of absorption conferred upon the parent compound by virtue of the presence of the metabolically cleavable group, thus, such compounds act as pro-drugs. A thorough discussion is provided in Design of Prodrugs, H. Bundgaard, ed., Elsevier (1985); Methods in Enzymology; K. Widder et al, Ed., Academic Press, 42, 309-396 (1985); A Textbook of Drug Design and Development, Krogsgaard-Larsen and H. Bandgaard, ed., Chapter 5; "Design and Applications of Prodrugs" 113-191 (1991); Advanced Drug Delivery Reviews, H. Bundgaard, 8, 1-38, (1992); J. Pharm. Sci., 77, 285 (1988); Chem. Pharm. Bull., N. Nakeya et al, 32, 692 (1984); Pro-drugs as Novel Delivery Systems, T. Higuchi and V. Stella, 14 A.C.S. Symposium Series, and Bioreversible Carriers in Drug Design, E.B. Roche, ed., American Pharmaceutical Association and Pergamon Press, 1987; J. Med. Chem., Vol. 47, No. 10, 1-12 (2004), which are incorporated herein by reference.

The prodrugs of a compound of the invention are ester prodrugs. "Ester prodrug" means a compound that is convertible *in vivo* by metabolic means (e.g., by hydrolysis) to a compound of the invention. For example, an ester prodrug of a compound of the invention containing a carboxy group may be convertible by hydrolysis *in vivo* to the corresponding compound of the invention, such as methyl ester prodrug, ethyl ester prodrug or 2-dimethylamino-ethyl ester prodrug.

"Pharmaceutically acceptable salts" refers to the non-toxic, inorganic and organic acid addition salts, and base addition salts, of compounds of the present invention. These salts can be prepared *in situ* during the final isolation and purification of the compounds.

"Pharmaceutically effective amount" means an amount of compound, or compounds, according to the present invention effective that produces the desired therapeutic effect described herein, such as allergy relieving, or inflammatory relieving effect.

"Solvate" means a physical association of a compound of this invention with one or more solvent molecules. This physical association includes hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include hydrates, ethanolates and methanolates.

Some of the compounds of the present invention are basic, and such compounds are useful in the form of the free base, or in the form of a pharmaceutically acceptable acid addition salt thereof.

Acid addition salts are a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the patient in pharmaceutical doses of the salts, so that the beneficial inhibitory effects inherent in the free base are not vitiated by side effects ascribable to the anions. Although pharmaceutically acceptable salts of said basic compounds are preferred, all acid addition salts are useful as sources of the free base form even if the particular salt, *per se*, is desired only as an intermediate product as, for example, when the salt is formed only for purposes of purification, and identification, or when it is used as intermediate in preparing a pharmaceutically acceptable salt by ion exchange procedures. In particular, acid addition salts can be prepared by separately reacting the purified compound in its free base form with a suitable organic or inorganic acid and isolating the salt thus formed. Pharmaceutically acceptable salts within the scope of the invention include those derived from mineral acids and organic acids. Exemplary acid addition salts include the hydrobromide, hydrochloride, sulfate, bisulfate, phosphate, nitrate, acetate, oxalate, valerate, oleate, palmitate, quinates, stearate, laurate, borate, benzoate, lactate, phosphate, tosylate, citrate, maleate, fumarate, succinate, tartrate, naphthylate, mesylate, glucoheptonate, lactiobionate, sulfamates, malonates, salicylates, propionates, methylene-bis-β-hydroxynaphthoates, gentisates, isethionates, di-*para*-toluoyltartrates, ethanesulfonates, benzenesulfonates, cyclohexylsulfamates and laurylsulfonate salts. See, for example S.M. Berge, et al., "Pharmaceutical Salts," J. Pharm. Sci., 66, 1-19 (1977), which is incorporated herein by reference.

Where the compound of the invention is substituted with an acidic moiety, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the patient in pharmaceutical doses of the salts, so that the beneficial inhibitory effects inherent in the free base are not vitiated by side effects ascribable to the cations. Base addition salts can also be prepared by separately reacting the purified compound in its acid form with a suitable organic or inorganic base derived from alkali and alkaline earth metal salts and isolating the salt thus formed. Base addition salts include pharmaceutically acceptable metal and amine salts. Suitable metal salts include the sodium, potassium, calcium, barium, zinc, magnesium, and aluminum salts. Particular salts are the sodium and potassium salts. Suitable inorganic base addition salts are prepared from metal bases which include sodium hydride, sodium hydroxide, potassium hydroxide, calcium hydroxide, aluminum hydroxide, lithium hydroxide, magnesium hydroxide, zinc hydroxide and the like. Suitable amine base addition salts are prepared from amines which have sufficient basicity to form a stable salt, and preferably include those amines which are frequently used in medicinal chemistry because of their low toxicity and acceptability for medical use. Ammonia, ethylenediamine, N-methylglucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e.g., lysine and arginine, and dicyclohexylamine.

As well as being useful in themselves as active compounds, salts of compounds of the invention are useful for the purposes of purification of the compounds, for example by exploitation of the solubility differences between the salts and the parent compounds, side products and/or starting materials by techniques well known to those skilled in the art.

It will be appreciated that compounds of the present invention may contain asymmetric centers. These asymmetric centers may independently be either the R or S configuration. It will be apparent to those skilled in the art that certain compounds of the invention may also exhibit geometrical isomerism. It is to be understood that the present invention includes individual geometrical isomers and stereoisomers and mixtures thereof, including racemic mixtures, of compounds of the invention. Such isomers can be separated from their mixtures, by the application or adaptation of known methods, for example chromatographic techniques and recrystallization techniques, or they are separately prepared from the appropriate isomers of their intermediates. Additionally, in situations where tautomers of the compounds of the invention are possible, the present invention is intended to include all tautomeric forms of the compounds.

One particular embodiment of the present invention is a compound of formula (I),

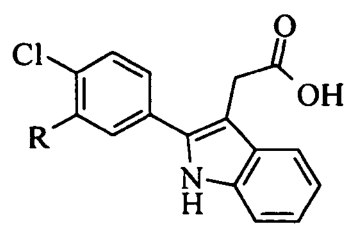

(I)

wherein:

- R is $R^1CH_2SO_2$-, $R^2CH_2SO_2NH$-, or $R^3NHSO_2$-;
- $R^1$ is phenyl optionally substituted with halo;
- $R^2$ is phenyl substituted with halo; and
- $R^3$ is 2,6-dichloro-benzyl, 3,5-dichloro-benzyl, 2,4-dichloro-phenylethyl, 2-methoxy- phenylethyl, 3-methoxy-phenylethyl, 4-methoxy-phenylethyl, 2-trifluoromethyl- phenylethyl, phenylethyl or 3-phenyl-n-propyl;

or a pharmaceutically acceptable salt, hydrate, or solvate thereof, a pharmaceutically acceptable ester prodrug thereof, or a pharmaceutically acceptable salt, hydrate or solvate of the ester prodrug.

Another particular embodiment of the present invention is a compound of formula (I), wherein R is $R^3NHSO_2$-,

a pharmaceutically acceptable salt, hydrate, or solvate thereof, a pharmaceutically acceptable ester prodrug thereof, or a pharmaceutically acceptable salt, hydrate or solvate of the ester prodrug.

Another particular embodiment of the present invention is a compound of formula (II),

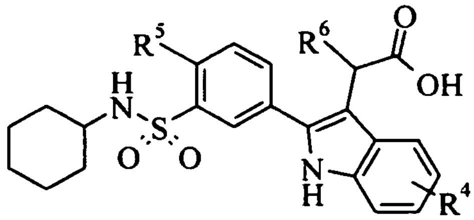

(II)

wherein:

$R^4$ is 4-chloro, 4-fluoro, 4-methyl or 7-chloro;
$R^5$ is chloro or ethyl; and
$R^6$ is hydrogen or methyl;

or a pharmaceutically acceptable salt, hydrate, or solvate thereof, a pharmaceutically acceptable ester prodrug thereof, or a pharmaceutically acceptable salt, hydrate or solvate of the ester prodrug.

Another particular embodiment of the present invention is a compound of formula (II), wherein $R^5$ is chloro and $R^6$ is hydrogen, or a pharmaceutically acceptable salt, hydrate, or solvate thereof, a pharmaceutically acceptable ester prodrug thereof, or a pharmaceutically acceptable salt, hydrate or solvate of the ester prodrug.

Another particular embodiment of the present invention is a compound of formula (III),

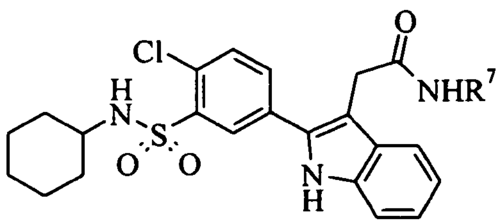

(III)

wherein $R^7$ is methyl, methylsulfonyl, ethylsulfonyl, haloalkylsulfonyl or tetrazolyl, or a pharmaceutically acceptable salt, hydrate, or solvate thereof, a pharmaceutically acceptable ester prodrug thereof, or a pharmaceutically acceptable salt, hydrate or solvate of the ester prodrug.

Another particular embodiment of the present invention is a compound selected from
{2-[4-Chloro-3-(2,6-dichloro-benzylsulfamoyl)-phenyl]-1H-indol-3-yl}-acetic acid,
{2-[4-Chloro-3-(3,5-dichloro-benzylsulfamoyl)-phenyl]-1H-indol-3-yl}-acetic acid,
(2-{4-Chloro-3-[2-(2,4-dichloro-phenyl)-ethylsulfamoyl]-phenyl}-1H-indol-3-yl)-acetic acid,
(2-{4-Chloro-3-[2-(2-methoxy-phenyl)-ethylsulfamoyl]-phenyl}-1H-indol-3-yl)-acetic acid,
(2-{4-Chloro-3-[2-(3-methoxy-phenyl)-ethylsulfamoyl]-phenyl}-1H-indol-3-yl)-acetic acid,
(2-{4-Chloro-3-[2-(4-methoxy-phenyl)-ethylsulfamoyl]-phenyl}-1H-indol-3-yl)-acetic acid,
(2-{4-Chloro-3-[2-(2-trifluoromethoxy-phenyl)-ethylsulfamoyl]-phenyl}-1H-indol-3-yl)-acetic acid,
[2-(4-Chloro-3-phenethylsulfamoyl-phenyl)-1H-indol-3-yl]-acetic acid,
{2-[4-Chloro-3-(3-phenyl-propylsulfamoyl)-phenyl]-1H-indol-3-yl}-acetic acid,
2-[2-(4-Chloro-3-cyclohexylsulfamoyl-phenyl)-1H-indol-3-yl]-N-methyl-acetamide,
[4-Chloro-2-(4-chloro-3-cyclohexylsulfamoyl-phenyl)-1H-indol-3-yl]-acetic acid,
Potassium, [4-chloro-2-(4-chloro-3-cyclohexylsulfamoyl-phenyl)-1H-indol-3-yl]-acetate,
[2-(4-Chloro-3-cyclohexylsulfamoyl-phenyl)-4-fluoro-1H-indol-3-yl]-acetic acid,
[2-(4-Chloro-3-cyclohexylsulfamoyl-phenyl)-4-methyl-1H-indol-3-yl]-acetic acid,

[7-Chloro-2-(4-chloro-3-cyclohexylsulfamoyl-phenyl)-1H-indol-3-yl]-acetic acid,
2-Chloro-N-cyclohexyl-5-[3-(2-methanesulfonylamino-2-oxo-ethyl)-1H-indol-2-yl]-benzenesulfonamide,
2-Chloro-N-cyclohexyl-5-[3-(2-ethanesulfonylamino-2-oxo-ethyl)-1H-indol-2-yl]-benzenesulfonamide,
2-Chloro-N-cyclohexyl-5-[3-(2-oxo-2-trifluoromethanesulfonylamino-ethyl)-1H-indol-2-yl]-benzenesulfonamide,
2-[2-(4-Chloro-3-cyclohexylsulfamoyl-phenyl)-1H-indol-3-yl]-N-(1H-tetrazol-5-yl)-acetamide,
[2-(3-cyclohexylsulfamoyl-4-ethyl-phenyl)-1H-indol-3-yl]-acetic acid,
2-[2-(4-Chloro-3-cyclohexylsulfamoyl-phenyl)-1H-indol-3-yl]-propionic acid, {2-[4-Chloro-3-(3-chloro-phenylmethanesulfonyl)-phenyl]-1H-indol-3-yl}-acetic acid, or {2-[4-Chloro-3-(3-chloro-phenylmethanesulfonylamino)-phenyl]-1H-indol-3-yl}-acetic acid,
or a pharmaceutically acceptable salt, hydrate, or solvate thereof, a pharmaceutically acceptable ester prodrug thereof, or a pharmaceutically acceptable salt, hydrate or solvate of the ester prodrug.

The compounds of the invention exhibit prostaglandin D2 receptor antagonist activity and are useful as pharmacological acting agents. Accordingly, they are incorporated into pharmaceutical compositions and used in the treatment of patients suffering from certain medical disorders.

Compounds within the scope of the present invention are antagonists of the prostaglandin D2 receptor, according to tests described in the literature and described in pharmacological testing section hereinafter, and which tests results are believed to correlate to pharmacological activity in humans and other mammals. Thus, in a further embodiment, the present invention provides compounds of the invention and compositions containing compounds of the invention for use in the treatment of a patient suffering from, or subject to, conditions, which can be ameliorated by the administration of a PGD2 antagonist. For example, compounds of the present invention could therefore be useful in the treatment of a variety of PGD2-mediated disorders including, but not limited to, allergic disease (such as allergic rhinitis, allergic conjunctivitis, atopic dermatitis, bronchial asthma and food allergy), systemic mastocytosis, disorders accompanied by systemic mast cell activation, anaphylaxis shock, bronchoconstriction, bronchitis, urticaria, eczema, diseases accompanied by itch (such as atopic dermatitis and urticaria), diseases (such as cataract, retinal detachment, inflammation, infection and sleeping disorders) which are generated secondarily as a result of behavior accompanied by itch (such as scratching and beating), inflammation, chronic obstructive pulmonary diseases, ischemic reperfusion injury, cerebrovascular accident, chronic rheumatoid arthritis, pleurisy, ulcerative colitis and the like.

Compounds of the present invention are further useful in treatments involving a combination therapy with:

(i) antihistamines, such as fexofenadine, loratadine and citirizine, for the treatment of allergic rhinitis;
(ii) leukotriene antagonists, such as montelukast and zafirlukast, for the treatment of allergic rhinitis, COPD, allergic dermatitis, allergic conjunctivitis, etc - please specifically refer to the claims in WO 01/78697 A2;
(iii) beta agonists, such as albuterol, salbuterol and terbutaline, for the treatment of asthma, COPD, allergic dermatitis, allergic conjunctivitis, etc;
(iv) antihistamines, such as fexofenadine, loratadine, desloratadine and cetirizine, for the treatment of asthma, COPD, allergic dermatitis, allergic conjunctivitis, etc;
(v) PDE4 (Phosphodiesterase 4) inhibitors, such as roflumilast and cilomilast, for the treatment of asthma, COPD, allergic dermatitis, allergic conjunctivitis, etc; or
(vi) with TP (Thromboxane A2 receptor) or CrTh2 (chemoattractant receptor-homologous molecule expressed on Th2 cells) antagonists, such as Ramatrobran (BAY-u3405), for the treatment of COPD, allergic dermatitis, allergic conjunctivitis, etc.

A special embodiment of the therapeutic methods of the present invention is the treating of allergic rhinitis.

Another special embodiment of the therapeutic methods of the present invention is the treating of bronchial asthma.

References herein to treatment should be understood to include prophylactic therapy as well as treatment of established conditions.

The present invention also includes within its scope pharmaceutical compositions comprising at least one of the compounds of the invention in admixture with a pharmaceutically acceptable carrier.

In practice, the compound of the present invention may be administered in pharmaceutically acceptable dosage form to humans and other animals by topical or systemic administration, including oral, inhalational, rectal, nasal, buccal, sublingual, vaginal, colonic, parenteral (including subcutaneous, intramuscular, intravenous, intradermal, intrathecal and epidural), intracisternal and intraperitoneal. It will be appreciated that the preferred route may vary with for example the condition of the recipient.

"Pharmaceutically acceptable dosage forms" refers to dosage forms of the compound of the invention, and includes, for example, tablets, dragées, powders, elixirs, syrups, liquid preparations, including suspensions, sprays, inhalants tablets, lozenges, emulsions, solutions, granules, capsules and suppositories, as well as liquid preparations for injections, including liposome preparations. Techniques and formulations generally may be found in Remington's

Pharmaceutical Sciences, Mack Publishing Co., Easton, PA, latest edition.

A particular aspect of the invention provides for a compound according to the present invention to be administered in the form of a pharmaceutical composition. Pharmaceutical compositions, according to the present invention, comprise compounds of the present invention and pharmaceutically acceptable carriers.

Pharmaceutically acceptable carriers include at least one component selected from the group comprising pharmaceutically acceptable carriers, diluents, coatings, adjuvants, excipients, or vehicles, such as preserving agents, fillers, disintegrating agents, wetting agents, emulsifying agents, emulsion stabilizing agents, suspending agents, isotonic agents, sweetening agents, flavoring agents, perfuming agents, coloring agents, antibacterial agents, antifungal agents, other therapeutic agents, lubricating agents, adsorption delaying or promoting agents, and dispensing agents, depending on the nature of the mode of administration and dosage forms.

Exemplary suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances.

Exemplary antibacterial and antifungal agents for the prevention of the action of microorganisms include parabens, chlorobutanol, phenol, sorbic acid, and the like.

Exemplary isotonic agents include sugars, sodium chloride, and the like.

Exemplary adsorption delaying agents to prolong absorption include aluminum monostearate and gelatin.

Exemplary adsorption promoting agents to enhance absorption include dimethyl sulfoxide and related analogs.

Exemplary diluents, solvents, vehicles, solubilizing agents, emulsifiers and emulsion stabilizers, include water, chloroform, sucrose, ethanol, isopropyl alcohol, ethyl carbonate, ethyl acetate, benzyl alcohol, tetrahydrofurfuryl alcohol, benzyl benzoate, polyols, propylene glycol, 1,3-butylene glycol, glycerol, polyethylene glycols, dimethylformamide, Tween® 60, Span® 60, cetostearyl alcohol, myristyl alcohol, glyceryl mono-stearate and sodium lauryl sulfate, fatty acid esters of sorbitan, vegetable oils (such as cottonseed oil, groundnut oil, com germ oil, olive oil, castor oil and sesame oil) and injectable organic esters such as ethyl oleate, and the like, or suitable mixtures of these substances.

Exemplary excipients include lactose, milk sugar, sodium citrate, calcium carbonate and dicalcium phosphate.

Exemplary disintegrating agents include starch, alginic acids and certain complex silicates.

Exemplary lubricants include magnesium stearate, sodium lauryl sulfate, talc, as well as high molecular weight polyethylene glycols.

The choice of pharmaceutical acceptable carrier is generally determined in accordance with the chemical properties of the active compound such as solubility, the particular mode of administration and the provisions to be observed in pharmaceutical practice.

Pharmaceutical compositions of the present invention suitable for oral administration may be presented as discrete units such as a solid dosage form, such as capsules, cachets or tablets each containing a predetermined amount of the active ingredient, or as a powder or granules; as a liquid dosage form such as a solution or a suspension in an aqueous liquid or a nonaqueous liquid, or as an oil-in-water liquid emulsion or a water-in-oil liquid emulsion. The active ingredient may also be presented as a bolus, electuary or paste.

"Solid dosage form" means the dosage form of the compound of the invention is solid form, for example capsules, tablets, pills, powders, dragées or granules. In such solid dosage forms, the compound of the invention is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alginates, gelatin, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and $Na_2CO_3$, (e) solution retarders, as for example paraffin, (f) absorption accelerators, as for example, quaternary ammonium compounds, (g) wetting agents, as for example, cetyl alcohol and glycerol monostearate. (h) adsorbents, as for example, kaolin and bentonite, (i) lubricants, as for example, talc, calcium stearate, magnesium stearate, solid polyethylene glycols, sodium lauryl sulfate, (j) opacifying agents, (k) buffering agents, and agents which release the compound(s) of the invention in a certain part of the intestinal tract in a delayed manner.

A tablet may be made by compression or molding, optionally with one or more accessory ingredients. Compressed tables may be prepared by compressing in a suitable machine the active ingredient in a free-flowing form such as a powder or granules, optionally mixed with a binder, lubricant, inert diluent, preservative, surface active or dispersing agent. Excipients such as lactose, sodium citrate, calcium carbonate, dicalcium phosphate and disintegrating agents such as starch, alginic acids and certain complex silicates combined with lubricants such as magnesium stearate, sodium lauryl sulfate and talc may be used. A mixture of the powdered compounds moistened with an inert liquid diluent may be molded in a suitable machine to make molded tablets. The tablets may optionally be coated or scored and may be formulated so as to provide slow or controlled release of the active ingredient therein.

Solid compositions may also be employed as fillers in soft and hard-filled gelatin capsules using such excipients as lactose or milk sugar as well as high molecular weight polyethylene glycols, and the like.

If desired, and for more effective distribution, the compounds can be microencapsulated in, or attached to, a

slow release or targeted delivery systems such as a biocompatible, biodegradable polymer matrices (e.g., poly(d,l-lactide co-glycolide)), liposomes, and microspheres and subcutaneously or intramuscularly injected by a technique called subcutaneous or intramuscular depot to provide continuous slow release of the compound(s) for a period of 2 weeks or longer. The compounds may be sterilized, for example, by filtration through a bacteria-retaining filter, or by incorporating sterilizing agents in the form of sterile solid compositions that can be dissolved in sterile water, or some other sterile injectable medium immediately before use.

"Liquid dosage form" means the dose of the active compound to be administered to the patient is in liquid form, for, example, pharmaceutically acceptable emulsions, solutions, suspensions, syrups and elixirs. In addition to the active compounds, the liquid dosage forms may contain inert diluents commonly used in the art, such solvents, solubilizing agents and emulsifiers.

When aqueous suspensions are used they can contain emulsifying agents or agents which facilitate suspension.

Pharmaceutical compositions suitable for topical administration means formulations that are in a form suitable to be administered topically to a patient. The formulation may be presented as a topical ointment, salves, powders, sprays and inhalants, gels (water or alcohol based), creams, as is generally known in the art, or incorporated into a matrix base for application in a patch, which would allow a controlled release of compound through the transdermal barrier. When formulated in an ointment, the active ingredients may be employed with either a paraffinic or a water-miscible ointment base. Alternatively, the active ingredients may be formulated in a cream with an oil-in-water cream base. Formulations suitable for topical administration in the eye include eye drops wherein the active ingredient is dissolved or suspended in a suitable carrier, especially an aqueous solvent for the active ingredient. Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

The oily phase of the emulsion pharmaceutical composition may be constituted from known ingredients in a known manner. While the phase may comprise merely an emulsifier (otherwise known as an emulgent), it desirably comprises a mixture of at least one emulsifier with a fat or an oil or with both a fat and an oil. In a particular embodiment, a hydrophilic emulsifier is included together with a lipophilic emulsifier that acts as a stabilizer. Together, the emulsifier (s) with or without stabilizer(s) make up the emulsifying wax, and the way together with the oil and fat make up the emulsifying ointment base which forms the oily dispersed phase of the cream formulations.

If desired, the aqueous phase of the cream base may include, for example, a least 30% w/w of a polyhydric alcohol, i.e. an alcohol having two or more hydroxy groups such as propylene glycol, butane 1,3-diol, mannitol, sorbitol, glycerol and polyethylene glycol (including PEG 400) and mixtures thereof. The topical formulations may desirably include a compound that enhances absorption or penetration of the active ingredient through the skin or other affected areas.

The choice of suitable oils or fats for a composition is based on achieving the desired properties. Thus a cream should preferably be a non-greasy, non-staining and washable product with suitable consistency to avoid leakage from tubes or other containers. Straight or branched chain, mono- or dibasic alkyl esters such as di-isopropyl myristate, decyl oleate, isopropyl palmitate, butyl stearate, 2-ethylhexyl palmitate or a blend of branched chain esters known as Crodamol CAP may be used. These may be used alone or in combination depending on the properties required. Alternatively, high melting point lipids such as white soft paraffin and/or liquid paraffin or other mineral oils can be used.

Pharmaceutical compositions suitable for rectal or vaginal administrations means formulations that are in a form suitable to be administered rectally or vaginally to a patient and containing at least one compound of the invention. Suppositories are a particular form for such formulations that can be prepared by mixing the compounds of this invention with suitable non-irritating excipients or carriers such as cocoa butter, polyethylene glycol or a suppository wax, which are solid at ordinary temperatures but liquid at body temperature and therefore, melt in the rectum or vaginal cavity and release the active component.

Pharmaceutical composition administered by injection may be by transmuscular, intravenous, intraperitoneal, and/or subcutaneous injection. The compositions of the present invention are formulated in liquid solutions, in particular in physiologically compatible buffers such as Hank's solution or Ringer's solution. In addition, the compositions may be formulated in solid form and redissolved or suspended immediately prior to use. Lyophilized forms are also included. The formulations are sterile and include emulsions, suspensions, aqueous and nonaqueous injection solutions, which may contain suspending agents and thickening agents and anti-oxidants, buffers, bacteriostats and solutes which render the formulation isotonic, and have a suitably adjusted pH, with the blood of the intended recipient.

Pharmaceutical composition of the present invention suitable for nasal or inhalational administration means compositions that are in a form suitable to be administered nasally or by inhalation to a patient. The composition may contain a carrier, in a powder form, having a particle size for example in the range 1 to 500 microns (including particle sizes in a range between 20 and 500 microns in increments of 5 microns such as 30 microns, 35 microns, etc.). Suitable compositions wherein the carrier is a liquid, for administration as for example a nasal spray or as nasal drops, include aqueous or oily solutions of the active ingredient. Compositions suitable for aerosol administration may be prepared

according to conventional methods and may be delivered with other therapeutic agents. Metered dose inhalers are useful for administering compositions according to the invention for an inhalational therapy.

Actual dosage levels of active ingredient(s) in the compositions of the invention may be varied so as to obtain an amount of active ingredient(s) that is (are) effective to obtain a desired therapeutic response for a particular composition and method of administration for a patient. A selected dosage level for any particular patient therefore depends upon a variety of factors including the desired therapeutic effect, on the route of administration, on the desired duration of treatment, the etiology and severity of the disease, the patient's condition, weight, sex, diet and age, the type and potency of each active ingredient, rates of absorption, metabolism and/or excretion and other factors.

Total daily dose of the compounds of this invention administered to a patient in single or divided doses may be in amounts, for example, of from about 0.001 to about 100 mg/kg body weight daily and preferably 0.01 to 10 mg/kg/day. For example, in an adult, the doses are generally from about 0.01 to about 100, preferably about 0.01 to about 10, mg/kg body weight per day by inhalation, from about 0.01 to about 100, preferably 0.1 to 70, more especially 0.5 to 10, mg/kg body weight per day by oral administration, and from about 0.01 to about 50, preferably 0.01 to 10, mg/kg body weight per day by intravenous administration. The percentage of active ingredient in a composition may be varied, though it should constitute a proportion such that a suitable dosage shall be obtained. Dosage unit compositions may contain such amounts of such submultiples thereof as may be used to make up the daily dose. Obviously, several unit dosage forms may be administered at about the same time. A dosage may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. It goes without saying that, for other patients, it will be necessary to prescribe not more than one or two doses per day.

The formulations can be prepared in unit dosage form by any of the methods well known in the art of pharmacy. Such methods include the step of bringing into association the active ingredient with the carrier that constitutes one or more accessory ingredients. In general the formulations are prepared by uniformly and intimately bringing into association the active ingredient with liquid carriers or finely divided solid carriers or both, and then, if necessary, shaping the product.

The formulations may be presented in unit-dose or multi-dose containers, for example sealed ampoules and vials with elastomeric stoppers, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Compounds of the invention may be prepared by the application or adaptation of known methods, by which is meant methods used heretofore or described in the literature, for example those described by R.C. Larock in Comprehensive Organic Transformations, VCH publishers, 1989.

An ester prodrugs of the compounds of the invention may be prepared by coupling compounds of the invention having a carboxy group, with an alcohol of Formula YOH (wherein Y is alkyl or alkyl substituted by amino, alkylamino or dialkylamino), to give an ester bond using standard coupling procedures. Examples include coupling in the presence of HBTU, and optionally in the presence of DIEA, in DCM at room temperature.

According to a further feature of the invention, acid addition salts of the compounds of this invention may be prepared by reaction of the free base with the appropriate acid, by the application or adaptation of known methods. For example, the acid addition salts of the compounds of this invention may be prepared either by dissolving the free base in water or aqueous alcohol solution or other suitable solvents containing the appropriate acid and isolating the salt by evaporating the solution, or by reacting the free base and acid in an organic solvent, in which case the salt separates directly or can be obtained by concentration of the solution.

The acid addition salts of the compounds of this invention can be regenerated from the salts by the application or adaptation of known methods. For example, parent compounds of the invention can be regenerated from their acid addition salts by treatment with an alkali, e.g. aqueous sodium bicarbonate solution or aqueous ammonia solution.

Compounds of this invention can be regenerated from their base addition salts by the application or adaptation of known methods. For example, parent compounds of the invention can be regenerated from their base addition salts by treatment with an acid, e.g. hydrochloric acid.

Compounds of the present invention may be conveniently prepared, or formed during the process of the invention, as solvates (e.g. hydrates). Hydrates of compounds of the present invention may be conveniently prepared by recrystallization from an aqueous/organic solvent mixture, using organic solvents such as dioxane, THF or MeOH.

According to a further feature of the invention, base addition salts of the compounds of this invention may be prepared by reaction of the free acid with the appropriate base, by the application or adaptation of known methods. For example, the base addition salts of the compounds of this invention may be prepared either by dissolving the free acid in water or aqueous alcohol solution or other suitable solvents containing the appropriate base and isolating the salt by evaporating the solution, or by reacting the free acid and base in an organic solvent, in which case the salt separates directly or can be obtained by concentration of the solution.

The starting materials and intermediates may be prepared by the methods described in the present application or adaptation of known methods.

The compounds of the invention, their methods or preparation and their biological activity will appear more clearly from the examination of the following examples.

Compounds of the invention are identified, for example, by the following analytical methods.

High Pressure Liquid Chromatography - Mass Spectrometry (LCMS) experiments to determine retention times ($R_T$) and associated mass ions are performed using one of the following methods.

Mass Spectra (MS) are recorded using a Micromass LCT mass spectrometer. The method is positive electrospray ionization, scanning mass m/z from 100 to 1000. Liquid chromatography is performed on a Hewlett Packard 1100 Series Binary Pump & Degasser; stationary phase: Phenomenex Synergi 2 μ Hydro-RP 20 X 4.0mm column, mobile phase: A = 0.1% formic acid (FA) in water, B = 0.1% FA in acetonitrile. Injection volume of 5μL by CTC Analytical PAL System. Flow is 1 mL/minute. Gradient is 10% B to 90% B in 3 minutes and 90% B to 100% B in 2 minutes. Auxiliary detectors are: Hewlett Packard 1100 Series UV detector, wavelength = 220 nm and Sedere SEDEX 75 Evaporative Light Scattering (ELS) detector temperature = 46˚C, $N_2$ pressure = 4 bar.

300MHz $^1$H nuclear magnetic resonance spectra (NMR) are recorded at ambient temperature using a Varian Mercury (300 MHz) spectrometer with an ASW 5 mm probe. In the NMR chemical shifts (δ) are indicated in parts per million (ppm) with reference to tetramethylsilane (TMS) as the internal standard.

As used in the examples and preparations that follow, as well as the rest of the application, the terms used therein shall have the meanings indicated: "kg" refers to kilograms, "g" refers to grams, "mg" refers to milligrams, "μg" refers to micrograms, "mol" refers to moles, "mmol" refers to millimoles, "M" refers to molar, "mM" refers to millimolar, "μM" refers to micromolar, "nM" refers to nanomolar, "L" refers to liters, "mL" or "ml" refers to milliliters, "μL" refers to microliters, ""C" refers to degrees Celsius, "mp" or "m.p." refers to melting point, "bp" or "b.p." refers to boiling point, "mm of Hg" refers to pressure in millimeters of mercury, "cm" refers to centimeters, "nm" refers to nanometers, "abs." refers to absolute, "conc." refers to concentrated, "c" refers to concentration in g/mL, "rt" refers to room temperature, "TLC" refers to thin layer chromatography, "HPLC" refers to high performance liquid chromatography, "i.p." refers to intraperitoneally, "i.v." refers to intravenously, "s" = singlet, "d" = doublet. "t" = triplet; "q" = quartet; "m" = multiplet, "dd" = doublet of doublets; "br" = broad, "LC"' = liquid chromatograph, "MS" = mass spectrograph, "ESI/MS" = electrospray ionization/mass spectrograph, "$R_T$" = retention time, "M" = molecular ion, "PSI" = pounds per square inch, "DMSO" = dimethyl sulfoxide, "DMF" = N,N-dimethylformamide. "CDI" = 1,1'-carbonyldiimidazole, "DCM" or "$CH_2Cl_2$" = dichloromethane. "HCl" = hydrochloric acid, "SPA" = Scintillation Proximity Assay, "ATTC" = American Type Culture Collection, "FBS" = Foetal Bovine Serum , "MEM" = Minimal Essential Medium, "CPM" = Counts Per Minute, "EtOAc" = ethyl acetate, "PBS"= Phosphate Buffered Saline, "TMD" = transmembrane domain, "IBMX" = 3-isobutyl-1-methylxanthine, "cAMP" = cyclic adenosine monophosphate, "IUPAC" = International Union of Pure and Applied Chemistry, "MHz" = megahertz, "PEG" = polyethylene glycol, "MeOH " = methanol, "N" = normality, "THF" = tetrahydrofuran, "h" = hours, "min" = minute(s), "$MeNH_2$" = methyl amine, "$N_2$" = nitrogen gas, "iPrOH" = isopropyl alcohol, "O.D." = outer diameter, "MeCN" or "$CH_3CN$" = acetonitrile, "$Et_2O$" = ethyl ether, "TFA" = TFA, "Prep LC" = preparatory "flash" liquid chromatography, "SPE" = solid phase extraction, "LAH" = lithium aluminum hydride, "pmol" = picomolar, "heptane" = n-heptane, "HMBA-AM" resin = 4-hydroxymethylbenzoic acid amino methyl resin, "$PdCl_2(dppf)_2$" = 1,1'-bis(diphenylphosphino)ferrocene-palladium (II) dichloride DCM complex, "HBTU" = 2-(1H-benzotriazol-lyl)-1,1,3,3-tetramethyluronium hexafluorophosphate, "DIEA" = diisopropylethylamine, "CsF" = cesium fluoride, "LiOH" = lithium hydroxide, "~" = approximately, "$IC_{50}$" = the concentration of the compound producing 50% inhibition in the SPA cAMP assay in human LS 174 T cells.

EXAMPLES

Example 1:

(a) {2-[4-Chloro-3-(2,4-dichloro-benzylsulfamoyl)-phenyl]-1H-indol-3-yl}-acetic acid

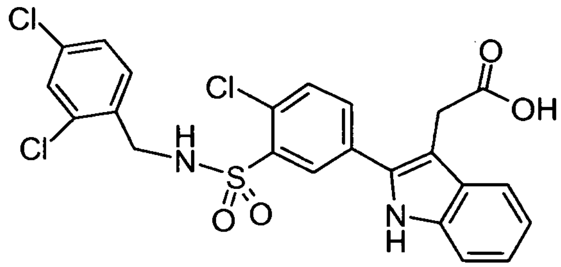

Step 1. Fuming nitric acid (1.5 L) is cooled to about -5˚C in an ice/salt bath. Over a period of 30 minutes, 4-(4-chloro-phenyl)-4-oxo-butyric acid (150 g, 0.706 mol) is added in portions to the mechanically stirred solution, and the reaction mixture is stirred at the temperature between about -5˚C and about -7˚C for 3.5 hours. The reaction mixture is poured onto crushed ice/water (3 L) and stirred overnight at room temperature. The solid material is filtered, washed with water until the washes are neutral, air dried, and finally dried in a vacuum oven at about 85˚C to afford 4-(4-chloro-3-nitro-phenyl)-4-oxo-butyric acid as a solid (159.1 g).

Step 2. To a mechanically stirred suspension of 4-(4-chloro-3-nitro-phenyl)-4-oxo-butyric acid (150 g, 0.582 mol) in water (900 mL) and concentrated HCl (12 mL) is added sodium bisulfite solution (393 g, 2.07 mol, in 800 mL of water) over a period of 40 minutes at 100 - 105˚C. After the addition, the mixture is refluxed for 1 hour. The pH is adjusted to ~2 by the addition of 4 N HCl (100 mL). The mixture is refluxed for an additional 30 minutes, cooled to room temperature and filtered to afford 4-(3-amino-4-chloro-phenyl)-4-oxo-butyric acid as a solid (79.3 g). LCMS: $R_T$ = 2.39 minutes, MS: 228 (M+H); $^1$H NMR (300 MHz, DMSO-$D_6$) δ 2.51 (t, J=6 Hz, 2H) 3.11 (t, J=6 Hz, 2H) 5.58 (s, 2H), 7.1 (dd, J=6.2 Hz, J=2 Hz, 1H) 7.29 (d, J=8 Hz, 1H) 7.36 (d, J=2 Hz, 1H) 12.08 (broad s, 1 H).

Step 3: 4-(3-Amino-4-chloro-phenyl)-4-oxo-butyric acid (16.2 g, 71.16 mmol) in DMF (20 mL) is added to a mixture of concentrated HCl (35 mL) and ice (150 g). A solution of sodium nitrite (5.25 g, 76.1 mmol) in water (18 mL) is added via a pipette below the surface of the solution over 5 minutes, at a temperature between -5˚C and -10˚C. The reaction mixture is warmed to 0˚C and stirred for 15 min. This solution is slowly added at room temperature to a mixture of copper chloride dihydrate (5.58 g, 32.7 mmol) in glacial acetic acid (175 mL) that has been saturated with sulfur dioxide gas. The resulting solution is stirred 45 minutes at room temperature, water (500 mL) is added and the solution is stirred for 1 hour. The flask is cooled to 10˚C and the solid is filtered and washed with water to afford 4-(4-chloro-3-chlorosulfonyl-phenyl)-4-oxo-butyric acid as a solid (12.94 g,). LCMS: $R_T$ = 2.68 minutes, MS: 310 (M+H); $^1$H NMR (300 MHz, DMSO-$D_6$) δ ppm 2.56 (t, J=6 Hz, 2H) 3.19 (t, J=6 Hz, 2H) 7.51 (d, J=8 Hz, 1H) 7.87 (dd, J=6 Hz, J=2 Hz, 1H) 8.39 (d, J=2 Hz, 1H) 12.66 (broad s, I H).

Step 4: 4-(4-Chloro-3-chlorosulfonyl-phenyl)-4-oxo-butyric acid (2 g, 6.43 mmol) is added to a stirred solution of 2,4-dichlorobenzylamine (2.82 g, 16 mmol) in DCM : MeOH mixture (1:1, 50 mL) at 0˚C. The reaction mixture is warmed to room temperature and stirred for 20 hours. The reaction mixture is acidified with 2 N aqueous HCl (pH ~ 2) and extracted twice with DCM. The combined organic layer is washed with water, brine, dried over sodium sulfate and evaporated *in vacuo* to afford 4-[4-chloro-3-(2,4-dichloro-benzylsulfamoyl)-phenyl]-4-oxo-butyric acid as a semi-solid (2.1 g). LCMS: $R_T$ = 2.38 minutes, MS: 448 (M-H).

Step 5: To a mixture of 4-[4-chloro-3-(2,4-dichloro-benzylsulfamoyl)-phenyl]-4-oxo-butyric acid (800 mg, 1.78 mmol), p-toluene sulfonic acid monohydrate (520 mg, 2.7 mmol), and zinc chloride (370 mg, 2.7 mmol) in glacial acetic acid (15 mL) in a microwave vessel is added phenylhydrazine (300 mg, 2.78 mmol). The capped vessel is heated in a microwave at 180˚C for 40 minutes. The reaction mixture is diluted with EtOAc, transferred to a conical flask, and aqueous 2 N HCl (~ 50 mL) is added. The organic layer is separated and the aqueous layer is extracted with EtOAc. The combined organic layer is washed with water, dried over sodium sulfate and concentrated. The residue is purified by preparative HPLC separation (mobile phase: acetonitrile-water with 0.1% TFA; gradient 10-100% over 10 minutes) to afford {2-[4-chloro-3-(2,4-dichloro-benzylsulfamoyl)-phenyl]-1H-indol-3-yl}-acetic acid as a solid (145 mg). LCMS: $R_T$ = 2.78 minutes, MS: 523 (M+H). $^1$H NMR (300 MHz, DMSO- $D_6$) δ 3.74 (s, 2H) 4.23 (d, J=6 Hz, 2H) 7.06 (t, J=7 Hz, 1H) 7.17 (t, J=7 Hz, 1H) 7.3-7.48 (m, 4H), 7.56 (d, J=8 Hz, 1H) 7.75 (d, J=8.3 Hz, 1H) 7.87 (d, J=8 Hz, 1H) 8.22 (d, J=2 Hz, 1H) 8.64 (t, J=6.9 Hz, 1H) 11.52 (s, 1H), 12.4 (broad s, 1H). $IC_{50}$ = 4 nM

(b){2-[4-Chloro-3-(2,6-dichloro-benzylsulfamoyl)-phenyl]-1H-indol-3-yl}-acetic acid

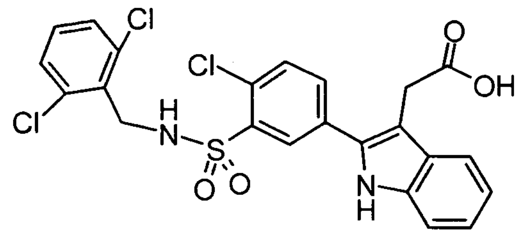

Step 1: By proceeding in a similar manner to Example 1(a), step 4, but substituting 2,6-dichlorobenzylamine (2.82 g) for 2,4-dichlorobenzylamine, there is prepared 4-[4-chloro-3-(2,6-dichloro-benzylsulfamoyl)-phenyl]-4-oxo-butyric acid as a powder(2.12 g). LCMS: $R_T$ = 2.1 minutes, MS: 448 (M-H).

Step 2: By proceeding in a similar manner to Example 1(a), step 5, but substituting 4-[4-chloro-3-(2,6-dichlorobenzylsulfamoyl)-phenyl]-4-oxo-butyric acid (0.8 g) for 4-[4-chloro-3-(2,4-dichloro-benzylsulfamoyl)-phenyl]-4-oxo-butyric acid, there is prepared {2-[4-chloro-3-(2,4-dichloro-benzylsulfamoyl)-phenyl]-1H-indol-3-yl}-acetic acid as a solid (80 mg). LCMS: $R_T$ = 2.72 minutes, MS: 523 (M+H); $^1$H NMR (300 MHz, DMSO- $D_6$) δ 3.75 (s, 2H) 4.36 (d, 2H, J= 5.2 Hz), 7.06 (t, J=7 Hz, 1H) 7.1-7.45 (m, 5H) 7.73 (d, J=8.5 Hz, 1H) 7.57 (d, J=8Hz, 1H) 7.88 (dd, J=6 Hz, J=2.2 Hz, 1 H) 8.25 (d, J=2 Hz, 1H) 8.33 (t, J=5 Hz, 1H) 11.5 (s, 1H), 12.4 (broad s, 1H). $IC_{50}$ = 3 nM

(c) {2-[4-Chloro-3-(3,5-dichloro-benzylsulfamoyl)-phenyl]-1H-indol-3-yl}-acetic acid

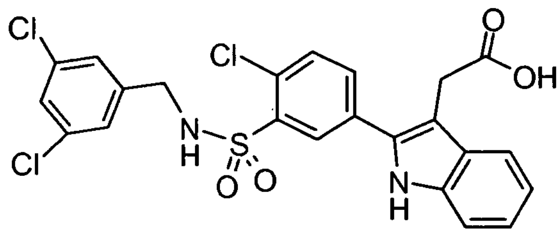

Step 1: By proceeding in a similar manner to Example 1(a), step 4, but substituting 3,5-dichlorobenzylamine (2.82 g) for 2,4-dichlorobenzylamine, there is prepared 4-[4-chloro-3-(3,5-dichloro-benzylsulfamoyl)-phenyl]-4-oxo-bulyric acid as a solid (2.12g). LCMS: $R_T$ = 2.42 minutes, MS: 450 (M+H).

Step 2: By proceeding in a similar manner to Example 1(a) method A, step 5, but substituting 4-[4-chloro-3-(3,5-dichloro-benzylsulfamoyl)-phenyl]-4-oxo-butyric acid (0.8 g) for 4-[4-chloro-3-(2,4-dichloro-benzylsulfamoyl)-phenyl]-4-oxo-butyric acid, there is prepared {2-[4-chloro-3-(3,5-dichloro-benzylsulfamoyl)-phenyl]-1H-indol-3-yl}-acetic acid as a solid (160 mg). LCMS: $R_T$ = 2.78 minutes, MS: 523 (M+H). $^1$H NMR (300 MHz, DMSO- $D_6$) δ 3.72 (s, 2H) 4.19 (d, J= 6.2 Hz, 2H), 7.06 (t, J=7 Hz, 1H) 7.1-7.45 (m, 5H) 7.57 (d, J=8 Hz, 1H) 7.71 (d, J=8.2 Hz, 1H) 7.85 (dd, J=6.2 Hz, J=2.2 Hz, 1 H) 8.19 (d, J=2.2 Hz, 1H) 8.65 (t, J=6.4 Hz, 1H) 11.5 (s, 1H), 12.4 (broad s, 1H). $IC_{50}$ = 12 nM

(d) (2-{4-Chloro-3-[2-(2,4-dichloro-phenyl)-ethylsulfamoyl]-phenyl}-1H-indol-3-yl)-acetic acid

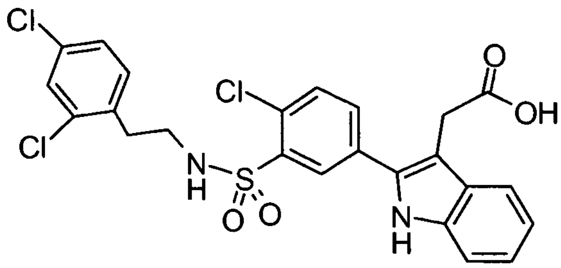

Step 1: By proceeding in a similar manner to Example 1(a), step 4, but substituting 2,4-dichlorophenethylamine (3.04 g) for 2,4-dichlorobenzylamine, there is prepared 4-{4-chloro-3-[2-(2,4-dichloro-phenyl)-ethylsulfamoyl]-phenyl}-4-oxo-butyric acid as a solid (2.3 g). LCMS: $R_T$ = 2.52 minutes, MS: 464 (M+H).

Step 2: By proceeding in a similar manner to Example 1(a) method A, step 5, but substituting 4-{4-chloro-3-[2-(2,4-dichloro-phenyl)-ethylsulfamoyl]-phenyl}-4-oxo-butyric acid (0.83 g) for 4-[4-chloro-3-(2,4-dichloro-benzylsulfamoyl)-phenyl]-4-oxo-butyric acid, there is prepared (2-{4-chloro-3-[2-(2,4-dichloro-phenyl)-ethylsulfamoyl]-phenyl}-1H-indol-3-yl)-acetic acid (150 mg). LCMS: $R_T$ = 2.64 minutes, MS: 537 (M+H). $^1$H NMR (300 MHz, DMSO- $D_6$) δ 2.81 (t, J=7 Hz, 2H) 3.2 (m, 2H) 3.75 (s, 2H), 7.06 (t, J=7.2 Hz, 1H) 7.16(t, J=7.3 Hz, 1H) 7.29 (s, 2H) 7.43 (m, 2H) 7.56 (d, J=7.7 Hz, 1H) 7.74 (d, J= 8.2Hz, 1H) 7.9 (dd, J=6.3 Hz, J=2.2 Hz, 1H) 8.13 (t, J=5.7 Hz, 1H) 8.23 (d, J=2.2 Hz, 1H) 11.52 (s, 1H), 12.4 (broad s, 1H). $IC_{50}$ = 2nM

Example 2:

(a) (2-{4-Chloro-3-[2-(2-methoxy-phenyl)-ethylsulfamoyl]-phenyl}-1H-indol-3-yl)-acetic acid

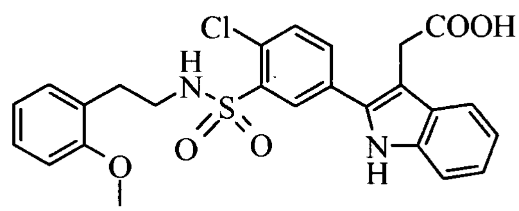

Step 1. A mixture of 2-chloronitrobenzene (53 g, 0.34 mol), iron (1.5 g) and bromine (23 mL, 0.45 mol) is stirred at reflux under $N_2$ for 20 hours. The reaction is concentrated and the residue is purified by flash chromatography on silica gel eluting with 10% EtOAc-heptane. The appropriate fractions are concentrated, filtered, and rinsed with ethanol, and dried. The solid is recrystallized from ethanol to afford 5-bromo-2-chloronitrobenzene (37.9 g). After storage of the mother liquors at 0˚C overnight, a second crop of product is isolated and dried to afford an additional 5-bromo-2-chloronitrobenzene (7 g). MS: 235 (M+H); m.p. 65-67˚C. Step 2. A solution of 5-bromo-2-chloronitrobenzene (10.3 g, 43.6 mmol) in EtOAc (200 mL) is hydrogenated over Raney nickel (6 g of 50% in $H_2O$) at 55 psi $H_2$ for 5 hours. The mixture is filtered through a bed of Celite and rinsed with EtOAc. The filtrate is treated with ethereal HCl (60 mL, 1 M solution in $Et_2O$) under $N_2$. The resulting suspension is stirred for 1 hour and $Et_2O$ (100-200 mL) is added. The mixture is filtered to afford 5-bromo-2-chloroaniline hydrochloride (4.85 g) as a solid. MS: 205 (M+H); m.p. 152-155˚C.

Step 3. A suspension of 5-bromo-2-chloroaniline hydrochloride (41.4 g, 0.17 mol) in $CH_3CN$ (380 mL) is cooled to 5˚C and concentrated HCl (277 mL) is added over 10 minutes. The suspension is cooled to -5˚C and a solution of $NaNO_2$ (14.2 g, 0.21 mol) in $H_2O$ (40 mL) is added dropwise over 10-15 minutes. The mixture is stirred for additional 5 minutes and 30% (w/w) $SO_2$ in HOAc (435 mL) is added at 0˚C, followed by an addition of a solution of copper(II) chloride dihydrate (15.3 g, 0.09 mol) in $H_2O$ (40 mL). The reaction is stirred at room temperature for 1.5 hours. The reaction mixture is filtered and the solid is dried to afford 5-bromo-2-chlorobenzenesulfonyl chloride (18.4 g). The filtrate is stored at 0˚C for 18 hours. The precipitate is collected and dried to afford additional 5-bromo-2-chlorobenzenesulfonyl chloride (9.6 g). MS: 288 (M+H).

Step 4. 5-Bromo-2-chlorobenzenesulfonyl chloride (2 g, 6.9 mmol) is slowly added to a solution of 2-(2-methoxy-phenyl)-ethylamine (1.6 g, 10.74 mmol) and DIEA (2.3 g, 17.8 mmol) in DCM: MeOH (1:1, 50 mL) at 0˚C. The resulting mixture is warmed to room temperature and stirred for 20 hours. The reaction mixture is acidified with 2 N aqueous HCl (~25 mL) and extracted twice with DCM (~50 mL). The organic layer is washed with water, brine, dried over sodium sulfate and evaporated *in vacuo* to afford 5-bromo-2-chloro-N-[2-(2-methoxy-phenyl)-ethyl]-benzenesulfonamide (2.23g) as a white powder. LCMS: $R_T$ = 2.78 minutes, MS: 403 (M+H).

Step 5. To a solution of 1-(*tert*-butoxycarbonyl)-5-methoxy-1H-indol-2-ylboronic acid (2.2 g, 7.5 mmol), 5-bromo-2-chloro-N-[2-(2-methoxy-phenyl)-ethyl]-benzenesulfonamide (2 g, 5 mmol) and CsF (1.14 g, 7.5 mmol) in dioxane-$H_2O$ (100 mL, 9:1) is added $PdCl_2(dppf)_2$ (400 mg) at room temperature under $N_2$. The reaction is heated to 80˚C and stirred for 2 hr. The reaction mixture is concentrated *in vacuo.* The residue is dissolved in EtOAc and filter through a short silica column. The filtrate is concentrated *in vacuo* and the residue is purified by flash chromatography on silica gel eluting with 3% to 30% EtOAc in heptane to 2-{4-chloro-3-[2-(2-methoxy-phenyl)-ethylsulfamoyl]-phenyl}-indole-1-carboxylic acid *tert*-butyl ester (1.9 g). LCMS: $R_T$ = 3.4 minutes, MS: 541 (M+H).

Step 6. A mixture of 2-{4-chloro-3-[2-(2-methoxy-phenyl)-ethylsulfamoyl]-phenyl}-indole-1-carboxylic acid *tert*-butyl ester (1.2 g, 2.2 mmol) and TFA : DCM (1:1, 20mL) is stirred at room temperature for 3 hours and concentrated *in vacuo.* The residue is dissolved in EtOAc and washed with saturated aqueous $NaHCO_3$, water, and brine. The organic layer is separated, dried over $Na_2SO_4$ and concentrated. The residue is purified by flash chromatography on silica gel eluting with 5% to 40% EtOAc in heptane afford 2-chloro-5-(1H-indol-2-yl)-N-[2 (2-methoxy-phenyl)-ethyl]-benzenesulfonamide as a powder (980 mg). LCMS: $R_T$ = 3 minutes, MS: 441 (M+H).

Step 7. Oxalyl chloride (2M in DCM, 2 mL) is slowly added to a solution of 2-chloro-5-(1H-indol-2-yl)-N-[2-(2-methoxy-phenyl)-ethyl]-benzenesulfonamide (600 mg, 1.36 mmol) in DCM (15 mL) at 0˚C. The reaction mixture is allowed to warm up to room temperature. After stirring for 3 hours. MeOH (5 mL) is added and stirred for another 10 minutes. The mixture is concentrated. The residue is purified by flash chromatography on silica gel eluting with 10% to 50% EtOAc in heptane to afford (2-{4-chloro-3-[2-(2-methoxy-phenyl)-ethylsulfamoyl]-phenyl}-1H-indol-3-yl)-oxo-acetic acid methyl ester as a semi-solid (540 mg). LCMS: $R_T$ = 2.72 minutes, MS: 527 (M+H).

Step 8. Triethylsilane (0.5 mL) is slowly added to a solution of (2-{4-chloro-3-[2-(2-methoxy-phenyl)-ethylsulfamoyl]-phenyl}-1H-indol-3-yl)-oxo-acetic acid methyl ester (500 mg; 0.95 mmol) in TFA (5 mL) at room temperature. After stirring for 16 hr, the reaction mixture is concentrated *in vacuo.* The residue is purified by flash chromatography on silica gel eluting with 10% to 40% EtOAc in heptane to afford (2-{4-chloro-3-[2-(2-methoxy-phenyl)-ethylsulfamoyl]-phenyl}-1H-indol-3-yl)-acetic acid methyl ester as a semi-solid (440 mg). LCMS: $R_T$ = 2.88 minutes, MS: 513 (M+H); $^1$H NMR (300 MHz, $CDCl_3$) δ 2.81 (t, J = 6.8 Hz, 2H), 3.23 (q, J=12.6 Hz, J=6.6 Hz, 2H), 3.72 (s, 3H), 3.73 (s, 3H), 3.81 (s, 2H), 5.24 (t, J = 5.7 Hz, 1H), 6.82 (m, 2H), 7.02 (d, J = 7.3 Hz, 1H), 7.2 (m, 3H), 7.4 (d, J = 7.9 Hz, 1H), 7.5 3 (d, J = 8.2 Hz, 1H), 7.67 (d, J = 7.7 Hz, 1H), 7.86 (dd, J = 6.1 Hz, 2.2 Hz, 1H), 8.3 (d, J = 2.2 Hz, 1H), 8.5 (s, 1H).

Step 9. To a mixture of (2-{4-chloro-3-[2-(2-methoxy-phenyl)-ethylsulfamoyl]-phenyl}-1H-indol-3-yl)-acetic acid methyl ester (400 mg, 0.8 mmol) in MeOH/$H_2O$ (2:1, 20 mL) is added lithium hydroxide monohydrate (200 mg, 4.8 mmol). The reaction mixture is stirred at 80˚C for 3 hr and is concentrated. The residue is acidified with 2 N aqueous HCl (pH ~ 2) and extracted twice with ethyl acetate. The combined organic layer is washed with water, brine, dried over sodium sulfate and evaporated *in vacuo.* The residue is purified by flash chromatography on silica gel eluting with 20% to 60% EtOAc in heptane to afford (2-{4-chloro-3-[2-(2-methoxy-phenyl)-ethylsulfamoyl]-phenyl}-1H-indol-3-yl)-acetic acid as a powder (310 mg). LCMS: $R_T$ = 2.57 minutes, MS: 497 (M-H); $^1$H NMR (300 MHz, DMSO-$D_6$) δ 2.67 (t, J = 8 Hz, 2H), 3.1 (m, 2H), 3.62 (s, 3H), 3.75 (s, 2H), 6.82 (m, 2H), 7.02-7.2 (m, 4H), 7.41 (d, J = 8.1 Hz, 1H), 7.56 (d, J = 7.9 Hz, 1H), 7.78 (d, J = 8.2 Hz, 1H), 7.91 (dd, J = 6.3 Hz, 2.2 Hz, 1H), 8.01 (t, J = 5.7 Hz, 1H), 8.25 (d, J = 2.2 Hz, 1H), 11.55 (s, 1H), 12.42 (s, 1H). $IC_{50}$ = 3.8nM

(b) (2-{4-Chloro-3-[2-(3-methoxy-phenyl)-ethylsulfamoyl]-phenyl}-1H-indol-3-yl)-acetic acid

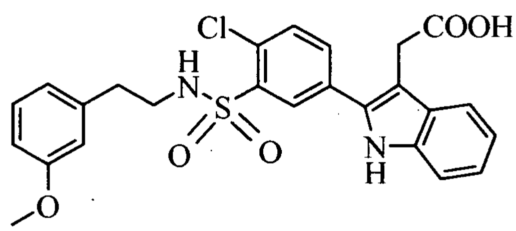

Step 1. By proceeding in a similar manner to Example 2 (a), step 4, but substituting 2-(3-methoxy-phenyl)-ethylamine for 2-(2-methoxy-phenyl)-ethylamine, there is prepared 5-bromo-2-chloro-N-[2-(3-methoxy-phenyl)-ethyl]-benzenesulfonamide as a solid (2.2 g). LCMS: $R_T$ = 2.71 minutes, MS: 402 (M-H).

Step2: By proceeding in a similar manner to Example 2 (a), step 5, but substituting 5-bromo-2-chloro-N-[2-(3-methoxy-phenyl)-ethyl]-benzenesulfonamide for 5-bromo-2-chloro-N-[2-(2-methoxy-phenyl)-ethyl]-benzenesulfonamide, there is prepared 2- {4-chloro-3-[2-(3-methoxyphenyl)-ethylsulfamoyl]-phenyl}-indole-1-carboxylic acid *tert*-butyl ester as an oil (1.69 g). LCMS: $R_T$ = 3.34 minutes, MS: 541 (M+H).

Step3: By proceeding in a similar manner to Example 2 (a), step 6, but substituting 2-{4-chloro-3-[2-(3-methoxy-phenyl)-ethylsulfamoyl]-phenyl}-indole-1-carboxylic acid *tert*-butyl ester for 2-{4-chloro-3-[2-(2-methoxy-phenyl)-ethylsulfamoyl]-phenyl}-indole-1-carboxylic acid *tert*-butyl ester, there is prepared 2-chloro-5-(1H-indol-2-yl)-N-[2-(3-methoxy-phenyl)-ethyl]-benzenesulfonamide as a solid (960 mg). LCMS: $R_T$ = 2.92 minutes, MS: 441 (M+H).

Step 4: By proceeding in a similar manner to Example 2 (a), step 7, but substituting 2-chloro-5-(1H-indol-2-yl)-N-[2-(3-methoxy-phenyl)-ethyl]-benzenesulfonamide for 2-chloro-5-(1H-indol-2-yl)-N-[2-(2-methoxy-phenyl)-ethyl]-benzenesulfonamide, there is prepared (2-{4-chloro-3-[2-(3-methoxy-phenyl)-ethylsulfamoyl]-phenyl}-1H-indol-3-yl)-oxo-acetic acid methyl ester as a semi-solid (551 mg). LCMS: $R_T$ = 2.66 minutes, MS: 527 (M+H).

Step 5: By proceeding in a similar manner to Example 2 (a), step 8, but substituting (2-{4-chloro-3-[2-(3-methoxy-phenyl)-ethylsulfamoyl]-phenyl}-1H-indol-3-yl)-oxo-acetic acid methyl ester for (2-{4-chloro-3-[2-(2-methoxy-phenyl)-ethylsulfamoyl]-phenyl}-1H-indol-3-yl)-oxo-acetic acid methyl ester, there is prepared (2-{4-chloro-3-[2-(3-methoxy-phenyl)-ethylsulfamoyl]-phenyl}-1H-indol-3-yl)-acetic acid methyl ester (450 mg). LCMS: $R_T$ = 2.82 minutes, MS: 513 (M+H). $^1$H NMR (300 MHz, $CDCl_3$) δ 2.78 (t, J = 6.8 Hz, 2H), 3.27 (q, J= 13 Hz, J=6.6 Hz, 2H), 3.73 (s, 3H), 3.75 (s, 3H), 3.81 (s, 2H), 5.07 (t, J = 6 Hz, 1H), 6.7 (m, 3H), 7.2 (m, 3H), 7.4 (d, J = 8.1 Hz, 1H), 7.55 (d, J = 8.1 Hz, 1H), 7.68 (d, J = 7.9 Hz, 1H), 7.86 (dd, J = 6.1 Hz, 2.2 Hz, 1H), 8.3 (d, J = 2.2 Hz, 1H), 8.47 (s, 1H).

Step 6: By proceeding in a similar manner to Example 2 (a), step 9, but substituting (2-{4-chloro-3-[2-(3-methoxy-phenyl)-ethylsulfamoyl]-phenyl}-1H-indol-3-yl)-acetic acid methyl ester for (2-{4-chloro-3-[2-(2-methoxy-phenyl)-ethylsulfamoyl]-phenyl}-1H-indol-3-yl)-acetic acid methyl ester, there is prepared (2-{4-chloro-3-[2-(3-methoxy-phenyl)-ethylsulfamoyl]-phenyl}-1H-indol-3-yl)-acetic acid as a solid (320 mg). LCMS: $R_T$ = 2.5 minutes, MS: 497 (M-H). $^1$H NMR (300 MHz, DMSO-$D_6$)) δ 2.69 (t, J = 7.2 Hz, 2H), 3.18 (m, 2H), 3.67 (s, 3H), 3.75 (s, 2H), 6.7 (t, J= 6.5Hz, 3H), 7.12 (m, 3H), 7.41 (d, J = 8.1 Hz, 1H), 7.57 (d, J = 7.9 Hz, 1H), 7.76 (d, J = 8.2 Hz, 1H), 7.9 (dd, J = 6.2 Hz, 2.1 Hz, 1H), 8.04 (broad t, 1H), 8.25 (d, J = 2.1 Hz, 1H), 11.55 (s, 1H), 12.45 (broad s, 1H). $IC_{50}$ = 3.3nM

(c) (2-{4-Chloro-3-[2-(4-methoxy-phenyl)-ethylsulfamoyl]-phenyl}-1H-indol-3-yl)-acetic acid

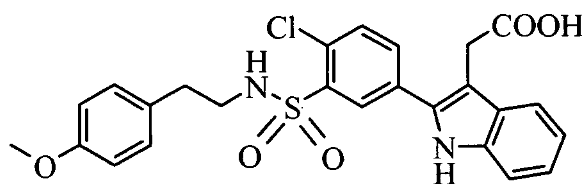

Step 1. By proceeding in a similar manner to Example 2 (a), step 4, but substituting 2-(4-methoxy-phenyl)-ethylamine for 2-(2-methoxy-phenyl)-ethylamine, there is prepared 5-bromo-2-chloro-N-[2-(4-methoxy-phenyl)-ethyl]-benzenesulfonamide as a semi-solid (2.3 g). LCMS: $R_T$ = 2.71 minutes, MS: 402 (M-H).

Step2: By proceeding in a similar manner to Example 2 (a), step 5, but substituting 5-bromo-2-chloro-N-[2-(4-methoxy-phenyl)-ethyl]-benzenesulfonamide for 5-bromo-2-chloro-N-[2-(2-methoxy-phenyl)-ethyl]-benzenesulfonamide, there is prepared 2-{4-chloro-3-[2-(4-methoxyphenyl)-ethylsulfamoyl]-phenyl}-indole-1-carboxylic acid *tert*-butyl ester as an oil (1.87 g). LCMS: $R_T$ = 3.33 minutes, MS: 541 (M+H).

Step3: By proceeding in a similar manner to Example 2 (a), step 6, but substituting 2-{4-chloro-3-[2-(4-methoxy-phenyl)-ethylsulfamoyl]-phenyl}-indole-1-carboxylic acid *tert*-butyl ester for 2-{4-chloro-3-[2-(2-methoxy-phenyl)-ethylsulfamoyl]-phenyl}-indole-1-carboxylic acid *tert*-butyl ester, there is prepared 2-chloro-5-(1H-indol-2-yl)-N-[2-(4-methoxy-phenyl)-ethyl]-benzenesulfonamide as a solid (950 mg). LCMS: $R_T$ = 2.91 minutes, MS: 441 (M+H).

Step 4: By proceeding in a similar manner to Example 2 (a), step 7, but substituting 2-chloro-5-(1H-indol-2-yl)-N-[2-(4-methoxy-phenyl)-ethyl]-benzenesulfonamide for 2-chloro-5-(1H-indol-2-yl)-N-[2-(2-methoxy-phenyl)-ethyl]-benzenesulfonamide, there is prepared (2-{4-chloro-3-[2-(4-methoxy-phenyl)-ethylsulfamoyl]-phenyl}-1H-indol-3-yl)-oxo-acetic acid methyl ester as a semi-solid (552 mg). LCMS: $R_T$ = 2.66 minutes, MS: 527 (M+H).

Step 5: By proceeding in a similar manner to Example 2 (a), step 8, but substituting (2-{4-chloro-3-[2-(4-methoxy-phenyl)-ethylsulfamoyl]-phenyl}-1H-indol-3-yl)-oxo-acetic acid methyl ester for (2-{4-chloro-3-[2-(2-methoxy-phenyl)-ethylsulfamoyl]-phenyl}-1H-indol-3-yl)-oxo-acetic acid methyl ester, there is prepared (2-{4-chloro-3-[2-(4-methoxy-phenyl)-ethylsulfamoyl]-phenyl}-1H-indol-3-yl)-acetic acid methyl ester (445 mg). LCMS: $R_T$ = 2.81 minutes, MS: 513 (M+H); $^1$H NMR (300 MHz, $CDCl_3$) δ 2.76 (t, J = 6.6 Hz, 2H), 3.23 (m, 2H), 3.73 (s, 2H), 3.77 (s, 3H), 3.82 (s, 2H), 5 (t, J = 6.1Hz, 1H), 6.78 (dd, J = 5 Hz, 2 Hz, 2H), 7.02 (dd, J = 6.6 Hz, 2 Hz, 2H,), 7.26 (m, 3H), 7.41 (d, J = 8.3 Hz, 1H), 7.58 (d, J = 8.3 Hz, 1H), 7.69 (d, J = 7.9 Hz, 1H), 7.88 (dd, J = 6.0Hz, 2.2 Hz, 1H), 8.3 (d, J = 2.2 Hz, 1H), 8.33 (s, 1H).

Step 6: By proceeding in a similar manner to Example 2 (a), step 9, but substituting (2-{4-chloro-3-[2-(4-methoxy-phenyl)-ethylsulfamoyl]-phenyl}-1H-indol-3-yl)-acetic acid methyl ester for (2-{4-chloro-3-[2-(2-methoxy-phenyl)-ethylsulfamoyl]-phenyl}-1H-indol-3-yl)-acetic acid methyl ester, there is prepared (2-{4-chloro-3-[2-(4-methoxy-phenyl)-ethylsulfamoyl]-phenyl}-1H-indol-3-yl)-acetic acid as a solid (295 mg). LCMS: $R_T$ = 2.51 minutes, MS: 497 (M-H). $^1$H NMR

(300 MHz, DMSO-$D_6$)) δ 2.64 (t, J = 7.4 Hz, 2H), 3.11 (m, 2H), 3.67(s, 3H), 3.75 (s, 2H), 6.75 (d, J=8.6 Hz, 2H), 7.05 (m, 3H), 7.17 (t, J = 7.3 Hz, 1H), 7.41 (d, J = 8.1 Hz, 1H), 7.58 (d, J = 7.9Hz, 1H), 7.76 (d, J = 8.2Hz, 1H), 7.9 (dd, J = 6.3 Hz, 2 Hz, 1H), 8.01 (broad s, 1H), 8.23 (d, J = 2 Hz, 1H), 11.54 (s, 1H), 12.42 (broad s, 1H). $IC_{50}$ = 3 nM

(d) 2-{4-Chloro-3-[2-(2-trifluoromethoxy-phenyl)-ethylsulfamoyl]-phenyl}-1H-indol-3-yl)-acetic acid

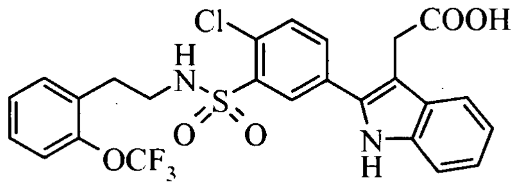

Step 1. By proceeding in a similar manner to Example 2 (a), step 4, but substituting 2-(2-trifluoromethoxy-phenyl)-ethylamine (2.2 g) for 2-(2-methoxy-phenyl)-ethylamine, there is prepared 5-bromo-2-chloro-N-[2-(2-trifluoromethoxy-phenyl)-ethyl]-benzenesulfonamide as a solid (2.4 g). LCMS: $R_T$ = 2.96 minutes, MS: 455.9 (M-H).

Step2: By proceeding in a similar manner to Example 2 (a), step 5, but substituting 5-bromo-2-chloro-N-[2-(2-trifluoromethoxy -phenyl)-ethyl]-benzenesulfonamide (2.3 g) for 5-bromo-2-chloro-N-[2-(2-methoxy-phenyl)-ethyl]-benzenesulfonamide, there is prepared 2-{4-chloro-3-[2-(2-trifluoromethoxy-phenyl)-ethylsulfamoyl]-phenyl}-indole-1-carboxylic acid *tert*-butyl ester as a solid (2.05 g). LCMS: $R_T$ = 3.49 minutes, MS: 595 (M+H).

Step3: By proceeding in a similar manner to Example 2 (a), step 6, but substituting 2-{4-chloro-3-[2-(2-trifluoromethoxy-phenyl)-ethylsulfamoyl]-phenyl}-indole-1-carboxylic acid *tert*-butyl ester for 2-{4-chloro-3-[2-(2-methoxy-phenyl)-ethylsulfamoyl]-phenyl}-indole-1-carboxylic acid *tert*-butyl ester, there is prepared 2-chloro-5-(1H-indol-2-yl)-N-[2-(2-trifluoromethoxy-phenyl)-ethyl]-benzenesulfonamide as a powder (985 mg). LCMS: $R_T$ = 3.1 minutes, MS: 493 (M-H).

Step 4: By proceeding in a similar manner to Example 2 (a), step 7, but substituting 2-chloro-5-(1H-indol-2-yl)-N-[2-(2-trifluoromethoxy-phenyl)-ethyl]-benzenesulfonamide for 2-chloro-5-(1H-indol-2-yl)-N-[2-(2-methoxy-phenyl)-ethyl]-benzenesulfonamide, there is prepared (2-{4-chloro-3-[2-(2-trifluoromethoxy-phenyl)-ethylsulfamoyl]-phenyl}-1H-indol-3-yl)-oxo-acetic acid methyl ester as a solid (575 mg). LCMS: $R_T$ = 2.84 minutes, MS: 581 (M+H).

Step 5: By proceeding in a similar manner to Example 2 (a), step 8, but substituting (2-{4-chloro-3-[2-(2-trifluoromethoxy -phenyl)-ethylsulfamoyl]-phenyl}-1H-indol-3-yl)-oxo-acetic acid methyl ester for (2-{4-chloro-3-[2-(2-methoxy-phenyl)-ethylsulfamoyl]-phenyl}-1H-indol-3-yl)-oxo-acetic acid methyl ester, there is prepared (2-{4-chloro-3-[2-(2-trifluoromethoxy-phenyl)-ethylsulfamoyl]-phenyl}-1H-indol-3-yl)-acetic acid methyl ester as a powder (470 mg). LCMS: $R_T$ = 3 minutes, MS: 567 (M+H). $^1$H NMR (300 MHz, $CDCl_3$) δ 2.91 (t, J = 7 Hz, 2H), 3.27 (q, J=13.4 Hz, J = 6.8 Hz, 2H), 3.73 (s, 3H), 3.81 (s, 2H), 5.07 (t, J = 6 Hz, 1H), 7.25 (m, 6H), 7.41 (d, J = 7.9 Hz, 1H), 7.59 (d, J = 8.2 Hz, 1H), 7.7 (d, J = 7.59 Hz, 1H), 7.91 (dd, J = 6.1, 2.2 Hz, 1H), 8.28 (s, 1H), 8.33 (d, J = 2.2 Hz, 1H).

Step 6: By proceeding in a similar manner to Example 2 (a), step 9, but substituting (2-{4-chloro-3-[2-(2-trifluoromethoxy-phenyl)-ethylsulfamoyl]-phenyl}-1H-indol-3-yl)-acetic acid methyl ester for (2-{4-chloro-3-[2-(2-methoxy-phenyl)-ethylsulfamoyl]-phenyl}-1H-indol-3-yl)-acetic acid methyl ester, there is prepared (2-{4-chloro-3-[2-(2-trifluoromethoxy-phenyl)-ethylsulfamoyl]-phenyl}-1H-indol-3-yl)-acetic acid as a powder (340 mg). LCMS: $R_T$ = 2.69 minutes, MS: 551 (M-H). $^1$H NMR (300 MHz, DMSO-$D_6$) δ 2.8 (t, J = 7 Hz, 2H), 3.17 (q, J=13.4 Hz, J=6.4 Hz, 2H), 3.74 (s, 2H), 7.06 (t, J= 7.5Hz, 1H), 7.17 -7.35 (m, 5H), 7.41 (d, J = 7.9 Hz, 1H), 7.57 (d, J = 7.9 Hz, 1H), 7.79 (d, J = 8.5 Hz, 2.1 Hz, 1H), 7.91 (dd J=6.2Hz, 2.1Hz, 1H), 8.17 (t, J = 5.6 Hz, 1H), 8.24 (d, J = 2 Hz, 1H), 11.57 (s, 1H), 12.42 (broad s, 1H). $IC_{50}$ = 20 nM

(e) [2-(4-Chloro-3-phenethylsulfamoyl-phenyl)-1H-indol-3 -yl]-acetic acid

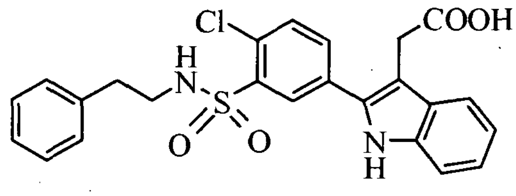

Step 1. By proceeding in a similar manner to Example 2 (a), step 4, but substituting phenethylamine (1.3 g) for 2-(2-methoxy-phenyl)-ethylamine, there is prepared 5-bromo-2-chloro-N-phenethyl-benzenesulfonamide (2.1 g) as a solid. LCMS: $R_T$ = 2.71 minutes, MS: 402 (M-H).

Step2: By proceeding in a similar manner to Example 2 (a), step 5, but substituting 5-bromo-2-chloro-N-phenethyl-benzenesulfonamide (1.9 g) for 5-bromo-2-chloro-N-[2-(2-methoxyphenyl)-ethyl]-benzenesulfonamide, there is prepared 2-(4-chloro-3-phenethylsulfamoyl-phenyl)-indole-1-carboxylic acid *tert*-butyl ester (1.69 g). LCMS: $R_T$ = 3.38 minutes, MS: 511(M+H).

Step3: By proceeding in a similar manner to Example 2 (a), step 6, but substituting 2-(4-chloro-3-phenethylsulfamoyl-phenyl)-indole-1-carboxylic acid *tert*-butyl ester for 2-{4-chloro-3-[2-(2-methoxy-phenyl)-ethylsulfamoyl]-phenyl}-indole-1-carboxylic acid *tert*-butyl ester, there is prepared 2-chloro-5-(1H-indol-2-yl)-N-phenethyl-benzenesulfonamide as a solid (900 mg). LCMS: $R_T$ = 2.96 minutes, MS: 411 (M+H).

Step 4: By proceeding in a similar manner to Example 2 (a), step 7, but substituting 2-chloro-5-(1H-indol-2-yl)-N-phenethyl-benzenesulfonamide for 2-chloro-5-(1H-indol-2-yl)-N-[2-(2-methoxy-phenyl)-ethyl]-benzenesulfonamide, there is prepared [2-(4-chloro-3-phenethylsulfamoyl-phenyl)-1H-indol-3-yl]-oxo-acetic acid methyl ester as a semi-solid (542 mg). LCMS: $R_T$ = 2.68 minutes, MS: 497 (M+H).

Step 5: By proceeding in a similar manner to Example 2 (a), step 8, but substituting [2-(4-chloro-3-phenethylsulfamoyl-phenyl)-1H-indol-3-yl]-oxo-acetic acid methyl ester for (2-{4-chloro-3-[2-(2-methoxy-phenyl)-ethylsulfamoyl]-phenyl}-1H-indol-3-yl)-oxo-acetic acid methyl ester, there is prepared [2-(4-chloro-3-phenethylsulfamoyl-phenyl)-1H-indol-3-yl]-acetic acid methyl ester as a solid (410 mg). LCMS: $R_T$ = 2.84 minutes, MS: 483 (M+H); $^1$H NMR (300 MHz, DMSO-D6) $\delta$ 2.72 (t, J = 7.5 Hz, 2H), 3.15 (q, J=13.4 Hz, J=6.5 Hz, 2H), 3.61 (s, 3H), 3.86 (s, 2H), 7.18 (m, 7H), 7.41 (d, J = 8.1 Hz, 1H), 7.55 (d, J = 7.9 Hz, 1H), 7.77 (d, J = 8.3 Hz, 1H), 7.87 (dd, J = 6.2 Hz, 2 Hz, 1H), 8.08 (t, 5.5 Hz, 1H), 8.2 (d, J = 2 Hz, 1H), 11. 6 (s, 1H).

Step 6: By proceeding in a similar manner to Example 2 (a), step 9, but substituting [2-(4-chloro-3-phenethylsulfamoyl-phenyl)-1H-indol-3-yl]-acetic acid methyl ester for (2-{4-chloro-3-[2-(2-methoxy-phenyl)-ethylsulfamoyl]-phenyl}-1H-indol-3-yl)-acetic acid methyl ester, there is prepared [2-(4-chloro-3-phenethylsulfamoyl-phenyl)-1H-indol-3-yl]-acetic acid (280 mg). LCMS: $R_T$ = 2.53minutes, MS: 467 (M-H); $^1$H NMR (300 MHz, DMSO-$D_6$) $\delta$ 2.72 (t, J = 7.4 Hz, 2H), 3.15 (m, 2H), 3.75 (s, 2H), 7.15 (m, 7H), 7.41 (d, J = 7.8 Hz, 1H), 7.57 (d, J = 7.8 Hz, 1H), 7.77 (d, J = 8.2 Hz, 1H), 7.89 (dd, J = 6.3 Hz, 2 Hz, 1H), 8.06 (t, 5.7 Hz, 1H), 8.24 (d, J = 2 Hz, 1H), 11.55 (s, 1H), 12.45 (broad s, 1H).

(f) {2-[4-Chloro-3-(3-phenyl-propylsulfamoyl)-phenyl]-1H-indol-3-yl}acetic acid

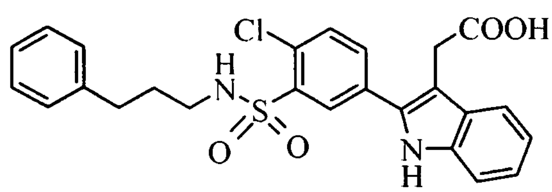

Step 1. By proceeding in a similar manner to Example 1, step 4, but substituting 3-phenyl-propylaminc (1.4 g) for 2-(2-methoxy-phenyl)-ethylamine, there is prepared 5-bromo-2-chloro-N-(3-phenyl-propyl)-benzenesulfonamide (2 g) as a semi-solid. LCMS: $R_T$ = 2.86 minutes, MS: 386 (M-H).

Step2: By proceeding in a similar manner to Example 2 (a), step 5, but substituting bromo-2-chloro-N-(3-phenyl-propyl)-benzenesulfonamide (1.9 g) for 5-bromo-2-chloro-N-[2-(2-methoxy-phenyl)-ethyl]-benzenesulfonamide, there is prepared 2-[4-chloro-3-(3-phenyl-propylsulfamoyl)-phenyl]-indole-1-carboxylic acid *tert*-butyl ester as a solid (1.73

g). LCMS: $R_T$ = 3.43 minutes, MS: 525 (M+H).

Step3: By proceeding in a similar manner to Example 2 (a), step 6, but substituting 2-[4-chloro-3-(3-phenyl-propylsulfamoyl)-phenyl]-indole-1-carboxylic acid *tert*-butyl ester for 2-{4-chloro-3-[2-(2-methoxy-phenyl)-ethylsulfamoyl]-phenyl}-indole-1-carboxylic acid *tert*-butyl ester, there is prepared 2-chloro-5-(1H-indol-2-yl)-N-(3-phenyl-propyl)-benzenesulfonamide as a powder (950 mg). LCMS: $R_T$ = 3.03 minutes, MS: 425 (M+H).

Step 4: By proceeding in a similar manner to Example 2 (a), step 7, but substituting 2-chloro-5-(1H-indol-2-yl)-N-(3-phenyl-propyl)-benzenesulfonamide for 2-chloro-5-(1H-indol-2-yl)-N-[2-(2-methoxy-phenyl)-ethyl]-benzenesulfonamide, there is prepared {2-[4-chloro-3-(3-phenyl-propylsulfamoyl)-phenyl]-1H-indol-3-yl}-oxo-acetic acid methyl ester as a semi-solid (540 mg). LCMS: $R_T$ = 2.76 minutes, MS: 511 (M+H).

Step 5: By proceeding in a similar manner to Example 2 (a), step 8, but substituting {2-[4-chloro-3-(3-phenyl-propylsulfamoyl)-phenyl]-1H-indol-3-yl}-oxo-acetic acid methyl ester for (2-{4-chloro-3-[2-(2-methoxy-phenyl)-ethylsulfamoyl]-phenyl}-1H-indol-3-yl)-oxo-acetic acid methyl ester, there is prepared {2-[4-chloro-3-(3-phenyl-propylsulfamoyl)-phenyl]-1H-indol-3-yl}-acetic acid methyl ester (430 mg). LCMS: $R_T$ = 2.92 minutes, MS: 497 (M+H); $^1$H NMR (300 MHz, $CDCl_3$) δ 1.82 (m, 2H), 2.62 (t, J = 7.5 Hz, 2H), 3 (m, 2H), 3.72 (s, 3H), 3.8 (s, 2H), 5.13 (t, J=6 Hz, 1H), 7.07 - 7.28 (m, 7H), 7.39 (d, J = 8.1 Hz, 1H), 7.6 (d, J = 8.3 Hz, 1H), 7.68 (d, J = 7.9 Hz, 1H), 7.89 (dd, J = 6.1 Hz, 2.2 Hz, 1H), 8.08 (t, 5.5 Hz, 1H), 8.31 (d, J = 2 Hz, 1H).

Step 6: By proceeding in a similar manner to Example 2 (a), step 9, but substituting {2-[4-chloro-3-(3-phenyl-propylsulfamoyl)-phenyl]-1H-indol-3-yl}-acetic acid methyl ester for (2-{4-chloro-3-[2-(2-methoxy-phenyl)-ethylsulfamoyl]-phenyl}-1H-indol-3-yl)-acetic acid methyl ester, there is prepared {2-[4-chloro-3-(3-phenyl-propylsulfamoyl)-phenyl]-1H-indol-3-yl}-acetic acid as a powder (300 mg). LCMS: $R_T$ = 2.61 minutes, MS: 481 (M-H); $^1$H NMR (300 MHz, DMSO-$D_6$) δ 1.67 (m, 2H), 2.5 (m, 2H, buried under DMSO peak), 2.93 (m, 2H), 3.73 (s, 2H), 7 - 7.3 (m, 7H), 7.41 (d, J = 8.1 Hz, 1H), 7.57 (d, J = 7.8 Hz, 1H), 7.83 (d, J = 8.4 Hz, 1H), 7.93 (d, J = 8.1Hz, 1H), 8.04 (apparent s, 1H), 8.26 (s, 1H), 11.6 (s, 1H), 12.45 (broad s, 1H). $IC_{50}$ = 7 nM

Example 3:

2-[2-(4-Chloro-3-cyclohexylsulfamoyl-phenyl)-1H-indol-3-yl]-N-methyl-acetamide

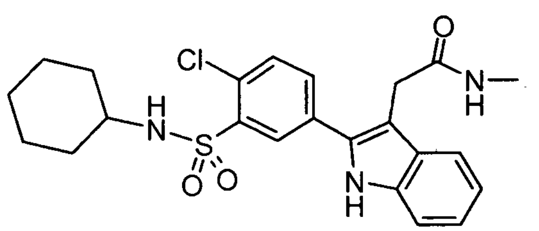

Step 1. 5-Bromo-2-chlorobenzenesulfonyl chloride (4 g, 13.8 mmol) is slowly added to a solution of cyclohexylamine (3.5 g, 35 mmol) in DCM: MeOH (1:1, 100 mL) at 0°C. The resulting mixture is warmed to room temperature and stirred for 20 hours. The reaction mixture is acidified with 2 N aqueous HCl (~100 mL) and extracted twice with DCM (~150 mL). The organic layer is washed with water (~100 mL), brine (~50 mL), dried over sodium sulfate and evaporated *in vacuo* to afford 5-bromo-2-chloro-N-cyclohexyl-benzenesulfonamide (4.2 g). LCMS: $R_T$ = 3 minutes, MS: 351 (M-H).

Step 2. To a solution of 1-(*tert*-butoxycarbonyl)-1H-indol-2-ylboronic acid (2.2 g), 5-bromo-2-chloro-N-cyclohexyl-benzenesulfonamide (1.8 g) and CsF (1.4 g) in 1,4-dioxane-$H_2O$ (60 mL, 10:1) is added $PdCl_2(dppf)_2$ (375mg) at room temperature under nitrogen. The reaction is heated to 80°C and stirred for 3 hr. The reaction mixture is concentrated *in vacuo*. The residue is dissolved in EtOAc and filter through a short silica column. The filtrate is concentrated *in vacuo* and purified by flash silica gel column chromatography eluting with 5% to 50% EtOAc in heptane to afford 2-(4-chloro-3-cyclohexylsulfamoyl-phenyl)-indole-1-carboxylic acid *tert*-butyl ester (1.9 g). LCMS: $R_T$ = 3.31 minutes, MS: 489 (M+H).

Step 3. A mixture of trifluoacetic acid (10 mL) and dichloromethane (10 mL) is added to 2-(4-chloro-3-cyclohexylsulfamoyl-phenyl)-indole-1-carboxylic acid *tert*-butyl ester (1.9 g). The reaction mixture is stirred at room temperature for 2 hours. The mixture is concentrated in *vacuo*. The residue is dissolved in EtOAc and washed with aqueous saturated $NaHCO_3$, water and brine. The organic layer is separated, dried over sodium sulfate and concentrated to afford 2-chloro-N-cyclohexyl-5-(5-methoxy-1H-indol-2-yl)-benzenesulfonamide (1.4 g). LCMS: $R_T$ = 3.17 minutes, MS: 389 (M+H).

Step 4: Oxalyl chloride (1.7 mL of a 2M solution in dichloromethane) is slowly added to a solution of 2-chloro-N-cyclohexyl-5-(1H-indol-2-yl)-benzenesulfonamide (300 mg, 0.77 mmol) in DCM (6 mL) at 0°C. The reaction mixture is allowed to warm up to room temperature and stirred for 3 hrs. Methylamine in THF (7 mL of a 2 M solution) is added and stirred for 15 minutes. The mixture is concentrated and the residue is chromatographed on silica gel eluting with 30-70 % EtOAc/heptane to afford 2-[2-(4-chloro-3-cyclohexylsulfamoyl-phenyl)-1H-indol-3-yl]-N-methyl-2-oxo-acetamide as a powder (285 mg). LCMS: $R_T$ = 2.22 minutes, MS: 474 (M+H); $^1$H NMR (300 MHz, DMSO- $D_6$) δ 0.9 - 1.7 (series of m, 10 H), 2.36 (d, J=4.7 Hz, 3H), 3.02 (m, 1H), 7.3 (m, 2H), 7.52 (d, J=8 Hz, 1H), 7.76 (m, 2H), 7.99 (d, J=8 Hz, 1H), 8.06 (dd, J=5 Hz, J=1.8 Hz, 1H), 8.15 (d, J=1.8 Hz, 1H), 8.49 (d, J=4.8 Hz, 1H), 12.65 (s, 1H).

Step 5. Triethylsilane (1 mL) is slowly added to a solution of 2-[2-(4-chloro-3-cyclohexylsulfamoyl-phenyl)-1H-indol-3-yl]-N-methyl-2-oxo-acetamide (150 mg; 0.32 mmol) in TFA (4 mL) at room temperature. After stirring for ~72 hr, the reaction mixture is concentrated *in vacuo*. The residue is dissolved in EtOAc and washed with saturated aqueous $NaHCO_3$, water, dried over $Na_2SO_4$ and concentrated. The residue is purified by preparative HPLC separation (mobile phase: acetonitrile-water with 0.1% TFA; gradient 10-100% over 10 minutes) to afford 2-[2-(4-Chloro-3-cyclohexylsulfamoyl-phenyl)-1H-indol-3-yl]-N-methylacetamide as a semi-solid (110 mg). LCMS: $R_T$ = 2.6 minutes, MS: 460 (M+H); $^1$H NMR (300 MHz, DMSO- $D_6$) δ 0.9-1.7 (m, 10 H), 2.6 (d, J=4.6 Hz, 3H), 3.04 (m, 1H), 3.6 (s, 2H), 7.03 (t, J=7.4 Hz, 1H), 7.16 (t, J=7.4 Hz, 1H), 7.4 (d, J=8 Hz, 1H), 7.6 (d, J=7.9 Hz, 1H), 7.76 (d, J=8.4 Hz, 1H), 7.89 (d, J=8.3 Hz, 1H), 8.01 (d, J=4.6 Hz, 1H), 8.14 (dd, J=6 Hz, J=2.2 Hz, 1H), 8.33 (d, J=2 Hz, 1H), 11.5 (s, 1H). $IC_{50}$ = 509 nM

Example 4:

[4-Chloro-2-(4-chloro-3-cyclohexylsulfamoyl-phenyl)-1H-indol-3-yl]-acetic acid

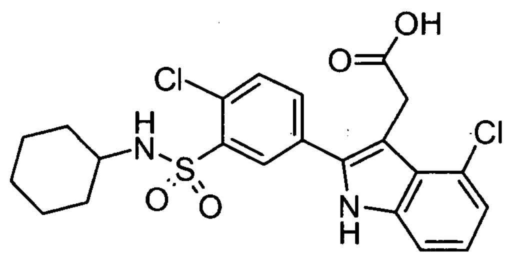

Method A:

Step 1. Di-*tert*-butyl dicarbonate (39.6 g) is added to a solution of 4-chloroindole (25 g) and 4-(dimethylamino) pyridine (2 g) in DCM (800 mL). The reaction is stirred at room temperature for 18 hr. The reaction mixture is washed with 1 N HCl (150 mL) and 1 N $NaHCO_3$ (150 mL). The organic layer is separated, dried over $MgSO_4$ and concentrated. The crude is recrystallized from heptane/ether to afford 4-chloro-indole-1-carboxylic acid *tert*-butyl ester (41.9 g). LCMS: $R_T$ = 3.34 minutes, MS: 251 (M+H).

Step 2. To a solution of 4-chloro-indole-1-carboxylic acid *tert*-butyl ester (10 g) in dry THF (50 mL) is added triisopropyl borate (13.7 mL) under $N_2$. The mixture is cooled to 0°C in an ice bath. Lithium diisopropylamine (33.8 mL, 2 M) is added over an hour at 0°C. The reaction is stirred at 0°C for 30 minutes. 2 N HCl (80 mL) is added. The resulting mixture is extracted with EtOAc. The organic layer is dried, filtered and concentrate. The residue is recrystallized in acetonirile/$H_2O$ to afford 1-(*tert*-butoxycarbonyl)-4-chloro-1H-indol-2-ylboronic acid as a solid (4.5 g).

Step 3. To a solution of 1-(*tert*-butoxycarbonyl)-4-chloro-1H-indol-2-ylboronic acid (4.27g, 14.45 mmol), 5-bromo-2-chloro-N-cyclohexyl-benzenesulfonamide (3 g, 8.5 mmol) and CsF (2.58g, 17 mmol) in dioxane-$H_2O$ (85 mL, 10:1) is added $PdCl_2(dppf)_2$ (694 mg, 0.85mmol) at room temperature under $N_2$. The reaction is heated to 80°C and stirred for 2 hr. The reaction mixture is concentrated *in vacuo*. The residue is dissolved in EtOAc and filter through a short silica column. The filtrate is concentrated *in vacuo* and the residue is purified by flash chromatography on silica gel eluting with 5% to 50% EtOAc in heptane to afford 4-chloro-2-(4-chloro-3-cyclohexylsulfamoyl-phenyl)-indole-1-carboxylic acid *tert*-butyl ester as a solid (3.42 g). LCMS: $R_T$ = 3.5 minutes, MS: 523 (M+H).

Step 4. TFA (20 mL) is added to a solution of 4-chloro-2-(4-chloro-3-cyclohexylsulfamoyl-phenyl)-indole-1-carboxylic acid *tert*-butyl ester (3.42g, 6.53 mmol) in DCM (40 mL). The reaction mixture is stirred at room temperature overnight. The mixture is concentrated *in vacuo*. The residue is dissolved in EtOAc and washed with 1 N $NaHCO_3$. The organic layer is separated, dried over $MgSO_4$ and concentrated to afford 2-chloro-5-(4-chloro-1H-indol-2-yl)-N-cyclohexyl-benzenesulfonamide as a solid (2.8 g). LCMS: $R_T$ = 3.04 minutes, MS: 423 (M+H).

Step 5: Ethyl oxalyl chloride (2.42 g, 17.8 mmol) is slowly added to a suspension of 2-chloro-N-cyclohexyl-5-(1H-indol-2-yl)-benzenesulfonamide (1.5 g, 3.54 mmol) in dichloroethane (150 mL) followed by $AlCl_3$ (2.36 g, 17.8 mmol) at 0˚C. The resulted dark brown solution is allowed to warm up to room temperature and stirred for 16 hrs. MeOH (5 mL) is added at 0˚C to the reaction mixture and diluted with DCM. The organic is washed with water, brine, dried over $Na_2SO_4$ and concentrated to afford [4-chloro-2-(4-chloro-3-cyclohexylsulfamoyl-phenyl)-1H-indol-3-yl]-oxo-acetic acid ethyl ester as a solid (1.8g). LCMS: $R_T$ = 2.87 minutes, MS: 523 (M+H). $^1$H NMR (300 MHz, DMSO-$D_6$) δ 0.8 -1.7 (series of m, 13H) 3.04 (m, 1H), 4.07 (q, J=14.3 Hz, J= 7.2Hz, 2H), 7.3 (m, 2H), 7.54 (dd, J=4.2 Hz, 2.4 Hz, 1H), 7.83 (m, 2H), 8.04 (d, J=8.1 Hz, 1H), 8.17 (d, J=2 Hz, 1H), 12.95 (s, 1H).

Step 6. [4-Chloro-2-(4-chloro-3-cyclohexylsulfamoyl-phenyl)-1H-indol-3-yl]-oxo-acetic acid ethyl ester (1.8 g, 3.45 mmol) is stirred with triethylsilane (6 mL) and TFA (24 mL) at room temperature. After stirring four ~72 hr, the reaction mixture is concentrated *in vacuo*. The residue is dissolved in DCM (~150 mL) and washed with water (~100 mL) twice, brine (~50 mL), dried over $Na_2SO_4$ and concentrated *in vacuo*. The residue is purified by flash chromatography on silica gel eluting with 5% to 50% EtOAc in heptane to afford [4-chloro-2-(4-chloro-3-cyclohexylsulfamoyl-phenyl)-1H-indol-3-yl]-acetic acid ethyl ester as a powder (1.45 g). LCMS: $R_T$ = 3.14 minutes, MS: 509 (M+H). $^1$H NMR (300 MHz, DMSO-$D_6$) δ 0.8 -1.7 (series ofm, 13H) 3.03 (m, 1H) 3.96 (s, 2H), 4.14 (q, J=14.2 Hz, J= 7.2 Hz, 2H), 7.07 (d, J=7.5 Hz, 1H) 7.14 (t, J=7.8 Hz, 1H) 7.4 (d, J=8 Hz, 1H) 7.8 (m, 2H), 7.98 (d, J=8.1 Hz, 1H) 8.14 (d, J=2 Hz, 1H) 11.95 (s, 1H).

Step 7: A mixture of [4-chloro-2-(4-chloro-3-cyclohexylsulfamoyl-phenyl)-1H-indol-3-yl]-acetic acid ethyl ester (1.45 g, 2.85 mmol) and lithium hydroxide monohydrate (600 mg, 14.3 mmol) in MeOH/$H_2O$ (2:1, 100 mL) is stirred at 80˚C for 4 hr. KOH (800 mg; 14.3 mmol) is added to the mixture and stirring continued at 80˚C for 16 hr. The reaction mixture is concentrated *in vacuo*. The residue is acidified with 2 N aqueous HCl (pH ~ 2). The resulted white solids were collected by filtration, washed with $Et_2O$, heptane and dried *in vacuo* for ~72hrs to afford [4-chloro-2-(4-chloro-3-cyclohexylsulfamoyl-phenyl)-1H-indol-3-yl]-acetic acid as a crystalline solid (1.1 g). LCMS: $R_T$ = 2.64 minutes, MS: 481 (M+H); $^1$H NMR (300 MHz, DMSO-$D_6$) δ 0.9 - 1.7 (series of m, 10H), 3.05 (m, 1H), 3.88 (s, 2H), 7.06 (d, J=7.4 Hz, 1H), 7.14 (t, J=7.8 Hz, 1H), 7.40 (d, J=7.9 Hz, 1H), 7.8 (m, 2H), 7.97 (d, J=8.1 Hz, 1H), 8.18 (d, J=1.8 Hz, 1H), 11.92 (s, 1H), 12.45 (broad s, 1H). $IC_{50}$ = 0.2 nM

Method B:

Step 1. Di-*tert*-butyl dicarbonate (39.6 g) is added to a solution of 4-chloroindole (25 g) and 4-(dimethylamino) pyridine (2 g) in DCM (800 mL). The reaction is stirred at room temperature for 18 hr. The reaction mixture is washed with 1 N HCl (150 mL) and 1 N $NaHCO_3$ (150 mL). The organic layer is separated, dried over $MgSO_4$ and concentrated. The crude is recrystallized from heptane/ether to afford 4-chloro-indole-1-carboxylic acid *tert*-butyl ester (41.9 g).

Step 2. To a solution of 4-chloro-indole-1-carboxylic acid *tert*-butyl ester (10 g) in dry THF (50 mL) is added triisopropyl borate (13.7 mL) under $N_2$. The mixture is cooled to 0˚C in an ice bath. Lithium diisopropylamine (33.8 mL, 2 M) is added over an hour at 0˚C. The reaction is stirred at 0˚C for 30 minutes. 2 N HCl (80 mL) is added. The resulting mixture is extracted with EtOAc. The organic layer is dried, filtered and concentrate. The residue is recrystallized in acetonitrile/$H_2O$ to afford 1-(*tert*-butoxycarbonyl)-4-chloro-1H-indol-2-ylboronic acid as a solid (4.5 g).

Step 3. To a solution of 1-(*tert*-butoxycarbonyl)-4-chloro-1H-indol-2-ylboronic acid (1.04 g), 5-bromo-2-chloro-N-cyclohexyl-benzenesulfonamide (1 g) and CsF (864 mg) in dioxane-$H_2O$ (29 mL, 10:1) is added $PdCl_2(dppf)_2$ (232 mg) at room temperature under nitrogen. The reaction is heated to 80 ˚C and stirred for overnight. The reaction mixture is concentrated *in vacuo*. The residue is dissolved in EtOAc and filter through a short silica column. The filtrate is concentrated *in vacuo* and purified by flash silica gel column chromatography eluting with 10% to 50% EtOAc in heptane to afford 4-chloro-2-(4-chloro-3-cyclohexylsulfamoyl-phenyl)-indole-1-carboxylic acid *tert*-butyl ester as a solid (1.04 g).

Step 4. Trifluoacetic acid (5 mL) is added to a solution of 4-chloro-2-(4-chloro-3-cyclohexylsulfamoyl-phenyl)-indole-1-carboxylic acid *tert*-butyl ester (1.04 g) in DCM (10 mL). The reaction mixture is stirred at room temperature for 4 hr. The mixture is concentrated *in vacuo*. The residue is dissolved in EtOAc and washed with 1N $NaHCO_3$. The organic layer is separated, dried over $MgSO_4$ and concentrated to afford 2-chloro-5-(4-chloro-1H-indol-2-yl)-N-cyclohexyl-benzenesulfonamide as a solid (860 mg). LCMS: $R_T$ = 3.06 minutes, MS: 423 (M+H).

Step 5. Oxalyl chloride (0.26 mL) is slowly added to a solution of 2-chloro-5-(4-chloro-1H-indol-2-yl)-N-cyclohexyl-benzenesulfonamide (860 mg) in dichloromethane (20 mL) at room temperature. After stirring for 2 hr, MeOH (5 mL) is added and stirred for 15 minutes. The mixture is concentrated. The residue is purified by flash silica gel column chromatography eluting with 0% to 50% EtOAc in heptane to afford [4-chloro-2-(4-chloro-3-cyclohexylsulfamoyl-phenyl)-1H-indol-3-yl]-oxo-acetic acid methyl ester as a solid (140 mg).

Step 6. Triethylsilane (0.086 mL) is slowly added to a solution of [4-chloro-2-(4-chloro-3-cyclohexylsulfamoyl-phenyl)-1H-indol-3-yl]-oxo-acetic acid methyl ester (140 mg) in trifluoacetic acid (1.4 mL) at room temperature. After stirring for overnight, the volatile is removed *in vacuo*. The residue is dissolved in EtOAc and washed with 1N $NaHCO_3$.

The organic layer is separated, dried over $MgSO_4$ and concentrated. The residue is purified by flash silica gel column chromatography eluting with 10% to 50% EtOAc in heptane to afford [4-chloro-2-(4-chloro-3-cyclohexylsulfamoyl-phenyl)-1H-indol-3-yl]-acetic acid methyl ester as a solid (93 mg).

Step 7. To a solution of [4-chloro-2-(4-chloro-3-cyclohexylsulfamoyl-phenyl)-1H-indol-3-yl]-acetic acid methyl ester (92 mg) in $MeOH/H_2O$ (1:1, 3.6 mL) is added lithium hydroxide monohydrate (16 mg). The reaction mixture is stirred at 80˚C for 18 hr. EtOAc (10 mL) is added and the solution is washed with 1N HCl (5 mL). The organic layer is separated, dried over $MgSO_4$ and concentrated to afford [4-chloro-2-(4-chloro-3-cyclohexylsulfamoyl-phenyl)-1H-indol-3-yl]-acetic acid as a solid (67 mg). LCMS: $R_T$ = 2.52 minutes, MS: 481 (M+H); $^1H$ NMR (300 MHz, $CD_3OD$) δ 1.09-1.35 (m, 5H), 1.51-1.74 (m, 5H), 3.11 (m, 1H), 3.81 (brs, 2H), 7.05 (m, 2H), 7.39 (m, 1H), 7.65 (m, 2H), 8.32 (m, 1H), 11.17 (brs, 1H).

Example 5:

Potassium, [4-chloro-2-(4-chloro-3-cyclohexylsulfamoyl-phenyl)-1H-indol-3-yl]-acetate

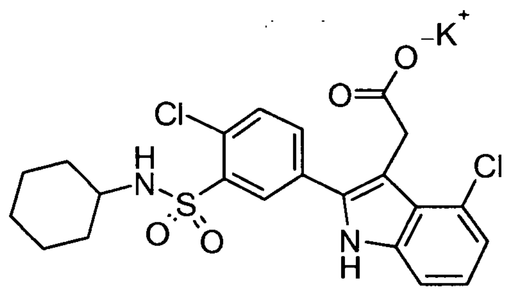

A mixture of [4-chloro-2-(4-chloro-3-cyclohexylsulfamoyl-phenyl)-1H-indol-3-yl]-acetic acid (537 mg, 1.115 mmol) and 200 mL of ethanol is stirred at ~40˚C for 10 minutes. The resulted solution is left to cool to room temperature and potassium hydroxide (62 mg, 1.1 mmol) is added. Stirring continued at room temperature until KOH is dissolved. The solution is concentrated *in vacuo* at ~40˚C. The resulted white solids are dried *in vacuo* for ~20 hrs to afford potassium, [4-chloro-2-(4-chloro-3-cyclohexylsulfamoyl-phenyl)-1H-indol-3-yl]-acetate as a crystalline solid (575 mg). LCMS: $R_T$ = 2.64 minutes, MS: 481 (M+H of parent acid). $^1H$ NMR (300 MHz, DMSO-$D_6$) δ 0.9 - 1.7 (series of m, 10H), 3.06 (m, 1H), 3.66 (s, 2H), 6.93 (d, J=7.2 Hz, 1H), 7.01 (t, J=7.8 Hz, 1H), 7.28 (d, J=7.7 Hz, 1H), 7.66 (d, J=8.3, 1H), 8.1 (dd, J=6.4 Hz, 2 Hz, 2H), 8.32 (d, J=2 Hz, 1H), 11.75 (s, 1H).

Example 6:

[2-(4-Chloro-3-cyclohexylsulfamoyl-phenyl)-4-fluoro-1H-indol-3-yl]-acetic acid

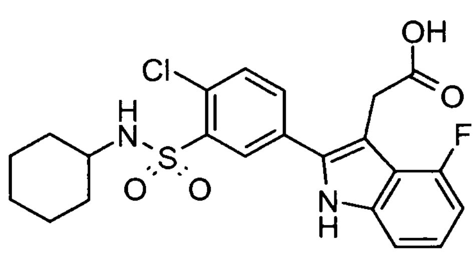

Step 1. Di-*tert*-butyl dicarbonate (8.88 g) is added to a solution of 4-fluoroindole (5 g) and 4-(dimethylamino) pyridine (0.45 g) in dichloromethane (185 mL). The reaction is stirred at room temperature for 4 hr. The reaction mixture

is washed with 1N HCl (100 mL) and 1N $NHCO_3$ (100 mL). The organic layer is separated, dried over $MgSO_4$ and concentrated to afford 4-fluoro-indole-1-carboxylic acid *tert*-butyl ester as an oil (8.32 g). LCMS: $R_T$ = 3.34 minutes, MS: 236.09 (M+H).

Step 2. To a solution of 4-fluoro-indole-1-carboxylic acid *tert*-butyl ester (3 g) in dry THF (16 mL) is added triisopropyl borate (3.6 mL) under nitrogen. The mixture is cooled to 0˚C in an ice bath. Lithium diisopropylamine (12.8 mL, 2 M) is added over an hour at 0˚C. The reaction is stirred at 0˚C for 30 minutes. 2N HCl (10 mL) is added to quench the reaction. The resulting mixture is extracted with EtOAc. The residue is purified by flash silica gel column chromatography eluting with 5% to 50% EtOAc in heptane to afford 1-(*tert*-butoxycarbonyl)-4-fluoro-1H-indol-2-ylboronic acid as a solid (1.65 g).

Step 3. To a solution of 1-(*tert*-butoxycarbonyl)-4-fluoro-1H-indol-2-ylboronic acid (1.19 g), 5-bromo-2-chloro-N-cyclohexyl-benzenesulfonamide (1 g) and CsF (863 mg) in dioxane-$H_2O$ (27.5 mL, 10:1) is added $PdCl_2(dppf)_2$ (231 mg) at room temperature under nitrogen. The reaction is heated to 80 ˚C and stirred for 2 days. The reaction mixture is concentrated *in vacuo*. The residue is dissolved in EtOAc and filter through a short silica column. The filtrate is concentrated *in vacuo* and purified by flash silica gel column chromatography eluting with 5% to 30% EtOAc in heptane to afford 2-chloro-5-(4-fluoro-1H-indol-2-yl)-N-cyclohexyl-benzenesulfonamide as a solid (516 mg). LCMS: $R_T$ = 4.46 minutes, MS: 407 (M+H).

Step 4. Oxalyl chloride (0.16 mL) is slowly added to a solution of 2-chloro-5-(4-fluoro-1H-indol-2-yl)-N-cyclohexyl-benzenesulfonamide (496 mg) in dichloromethane (12 mL) at room temperature. After stirring for 3 hr, MeOH (4 mL) is added and stirred for 15 minutes. The mixture is concentrated. The residue is purified by flash silica gel column chromatography eluting with 5% to 50% EtOAc in heptane to afford [4-fluoro-2-(4-chloro-3-cyclohexylsulfamoyl-phenyl)-1H-indol-3-yl]-oxo-acetic acid methyl ester as a solid (470 mg).

Step 5. Triethylsilane (0.3 mL) is slowly added to a solution of [4-fluoro-2-(4-chloro-3-cyclohexylsulfamoyl-phenyl)-1H-indol-3-yl]-oxo-acetic acid methyl ester (570 mg) in trifluoacetic acid (5 mL) at room temperature. After stirring for overnight, the volatile is removed *in vacuo*. The residue is dissolved in EtOAc and washed with 1N $NaHCO_3$. The organic layer is separated, dried over $MgSO_4$ and concentrated. The residue is purified by flash silica gel column chromatography eluting with 10% to 50% EtOAc in heptane to afford [4-fluoro-2-(4-chloro-3-cyclohexylsulfamoyl-phenyl)-1H-indol-3-yl]-acetic acid methyl ester as a white solid (350 mg). LCMS: $R_T$ = 3.18 minutes, MS: 479.1 (M+H).

Step 6. To a solution of [4-fluoro-2-(4-chloro-3-cyclohexylsulfamoyl-phenyl)-1H-indol-3-yl]-acetic acid methyl ester (250 mg) in MeOH/$H_2O$ (1:1, 7 mL) is added lithium hydroxide monohydrate (44 mg). The reaction mixture is stirred at 80 ˚C for overnight. EtOAc (15 mL) is added and the solution is washed with 1N HCl (10 mL). The organic layer is separated, dried over $MgSO_4$ and concentrated to afford [2-(4-chloro-3-cyclohexylsulfamoyl-phenyl)-4 fluoro-1H-indol-3-yl]-acetic acid as a solid (2.19 mg). LCMS: $R_T$ = 2.83 minutes, MS: 465 (M+H); $^1$H NMR (300 MHz, DMSO) δ 1.09-1.24 (m, 5H), 1.49-1.61 (m, 5H), 3.07 (m, 1H), 3.81 (s, 2H), 6.8 (m, 1H), 7.15 (m, 1H), 7.26 (m, 1H), 7.84 (m, 2H), 7.98 (m, 1H), 8.23 (m, 1H), 11.86 (brs, 1H). $IC_{50}$ = 0.7 nM

Example 7:

[2-(4-Chloro-3-cyclohexylsulfamoyl-phenyl)-4-methyl-1H-indol-3-yl]-acetic acid

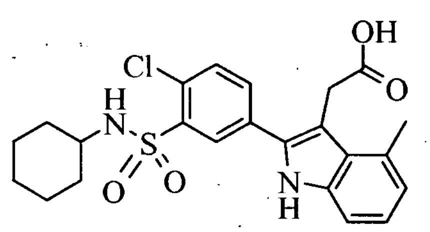

Step 1. Di-*tert*-butyl dicarbonate (9.15 g) is added to a solution of 4-methylindole (5 g) and 4-(dimethylamino) pyridine (0.46 g) in dichloromethane (190 mL). The reaction is stirred at room temperature for 4 hr. The reaction mixture is washed with 1N HCl (100 mL) and 1N $NHCO_3$ (100 mL). The organic layer is separated, dried over $MgSO_4$ and concentrated to afford 4-methyl-indole-1-carboxylic acid *tert*-butyl ester as an oil (8.75 g).

Step 2. To a solution of 4-methyl-indole-1-carboxylic acid *tert*-butyl ester (3 g) in dry THF (16 mL) is added triisopropyl borate (4.45 mL) under nitrogen. The mixture is cooled to 0˚C in an ice bath. Lithium diisopropylamine (11.6 mL, 2 M) is added over an hour at 0˚C. The reaction is stirred at 0˚C for 30 minutes. 2N HCl (10 mL) is added to quench

the reaction. The resulting mixture is extracted with EtOAc. The residue is recrystalized in $CH_3CN/H_2O$ to afford 1-(*tert*-butoxycarbonyl)-4-methyl-1H-indol-2-ylboronic acid as a solid (1.53 g).

Step 3. To a solution of 1-(*tert*-butoxycarbonyl)-4-methyl-1H-indol-2-ylboronic acid (1.41 g), 5-bromo-2-chloro-N-cyclohexyl-benzenesulfonamide (1 g) and CsF (863 mg) in dioxane-$H_2O$ (27.5 mL, 10:1) is added $PdCl_2(dppf)_2$ (232 mg) at room temperature under nitrogen. The reaction is heated to 80°C and stirred for overnight. The reaction mixture is concentrated *in vacuo*. The residue is dissolved in EtOAc and filter through a short silica column. The filtrate is concentrated *in vacuo* and purified by flash silica gel column chromatography eluting with 0% to 50% EtOAc in heptane to afford 2-(4-chloro-3-cyclohexylsulfamoyl-phenyl)-4-methylindole-1-carboxylic acid *tert*-butyl ester as a solid (845 mg).

Step 4. Trifluoacetic acid (5 mL) is added to a solution of 2-(4-chloro-3-cyclohexylsulfamoyl-phenyl)-4-methyl-indole-1-carboxylic acid *tert*-butyl ester (845 mg) in dichloromethane (10 mL). The reaction mixture is stirred at room temperature overnight. The mixture is concentrated *in vacuo*. The residue is dissolved in EtOAc and washed with 1N $NaHCO_3$. The organic layer is separated, dried over $MgSO_4$ and concentrated to afford 2-chloro-5-(4-methyl-1H-indol-2-yl)-N-cyclohexyl-benzenesulfonamide as a solid (652 mg). LCMS: $R_T$ = 3.11 minutes, MS: 403 (M+H).

Step 5. Oxalyl chloride (0.21 mL) is slowly added to a solution of 2-chloro-5-(4-methyl-1H-indol-2-yl)-N-cyclohexyl-benzenesulfonamide (650 mg) in dichloromethane (16 mL) at room temperature. After stirring for overnight, MeOH (5 mL) is added and stirred for 15 minutes. The mixture is concentrated. The residue is purified by flash silica gel column chromatography eluting with 0% to 50% EtOAc in heptane to afford [2-(4-chloro-3-cyclohexylsulfamoyl-phenyl)-4-methyl-1H-indol-3-yl]-oxo-acetic acid methyl ester as a yellow solid (588 mg). LCMS: $R_T$ = 2.8 minutes, MS: 489 (M+H).

Step 6. Triethylsilane (0.38 mL) is slowly added to a solution of [2-(4-chloro-3-cyclohexylsulfamoyl-phenyl)-4-methyl-1H-indol-3-yl]-oxo-acetic acid methyl ester (588 mg) in trifluoacetic acid (2 mL) at room temperature. After stirring for overnight, the volatile is removed *in vacuo*. The residue is dissolved in EtOAc and washed with 1N $NaHCO_3$. The organic layer is separated, dried over $MgSO_4$ and concentrated. The residue is purified by flash silica gel column chromatography eluting with 0% to 40% EtOAc in heptane to afford [2-(4-chloro-3-cyclohexylsulfamoyl-phenyl)-4-methyl-1H-indol-3-yl]-acetic acid methyl ester as a solid (338 mg). LCMS: $R_T$ = 2.95 minutes, MS: 475 (M+H).

Step 7. To a solution of [2-(4-chloro-3-cyclohexylsulfamoyl-phenyl)-4-methyl-1H-indol-3-yl]-acetic acid methyl ester (330 mg) in $MeOH/H_2O$ (1:1, 7 mL) is added lithium hydroxide monohydrate (58 mg). The reaction mixture is stirred at 80 °C for 18hr. EtOAc (15 mL) is added and the solution is washed with 1N HCl (10 mL). The organic layer is separated, dried over $MgSO_4$ and concentrated to afford [2-(4-chloro-3-cyclohexylsulfamoyl-phenyl)-4-methyl-1H-indol-3-yl]-acetic acid as a white solid (210 mg). LCMS: Rt = 2.60 minutes, MS: 461.12 (M+H); $^1H$ NMR (300 MHz, DMSO) δ 1.02-1.28 (m, 5H), 1.46-1.64 (m, 5H), 3.07 (m, 1H), 2.63 (s, 3H), 3.95 (s, 2H), 6.79 (d, J = 6.9 Hz, 1H), 7.05 (t, J = 8.1 Hz, 1H), 7.26 (d, J = 8.1 Hz, 1H), 7.80 (m, 2H), 7.96 (d, J = 8.1 Hz, 1H), 8.21 (s, 1H), 11.53 (s, 1H), 12.54 (brs, 1H). $IC_{50}$ = 1.5 nM

Example 8:

[7-Chloro-2-(4-chloro-3-cyclohexylsulfamoyl-phenyl)-1H-indol-3-yl]-acetic acid

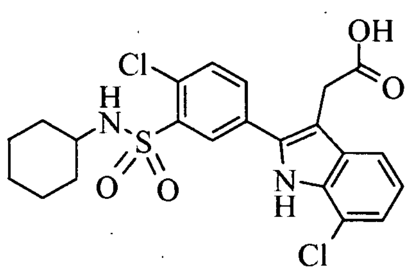

Step 1. Di-*tert*-butyl dicarbonate (7.92 g) is added to a solution of 7-chloroindole (5 g) and 4-(dimethylamino) pyridine (0.4 g) in DCM (165 mL). The reaction is stirred at room temperature for 18 hr. The reaction mixture is washed with 1N HCl (100 mL) and 1N $NHCO_3$ (100 mL). The organic layer is separated, dried over $MgSO_4$ and concentrated to afford 7-chloro-indole-1-carboxylic acid *tert*-butyl ester as an oil (8.22 g).

Step 2. To a solution of 7-chloro-indole-1-carboxylic acid *tert*-butyl ester (3 g) in dry THF (15 mL) is added triisopropyl borate (4.11 mL) under nitrogen. The mixture is cooled to 0°C in an ice bath. Lithium diisopropylamine (8.94 mL, 2 M) is added over an hour at 0°C. The reaction is stirred at 0°C for 30 minutes. 2N HCl (10 mL) is added to quench the reaction. The resulting mixture is extracted with EtOAc. The residue is purified by flash silica gel column chromatography eluting with 10% to 50% EtOAc in heptane to afford 1-(*tert*-butoxycarbonyl)-7-chloro-1H-indol-2-ylboronic acid as a solid (0.86 g).

Step 3. To a solution of 1-(*tert*-butoxycarbonyl)-7-chloro-1H-indol-2-ylboronic acid (860 mg), 5-bromo-2-chloro-N-cyclohexyl-benzenesulfonamide (733 mg) and CsF (632 mg) in dioxane-$H_2O$ (22 mL, 10:1) is added $PdCl_2(dppf)_2$ (163 mg) at room temperature under nitrogen. The reaction is heated to 80°C and stirred for overnight. The reaction mixture is concentrated *in vacuo*. The residue is dissolved in EtOAc and filter through a short silica column. The filtrate is concentrated *in vacuo* and purified by flash silica gel column chromatography eluting with 10% to 50% EtOAc in heptane to afford 7-chloro-2-(4-chloro-3-cyclohexylsulfamoyl-phenyl)-indole-1-carboxylic acid *tert*-butyl ester as a solid (630 mg).

Step 4. Trifluoacetic acid (3 mL) is added to a solution of 7-chloro-2-(4-chloro-3-cyclohexylsulfamoyl-phenyl)-indole-1-carboxylic acid *tert*-butyl ester (630 mg) in dichloromethane (7 mL). The reaction mixture is stirred at room temperature overnight. The mixture is concentrated *in vacuo*. The residue is dissolved in EtOAc and washed with 1N $NaHCO_3$. The organic layer is separated, dried over $MgSO_4$ and concentrated *in vacuo*. The crude is purified by flash silica gel column chromatography eluting with 10% to 40% EtOAc in heptane to afford 2-chloro-5-(7-chloro-1H-indol-2-yl)-N-cyclohexyl-benzenesulfonamide as a solid (386 mg).

Step 5. Oxalyl chloride (0.12 mL) is slowly added to a solution of 2-chloro-5-(7-chloro-1H-indol-2-yl)-N-cyclohexyl-benzenesulfonamide (386 mg) in dichloromethane (9 mL) at room temperature. After stirring for 18 hr, MeOH (3 mL) is added and stirred for 15 minutes. The mixture is concentrated. The residue is purified by flash silica gel column chromatography eluting with 5% to 45% EtOAc in heptane to afford [7-chloro-2-(4-chloro-3-cyclohexylsulfamoyl-phenyl)-1H-indol-3-yl]-oxo-acetic acid methyl ester as a solid (239 mg).

Step 6. Triethylsilane (0.15 mL) is slowly added to a solution of [7-chloro-2-(4-chloro-3-cyclohexylsulfamoyl-phenyl)-1H-indol-3-yl]-oxo-acetic acid methyl ester (239 mg) in trifluoacetic acid (2.4 mL) at room temperature. After stirring for overnight, the volatile is removed *in vacuo*. The residue is dissolved in EtOAc and washed with 1N $NaHCO_3$. The organic layer is separated, dried over $MgSO_4$ and concentrated. The residue is purified by flash silica gel column chromatography eluting with 10% to 50% EtOAc in heptane to afford [7-chloro-2-(4-chloro-3-cyclohexylsulfamoyl-phenyl)-1H-indol-3-yl]-acetic acid methyl ester as a solid (93 mg). LCMS: $R_T$ = 4.5 minutes, MS: 495 (M+H).

Step 7. To a solution of [7-chloro-2-(4-chloro-3-cyclohexylsulfamoyl-phenyl)-1H-indol-3-yl]-acetic acid methyl ester (93 mg) in MeOH/$H_2O$ (1:1, 4 mL) is added lithium hydroxide monohydrate (16 mg). The reaction mixture is stirred at 80°C for 18 hr. EtOAc (10 mL) is added and the solution is washed with 1N HCl (5 mL). The organic layer is separated, dried over $MgSO_4$ and concentrated to afford [7-chloro-2-(4-chloro-3-cyclohexylsulfamoyl-phenyl)-1H-indol-3-yl]-acetic acid as a solid (85 mg). LCMS: $R_T$ = 2.6 minutes, MS: 481 (M+H); $^1H$ NMR (300 MHz, DMSO) δ 1.09-1.35 (m, 5H), 1.59-1.73 (m, 5H), 3.19 (m; 1H), 3.84 (brs, 2H), 7.21 (m, 1H), 7.38 (m, 1H), 7.67 (m, 1H), 7.95 (m, 1H), 8.02-8.05 (m, 2H), 8.40 (brs, 1H), 11.9 (brs, 1H). $IC_{50}$ = 3.7 nM

Example 9:

2-Chloro-N-cyclohexyl-5-[3-(2-methanesulfonylamino-2-oxo-ethyl)-1H-indol-2-yl]-benzenesulfonamide

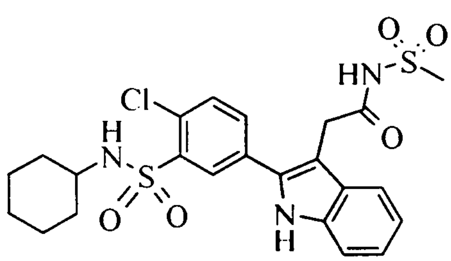

Step 1. To a solution of 1-(*tert*-butoxycarbonyl)-1H-indol-2-ylboronic acid (10 g), 5-bromo-2-chloro-N-cyclohexyl-benzensulfonamide (6.8 g) and CsF (5.8 g) in dioxane-$H_2O$ (220 mL, 10:1) is added $PdCl_2(dppf)_2$ (1.57 g) at room temperature under nitrogen. The reaction is heated to 80°C and stirred for 6 hours. The reaction mixture is concentrated *in vacuo*. The residue is dissolved in EtOAc and filter through a short silica column. The filtrate is concentrated *in vacuo* and purified by flash silica gel column chromatography eluting with 10% to 50% EtOAc in heptane to afford 2-(4-chloro-3-cyclohexylsulfamoyl-phenyl)-indole-1-carboxylic acid *tert*-butyl ester as a solid (8.2 g).

Step 2. Trifluoacetic acid (65 mL) is added to a solution of 2-(4-chloro-3-cyclohexylsulfamoyl-phenyl)-indole-1-carboxylic acid *tert*-butyl ester (13 g) in dichloromethane (150 mL). The reaction mixture is stirred at room temperature for 2 hr. The mixture is concentrated *in vacuo*. The residue is dissolved in EtOAc and washed with 1N $NaHCO_3$. The organic layer is separated, dried over $MgSO_4$ and concentrated to afford 2-chloro-5-(1H-indol-2-yl)-N-cyclohexyl-benzenesulfonamide as a solid (9.7 g). LCMS: $R_T$ = 3.17 minutes, MS: 389 (M+H).

Step 3. Oxalyl chloride (0.33 mL) is slowly added to a solution of 2-chloro-5-(1H-indol-2-yl)-N-cyclohexyl-benzenesulfonamide (1 g) in dichloromethane (25 mL) at room temperature. After stirring for 1.8 hr, MeOH (5 mL) is added and stirred for 15 minutes. The mixture is concentrated. The residue is purified by flash silica gel column chromatography eluting with 10% to 45% EtOAc in heptane to afford [2-(4-chloro-3-cyclohexylsulfamoyl-phenyl)-1H-indol-3-yl]-oxo-acetic acid methyl ester as a solid (1.2 g).

Step 4. Triethylsilane (0.59 mL) is slowly added to a solution of [2-(4-chloro-3-cyclohexylsulfamoyl-phenyl)-1H-indol-3-yl]-oxo-acetic acid methyl ester (1.2 g) in trifluoacetic acid (12 mL) at room temperature. After stirring for overnight, the volatile is removed *in vacuo*. The residue is dissolved in EtOAc and washed with 1N $NaHCO_3$. The organic layer is separated, dried over $MgSO_4$ and concentrated. The residue is purified by flash silica gel column chromatography eluting with 10% to 50% EtOAc in heptane to afford [2-(4-chloro-3-cyclohexylsulfamoyl-phenyl)-1H-indol-3-yl]-acetic acid methyl ester as a solid (818 mg).

Step 5. To a solution of [2-(4-chloro-3-cyclohexylsulfamoyl-phenyl)-1H-indol-3-yl]-acetic acid methyl ester (818 mg) in MeOH/$H_2O$ (1:1, 18 mL) is added lithium hydroxide monohydrate (149 mg). The reaction mixture is stirred at 80°C for 18 hr. EtOAc (15 mL) is added and the solution is washed with 1N HCl (10 mL). The organic layer is separated, dried over $MgSO_4$ and concentrated to afford [2-(4-chloro-3-cyclohexylsulfamoyl-phenyl)-1H-indol-3-yl]-acetic acid as a solid (740 mg).

Step 6. To a solution of [2-(4-chloro-3-cyclohexylsulfamoyl-phenyl)-1H-indol-3-yl]-acetic acid (185 mg), N-(3-dimethylaminopropyl)-N'-ethylcarbodiimide hydrochloride (82 mg) and dimethylamino pyridine (50 mg) in dichloromethane (4 mL) is added methanesulfonamide (41 mg) at 0°C. The reaction mixture is allowed to warm up to room temperature and stirred overnight. The resulting solution is concentrated *in vacuo*. The residue is dissolved in EtOAc and washed with 1N HCl. The organic layer is separated, dried over $MgSO_4$ and concentrated *in vacuo.* The crude is triturated with dichloromethane and filtered to afford 2-chloro-N-cyclohexyl-5-[3-(2-methanesulfonylamino-2-oxo-ethyl)-1H-indol-2-yl]-benzenesulfonamide as a solid (115 mg). LCMS: $R_T$ = 2.39 minutes, MS: 524 (M+H); $^1H$ NMR (300 MHz, DMSO) δ 1.10-1.28 (m, 5H), 1.61-1.64 (m, 5H), 3.07 (m, 1H), 3.26 (s, 3H), 3.88 (s, 2H), 7.10 (m, 1H), 7.21 (m, 1H), 7.44 (m, 1H), 7.61 (m, 1H), 7.82 (m, 1H), 7.98 (m, 2H), 8.25 (s, 1H), 11.65 (s, 1H), 12.12 (s, 1H). $IC_{50}$ = 2 nM

Example 10:

2-Chloro-N-cyclohexyl-5-[3-(2-ethanesulfonylamino-2-oxo-ethyl)-1H-indol-2-yl]-benzenesulfonamide

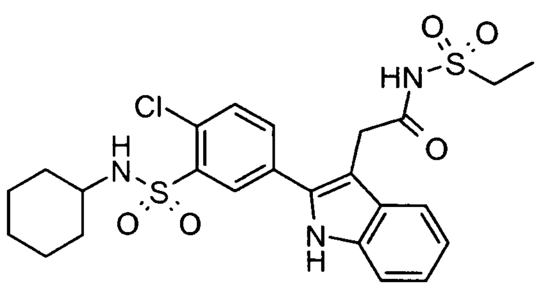

Step 1. To a solution of [2-(4-chloro-3-cyclohexylsulfamoyl-phenyl)-1H-indol-3-yl]-acetic acid (200 mg), N-(3-dimethylaminopropyl)-N'-ethylcarbodiimide hydrochloride (90 mg) and dimethylamino pyridine (55 mg) in dichloromethane (4.5 mL) is added ethanesulfonamide (51 mg) at 0°C. The reaction mixture is allowed to warm up to room temperature and stirred overnight. The resulting solution is concentrated *in vacuo*. The residue is dissolved in EtOAc and washed with 1N HCl. The organic layer is separated, dried over $MgSO_4$ and concentrated *in vacuo*. The crude is triturated with dichloromethane and filtered to afford 2-chloro-N-cyclohexyl-5-[3-(2-ethanesulfonylamino-2-oxo-ethyl)-1H-indol-2-yl]-benzenesulfonamide as a solid (174 mg). LCMS: $R_T$ = 2.44 minutes, MS: 538 (M+H); $^1H$ NMR (300 MHz, DMSO) δ 1.07-1.33 (m, 8H), 1.51-1.69 (m, 5H), 3.13 (m, 1H), 3.34 (m, 2H), 3.94 (s, 2H), 7.14 (t, J = 7.2 Hz, 1H), 7.26 (t, J = 7.2 Hz, 1H), 7.50 (d, J = 7.2 Hz, 1H), 7.67 (d, J = 7.2 Hz, 1H), 7.87 (d, J = 7.2 Hz, 1H), 8.00 (m, 1H), 8.34 (m, 1H), 11.70 (s, 1H), 12.06 (5, 1H). $IC_{50}$ = 2.7 nM

Example 11:

2-Chloro-N-cyclohexyl-5-[3-(2-oxo-2-trifluoromethanesulfonylamino-ethyl)-1H-indol-2-yl]-benzenesulfonamide

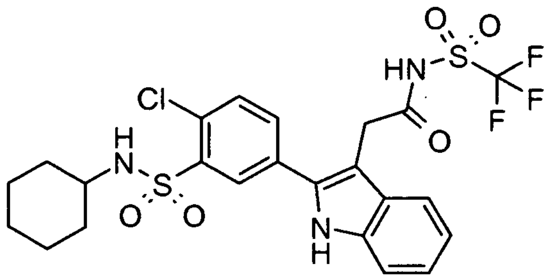

Step 1. To a solution of [2-(4-chloro-3-cyclohexylsulfamoyl-phenyl)-1H-indol-3-yl]-acetic acid (150 mg), N-(3-dimethylaminopropyl)-N'-ethylcarbodiimide hydrochloride (68 mg) and dimethylamino pyridine (40 mg) in dichloromethane (4 mL) is added trifluoromethanesulfonamide (52 mg) at 0˚C. The reaction mixture is allowed to warm up to room temperature and stirred overnight. The resulting solution is concentrated *in vacuo.* The residue is dissolved in EtOAc and washed with 1N HCl. The organic layer is separated, dried over $MgSO_4$ and concentrated *in vacuo* to afford 2-chloro-N-cyclohexyl-5-[3-(2-trifluoromethanesulfonylamino-2-oxo-ethyl)-1H-indol-2-yl]-benzenesulfonamide as a solid (206 mg). LCMS: $R_T$ = 2.58 minutes, MS: 576 (M+H); $^1H$ NMR (300 MHz, $CD_3OD$) δ 1.17-1.27 (m, 5H), 1.55-1.75 (m, 5H), 3.12 (m, 1H), 4.01 (s, 2H), 7.11 (m, 1H), 7.42 (m, 1H), 7.50 (m, I H), 7.68 (m, 1H), 7.80 (m, 1H), 8.3 (m, I H). $IC_{50}$ = 14 nM

Example 12:

2-[2-(4-Chloro-3-cyclohexylsulfamoyl-phenyl)-1H-indol-3-yl]-N-(1H-tetrazol-5-yl)-acetamide

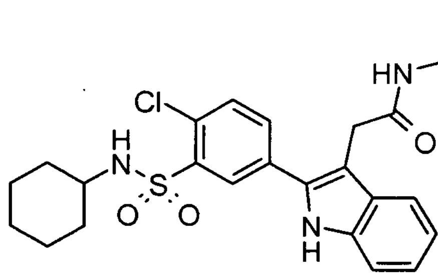

Step 1. To a solution of [2-(4-chloro-3-cyclohexylsulfamoyl-phenyl)-1H-indol-3-yl]-acetic acid (200 mg), N-(3-dimethylaminopropyl)-N'-ethylcarbodiimide hydrochloride (90 mg) and dimethylamino pyridine (55 mg) in dichloromethane (4.5 mL) is added 1H-tetrazol-5-ylamine (48 mg) at 0˚C. The reaction mixture is allowed to warm up to room temperature and stirred for 2 days. The resulting solution is concentrated *in vacuo.* The residue is dissolved in EtOAc and washed with 1N HCl. The organic layer is separated, dried over $MgSO_4$ and concentrated *in vacuo*. The crude is triturated with dichloromethane and filtered to afford 2-[2-(4-chloro-3-cyclohexylsulfamoyl-phenyl)-1H-indol-3-yl]-N-(1H-tetrazol-5-yl)-acetamide as a solid (50 mg). LCMS: $R_T$ = 2.26 minutes, MS: 514 (M+H); $^1H$ NMR (300 MHz, DMSO) δ 1.14-1.3 (m, 5H), 1.51-1.65 (m, 5H), 3.1 (m, 1H), 4.1 (s, 2H), 7.11 (m, 1H), 7.23 (m, 1H), 7.48 (m, 1H), 7.7 (m, 1H), 7.84 (m, 1H), 8.06 (m, 2H), 8.34 (s, 1H), 11.69 (brs, 1H), 12.46 (brs, 1H). $IC_{50}$ = 15 nM

Example 13:

[2-(3-cyclohexylsulfamoyl-4-ethyl-phenyl)-1H-indol-3-yl]-acetic acid

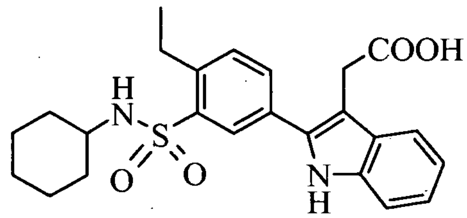

Step 1. 1-Bromo-4-ethyl-benzene (3 g) is dissolved in 30 mL of DCM and cooled to 0˚C in an ice bath. Chlorosulfonic acid (11.3 g) is added dropwise over the course of 20 minutes and the solution is stirred at 0˚C for 4 hours. The reaction mixture is poured cautiously onto ice and allowed to warm to room temperature. The mixture is transferred to a separatory funnel and the layers separated. The aqueous layer is washed with additional DCM. The organic layers are combined, dried ($MgSO_4$), filtered, and evaporated to afford 5-bromo-2-ethyl-benzenesulfonyl chloride (1.78 g) as an oil which is used without further purification in step 2.

Step 2. Cyclohexylamine (0.9 g) and diisopropylethylamine (1.5 g) are dissolved in 20 mL of DCM and the solution is cooled to 0˚C. To this is added 5-bromo-2-ethyl-benzenesulfonyl chloride (1.7 g in 20 mL of DCM) in portions over 5 minutes. The mixture is stirred at 0˚C for 30 minutes and at room temperature for 1 hour. The solvent is removed under reduced pressure and to the residue is added 10% aqueous HCl and DCM. The layers are separated and the aqueous layer is washed with additional DCM. The combined DCM layers are dried ($MgSO_4$), filtered and evaporated. The resulting solid is recrystallized from DCM/heptane to afford 5-bromo-N-cyclohexyl-2-ethyl-benzenesulfonamide (1.36 g). LCMS: $R_T$ = 2.96 minutes, MS: 346 (M+H).

Step 3. 5-Bromo-N-cyclohexyl-2-ethyl-benzenesulfonamide (1.3 g), 1-Boc-indole-2-boronic acid (1.48 g), and cesium fluoride (0.86 g) are mixed with 10:1 dioxane:$H_20$ (44 mL). The solution is degassed with nitrogen and $PdCl_2(dppf)_2$ (0.31 g) is added. The mixture is heated to 80˚C for 2.5 hours. The reaction mixture is poured into $H_2O$. EtOAc is added and the layers are separated. The EtOAc layer is concentrated. Heptane is added to afford a precipitate which is removed by filteration. The EtOAc filtrate is passed through a plug of silica, evaporated onto silica and purified on an 80 g silica gel column using an ISCO Companion purification system (EtOAC/heptane gradient) to afford 2-(3-Cyclohexylsulfamoyl-4-ethyl-phenyl)-indole-1-carboxylic acid *tert*-butyl ester (1.19 g). LCMS: $R_T$ = 3.54 minutes, MS: 483 (M+H).

Step 4. 2-(3-Cyclohexylsulfamoyl-4-ethyl-phenyl)-indole-1-carboxylic acid *tert*-butyl ester (1.18 g) is treated with 10 mL of TFA for 30 minutes at room temperature. TFA is removed under reduced pressure. The residue is partitioned between EtOAc and 10% aqueous $NaHCO_3$ and the layers are separated. The organic layer is washed with additional 10% aqueous $NaHCO_3$, water, and brine. The organic layer is dried ($MgSO_4$), filtered, evaporated onto silica and purified on a 40 g silica gel column using an ISCO Companion purification system (EtOAC/heptane gradient) to afford N-cyclohexyl-2-ethyl-5-(1H-indol-2-yl)-benzenesulfonamide (0.65 g). LCMS: $R_T$ = 3.07 minutes, MS: 383 (M+H).

Step 5. N-Cyclohexyl-2-ethyl-5-(1H-indol-2-yl)-benzenesulfonamide (0.64 g) is suspended in 30 mL of diethyl ether. Oxalyl chloride (0.32 g) is added dropwise at room temperature and the mixture is stirred for 6 hours. Methanol (2 mL) is added, the solution is stirred for 10 minutes, and the solvent is removed under reduced pressure. The crude material is purified on an 80 g silica gel column using an ISCO Companion purification system (EtOAc/heptane gradient) to afford [2-(3-cyclohexylsulfamoyl-4-ethyl-phenyl)-4H-indol-3-yl]oxo-acetic acid methyl ester (0.66 g). LCMS: $R_T$ = 2.75 minutes, MS: 469 (M+H).

Step 6. [2-(3-Cyclohexylsulfamoyl-4-ethyl-phenyl)-1H-indol-3-yl]oxo-acetic acid methyl ester (0.63 g) is dissolved in 10 mL of TFA. Triethylsilane (0.31 g) is added dropwise at room temperature and the solution is stirred for 18 hours. The reaction mixture is concentrated under reduced pressure. To the residue is added EtOAc and saturated $NaHCO_3$ and the layers are separated. The EtOAc layer is evaporated onto silica gel and purified on a 40 g silica gel column using an ISCO Companion purification system (EtOAc/heptane gradient) to afford [2-(3-cyclohexylsulfamoyl-4-ethyl-phenyl)-1H-indol-3-yl]-acetic acid methyl ester (0.53 g). LCMS: $R_T$ = 2.95 minutes, MS: 455 (M+H); $^1H$ NMR (300 MHz, $CDCl_3$) δ $^1H$ NMR (300 MHz, $CDCl_3$) δ 1.05-1.29 (m, 5H), 1.37 (t, J = 7.5 Hz, 3H), 1.50-1.79 (m, 5H), 3.14 (q, J = 7.5 Hz, 2H), 3.22 (m, 1H), 3.73 (s, 3H), 3.84 (s, 2H), 4.55 (d, J = 7.9 Hz, 1H), 7.16-7.28 (m, 2H), 7.41 (d, J = 8.1 Hz, 1H), 7.51 (d, J = 8.1 Hz, 1H), 7.69 (d, J = 7.7 Hz, 1H), 7.83 (dd, J = 7.9, 1.8 Hz, 1H), 8.28 (d, J = 1.9 Hz, 1H), 8.32 (s, 1H).

Step 7. [2-(3-Cyclohexylsulfamoyl-4-ethyl-phenyl)-1H-indol-3-yl]-acetic acid methyl ester (0.33 g) is dissolved

in 6 mL of 3:3:1 MeOH:THF:$H_2O$. LiOH monohydrate (2 equiv.) is added and the solution is heated to 80 ˚C overnight. The solvent is evaporated under reduced pressure. EtOAc and 10% aq. HCl are added and the layers are separated. The EtOAc layer is washed with additional 10% aq. HCl, water, and brine. The organic layer is dried ($MgSO_4$), filtered and evaporated and the residue is recrystallized from DCM/heptane to afford [2-(3-cyclohexylsulfamoyl-4-ethyl-phenyl)-1H-indol-3-yl]-acetic acid as a solid (193 mg). LCMS: $R_T$ = 2.6 minutes, MS: 441 (M+H); $^1$H NMR (300 MHz, DMSO-$D_6$) δ 1.0-1.24 (m, 5H), 1.31 (t, J = 7.5 Hz, 3H), 1.45-1.62 (m, 5H), 3.05 (m, 1H), 3.08 (q, J = 7.4 Hz, 2H), 3.75 (s, 2H), 7.07 (t, J = 7.8 Hz, 1H), 7.18 (t, J = 7.7 Hz, 1H), 7.42 (d, J = 8 Hz, 1H), 7.59 (m, 2H), 7.76 (d, J = 7.9 Hz, 1H), 7.87 (d, J = 7.9 Hz, 1H), 8.18 (s, 1H), 11.46 (s, 1H), 12.37 (s, 1H). $IC_{50}$ = 0.5 nM

Example 14:

2-[2-(4-Chloro-3-cyclohexylsulfamoyl-phenyl)-1H-indol-3-yl]-propionic acid

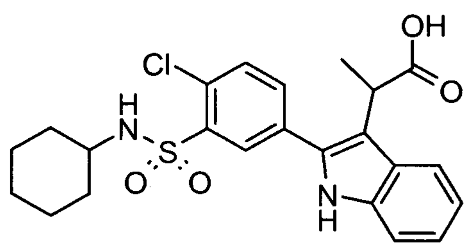

Step 1. To a solution of 1-(*tert*-butoxycarbonyl)-indol-2-ylboronic acid (5.5 g), 5-bromo-2-chloro-N-cyclohexylbenzenesulfonamide (5 g) and CsF (4.3 g) in dioxane-$H_2O$ (143 mL, 10:1) is added $PdCl_2(dppf)_2$ (1.16 g) at room temperature under nitrogen. The reaction is heated to 80˚C and stirred for 18 hours. The reaction mixture is concentrated *in vacuo.* The residue is dissolved in EtOAc and filter through a short silica column. The filtrate is concentrated *in vacuo* and purified by flash silica gel column chromatography eluting with 5% to 30% EtOAc in heptane to afford 2-(4-chloro-3-cyclohexylsulfamoyl-phenyl)-indole-1-carboxylic acid *tert*-butyl ester as a solid (6.2 g). LCMS: $R_T$ = 5.03 minutes, MS: 511 (M+Na).

Step 2. Trifluoacetic acid (10 mL) is added to a solution of 2-(4-chloro-3-cyclohexylsulfamoyl-phenyl)-indole-1-carboxylic acid *tert*-butyl ester (6.2 mg) in dichloromethane (20 mL). The reaction mixture is stirred at room temperature overnight. The mixture is concentrated *in vacuo.* The residue is dissolved in EtOAc and washed with 1N $NaHCO_3$. The organic layer is separated, dried over $MgSO_4$ and concentrated to afford 2-chloro-N-cyclohexyl-5-(1H-indol-2-yl)-benzenesulfonamide as a solid (5.3 g).

Step 3. Oxalyl chloride (1.59 mL) is slowly added to a solution of 2-chloro-N-cyclohexyl-5-(1H-indol-2-yl)-benzenesulfonamide (4.8 mg) in dichloromethane (120 mL) at room temperature. After stirring for 3 hr, MeOH (10 mL) is added and stirred for 15 minutes. The mixture is concentrated. The residue is purified by flash silica gel column chromatography eluting with 5% to 50% EtOAc in heptane to afford [2-(4-chloro-3-cyclohexylsulfamoyl-phenyl)-1H-indol-3-yl]-oxo-acetie acid methyl ester as a solid (2.5 g).

Step 4. Triethylsilane (1.7 mL) is slowly added to a solution of [2-(4-chloro-3-cyclohexylsulfamoyl-phenyl)-1H-indol-3-yl]-oxo-acetic acid methyl ester (2.5 g) in trifluoacetic acid (25 mL) at room temperature. After stirring for overnight, the volatile is removed *in vacuo.* The residue is dissolved in EtOAc and washed with 1N $NaHCO_3$. The organic layer is separated, dried over $MgSO_4$ and concentrated. The residue is purified by flash silica gel column chromatography eluting with 10% to 50% EtOAc in heptane to afford [2-(4-chloro-3-cyclohcxylsulfamoyl-phenyl)-1H-indol-3-yl]-acetic acid methyl ester as a solid (1.84 g). LCMS: $R_T$ = 4.14 minutes, MS: 461 (M+H).

Step 5. Di-*tert*-butyl dicarbonate (807 mg) is added to a solution of [2-(4-chloro-3-cyclohexylsulfamoyl-phenyl)-1H-indol-3-yl]-acetic acid methyl ester (775 mg) triethylamine (0.52 mL) and 4-(dimethylamino)pyridine (42 mg) in DCM (17 mL). The reaction is stirred at room temperature for 2 days. The reaction mixture is washed with 1N HCl (10 mL) and 1N $NHCO_3$ (10 mL). The organic layer is separated, dried over $MgSO_4$ and concentrated to afford 2-[4-chloro-3-(N-tert-butyloxycarbonyl)-cyclohexylsulfamoyl-phenyl]-3-methoxycarbonylmethyl-indole-1-carboxylic acid *tert*-butyl ester (1.03 g).

Step 6. To a solution of 2-[4-chloro-3-(N-tert-butyloxycarbonyl)-cyclohexylsulfamoyl-phenyl]-3-methoxycarbonylmethyl-indole-1-carboxylic acid *tert*-butyl ester (864 mg) in DMF (13 mL) is added NaH (157 mg) in portion at 0˚C.

The resulting mixture is stirred at 0˚C for 15 minutes and MeI (0.82 mL) is added at 0˚C. The reaction mixture is allowed to warm up to room temperature and stirred for 3 hr. The reaction is quenched by adding saturated $NH_4Cl$ (10 mL). The mixture is extracted with EtOAc (20 mL). The organic layer is washed with water (10 mL) 3 times, separated, dried over $MgSO_4$ and concentrated. The residue is purified by flash silica gel column chromatography eluting with 10% to 45% EtOAc in heptane to afford 2-[4-chloro-3-(N-*tert*-butyloxycarbonyl)-cyclohexylsulfamoyl-phenyl]-3-(1-methoxycarbonyl-ethyl)-indole-1-carboxylic acid tert-butyl ester as a white solid (400 mg). LCMS: $R_T$ = 4.3 minutes, MS: 675 (M+H).

Step 7. Trifluoacetic acid (2 mL) is added to a solution of 2-[4-chloro-3-(N-tert-butyloxycarbonyl)-cyclohexylsulfamoyl-phenyl]-3-(1-methoxycarbonyl-ethyl)-indole-1-carboxylic acid *tert*-butyl ester (165 mg) in dichloromethane (4 mL). The reaction mixture is stirred at room temperature for 4 hours. The mixture is concentrated *in vacuo.* The residue is dissolved in EtOAc and washed with 1N $NaHCO_3$. The organic layer is separated, dried over $MgSO_4$ and concentrated. The residue is purified by flash silica gel column chromatography eluting with 10% to 50% EtOAc in heptane to afford 2-[2-(4-chloro-3-cyclohexylsulfamoyl-phenyl)-1H-indol-3-yl]-propionic acid methyl ester as a white solid (94 mg). LCMS: $R_T$ = 3.2 minutes, MS: 475 (M+H).

Step 8. To a solution of 2-[2-(4-chloro-3-cyclohexylsulfamoyl-phenyl)-1H-indol-3-yl]-propionic acid methyl ester (94 mg) in MeOH/$H_2O$ (1:1, 2 mL) is added lithium hydroxide monohydrate (17 mg). The reaction mixture is stirred at 80˚C for 2 hr. EtOAc (10 mL) is added and the solution is washed with 1N HCl (5 mL). The organic layer is separated, dried over $MgSO_4$ and concentrated to afford 2-[2-(4-chloro-3-cyclohexylsulfamoyl-phenyl)-1H-indol-3-yl]-propionic acid as a solid (90 mg). LCMS: $R_T$ = 2.88 minutes, MS: 461 (M+H); $^1H$ NMR (300 MHz, DMSO) δ 1.15-1.37 (m, 5H), 1.51-1.71 (m, 8H), 3.13 (m, 1H), 4.06 (m, 1H), 7.02 (t, J = 7.2 Hz, 1H), 7.14 (t, J = 6.9 Hz, 1H), 7.38 (d, J = 8.1 Hz, 1H), 7.67 (m, 2H), 7.84 (d, J = 8.1 Hz, 1H), 8.37 (s, 1H). $IC_{50}$ = 5.3 nM

Example 15:

{2-[4-Chloro-3-(3-chloro-phenylmethanesulfonyl)-phenyl]-1H-indol-3-yl}-acetic acid

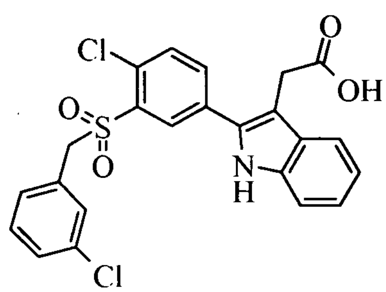

Step 1. Sodium sulfite (1.7 g) and sodium phosphate dibasic (0.98 g) are dissolved in 20 mL of water and heated to 30˚C until all is in solution. 5-Bromo-2-chloro-benzenesulfonyl chloride (2 g) is added and the reaction mixture is heated to 60˚C overnight. The reaction mixture is cooled and 1-bromomethyl-3-chlorobenzene (1.4 g) is added dropwise as a solution in 20 mL of acetone. The mixture is heated to 60˚C for 2 hours and cooled to room temperature. The reaction mixture is partitioned between EtOAc and water and the layers are separated. The aqueous layer is washed with additional EtOAc. The combined organic layers are washed with water and brine. The organic layer is dried ($MgSO_4$), filtered and evaporated. The crude material is recrystallized from EtOAc/heptane to afford 4-bromo-1-chloro-2-(3-chloro-phenylmethanesulfonyl)-benzene (1.47 g). LCMS: $R_T$ = 2.8 minutes, MS: 379 (M+Na), $^1H$ NMR (300 MHz, $CDCl_3$) δ 4.37 (s, 2H), 6.70 (brs, 1H), 7.15-7.35 (m, 6H), 7.73 (d, J = 2 Hz, 1H).

Step 2. To a solution of 4-bromo-1-chloro-2-(3-chloro-phenylmethanesulfonyl)-benzene (1 g), 1-(*tert*-butoxycarbonyl)-indol-2-ylboronic acid (1 g), and cesium fluoride (0.6 g) in 22 mL of 10:1 dioxane:water is added $PdCl_2(dppf)_2$ (0.216 g) at room temperature under nitrogen. The reaction is heated to 80˚C overnight. After cooling, the reaction mixture is poured into water and extracted with EtOAc. The organic layer is concentrated and heptane is added to afford a precipitate that is filtered. The filtrate is passed through a plug of silica and then evaporated onto silica gel. The crude material is purified by flash silica gel chromatography (EtOAc/heptane) to afford 2-[4-chloro-3-(3-chloro-phenylmethanesulfonyl)-phenyl]-indole-1-carboxylic acid *tert*-butyl ester (0.84 g). LCMS: $R_T$ = 3.42 minutes, MS: 516 (M+Na).

Step 3. Trifluoroacetic acid (10 mL) is added to 2-[4-chloro-3-(3-chlorophenylmethanesulfonyl)-phenyl]-indole-1-carboxylic acid tert-butyl ester (0.79 g) and the resulting solution is mixed for 35 minutes at room temperature. The mixture is concentrated *in vacuo.* The residue is dissolved in EtOAc and washed with 10% $NaHCO_3$. The organic layer

is dried ($MgSO_4$), filtered, evaporated onto silica gel, and purified by flash silica gel chromatography (EtOAc/heptane) to afford 2-[4-chloro-3-(3-chloro-phenylmethanesulfonyl)-phenyl]-1H-indole (0.49 g). LCMS: $R_T$ = 3 minutes, MS: 416 (M+Na).

Step 4. To a suspension of 2-[4-chloro-3-(3-chloro-phenylmethanesulfonyl)-phenyl]-1H-indole (0.48 g) in 25 mL of $Et_2O$ is added oxalyl chloride (0.22 g) dropwise at room temperature. After 7 hours, additional oxalyl chloride (0.22 g) is added and the mixture is stirred overnight. Methanol (2 mL) is added dropwise and the mixture is stirred for 10 minutes. The reaction mixture is poured into water and extracted with EtOAc. The organic layer is washed with aqueous $NaHCO_3$ and brine. The organic layer is dried ($Na_2SO_4$), filtered, and evaporated onto silica gel. The crude material is purified by flash silica gel chromatography (EtOAc/heptane) to afford {2-[4-chloro-3-(3-chlorophenylmethanesulfonyl)-phenyl]-1H-indol-3-yl}-oxo-acetic acid methyl ester (0.43 g). LCMS: $R_T$ = 2.73 minutes, MS: 502 (M+Na).

Step 5. Triethylsilane (0.23 g) is added dropwise to a solution of {2-[4-chloro-3-(3-chlorophenylmethanesulfonyl)-phenyl]-1H-indol-3-yl}-oxo-acetic acid methyl ester (0.5 g) in 10 mL of trifluoroacetic acid. After stirring for 5 hours the reaction mixture is concentrated under reduced pressure: The residue is partitioned between EtOAc and sat. $NaHCO_3$. The organic layer is evaporated onto silica gel and purified by flash silica gel chromatography (EtOAc/heptane) to afford {2-[4-chloro-3-(3-chloro-phenylmethanesulfonyl)-phenyl]-1H-indol-3-yl}-acetic acid methyl ester (0.34 g). LCMS: $R_T$ = 2.86 minutes, MS: 488 (M+Na).

Step 6. To a solution of {2-[4-Chloro-3-(3-chloro-phenylmethanesulfonyl)-phenyl]-1H-indol-3-yl}-acetic acid methyl ester (0.3 g) in 14 mL of 3:3:1 THF:MeOH:$H_2O$ is added lithium hydroxide (0.077 g). The solution is stirred at 80˚C overnight. The solvent is removed under reduced pressure and 10% aqueous HCl is added to the residue. The aqueous layer is extracted twice with EtOAc. The combined organic layers are dried ($MgSO_4$), filtered, and evaporated to afford {2-[4-chloro-3-(3-chloro-phenylmethanesulfonyl)-phenyl]-1H-indol-3-yl}-acetic acid (210 mg). LCMS: $R_T$ = 2.43 minutes, MS: 474.1 (M+Na). $^1$H NMR (300 MHz, DMSO) δ 3.61(s, 2H), 4.97 (s, 2H), 7.08 (t, J = 7.2 Hz, 1H), 7.21 (m, 2H), 7.34-7.44 (m, 4H), 7.57 (d, J = 7.9 Hz, 1H), 7.95-8.05 (m, 2H), 8.37 (d, J = 2 Hz, 1H), 11.58 (s, 1H), 12.43 (s, 1H). $IC_{50}$ = 106 nM

Example 16:

{2-[4-Chloro-3-(3-chloro-phenylmethanesulfonylamino)-phenyl]-1H-indol-3-yl}-acetic acid

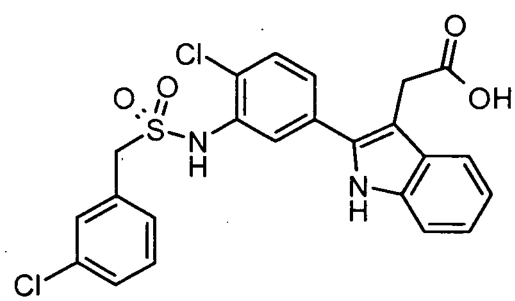

Step 1. To 5-bromo-2-chloro-phenylamine hydrochloride salt (0.81 g) in 20 mL of DCM is added $Et_3N$ (0.85 g) and the solution is cooled to 0˚C. 3-Chlorophenylmethanesulfonyl chloride (0.75 g) is added in portions as a solution in 5 mL of DCM. The mixture is allowed to warm to room temperature and is stirred overnight. The solvent is removed under reduced pressure and the residue is redissolved in EtOAc. The EtOAc is extracted with 10% aqueous HCl, saturated $Na_2CO_3$, and brine. The organic layer is dried ($MgSO_4$), filtered, evaporated onto silica gel and purified by flash silica gel chromatography (EtOAc/heptane) to afford N-(5-bromo-2-chloro-phenyl)-C-(3-chloro-phenyl)-methanesulfonamide (1.56 g). LCMS: $R_T$ = 2.77 minutes, MS: 394 (M+Na).

Step 2. To a solution of N-(5-bromo-2-chloro-phenyl)-C-(3-chloro-phenyl)-methanesulfonamide.(0.6 g), 1-(*tert*-butoxycarbonyl)-indol-2-ylboronic acid (0.6 g), and cesium fluoride (0.35 g) in 10:1 1 dioxane:water (11 mL) is added $PdCl_2(dppf)_2$ (0.125 g) under nitrogen. The mixture is heated to 80˚C for 3 hours. After cooling, the reaction mixture is poured into water and extracted with EtOAc. The organic layer is concentrated and heptane is added to afford a precipitate that is filtered. The filtrate is passed through a plug of silica and evaporated onto silica gel. The crude material is purified by flash silica gel chromatography (EtOAc/heptane) to afford 2-[4-chloro-3-(3-chloro-phenylmethanesulfonylamino)-phenyl]-indole-1-carboxylic acid *tert*-butyl ester (0.73 g). LCMS: $R_T$ = 3.36 minutes, MS: 531 (M+Na).

Step 3. Trifluoroacetic acid (10 mL) is added to 2-[4-chloro-3-(3-chloro-phenylmethanesulfonylamino)-phenyl]-indole-1-carboxylic acid tert-butyl ester (0.70 g) and the resulting solution is stirred at room temperature for 1 hour. The mixture is concentrated *in vacuo*. The residue is dissolved in EtOAc and washed with 10% $NaHCO_3$. The organic layer is dried ($MgSO_4$), evaporated onto silica gel, and purified by flash silica gel chromatography (EtOAc/heptane) to afford N-[2-Chloro-5-(1H-indol-2-yl)-phenyl]-C-(3-chloro-phenyl)-methanesulfonamide (0.56 g). LCMS: $R_T$ = 2.93 minutes, MS: 431 (M+Na).

Step 4. To a suspension of N-[2-chloro-5-(1H-indol-2-yl)-phenyl]-C-(3-chloro-phenyl)-methanesulfonamide (0.51 g) in 30 mL of DCM is added oxalyl chloride (0.23 g) dropwise at room temperature. After stirring for 2 hours, methanol (2 mL) is added dropwise and the mixture is stirred for 10 minutes. The reaction mixture is then evaporated onto silica gel and purified by flash silica gel chromatography (EtOAc/heptane) to afford {2-[4-chloro-3-(3-chloro-phenylmethanesulfonylamino)-phenyl]-1H-indol-3-yl}-oxo-acetic acid methyl ester (0.46 g). LCMS: $R_T$ = 2.67 minutes, MS: 517 (M+Na).

Step 5. Triethylsilane (0.19 g) is added dropwise to a solution of {2-[4-chloro-3-(3-chloro-phenylmethanesulfonylamino)-phenyl]-1H-indol-3-yl}-oxo-acetic acid methyl ester (0.42 g) in 10 mL of trifluoroacetic acid. The mixture is stirred for 6 hours. Additional triethylsilane (0.1 g) is added and the solution is stirred overnight at room temperature. The mixture is then concentrated under reduced pressure and the residue is partitioned between EtOAc and sat. $NaHCO_3$. The organic layer is evaporated onto silica gel and purified by flash silica gel chromatography (EtOAc/heptane) to afford {2-[4-chloro-3-(3-chloro-phenylmethane-sulfonylamino)-phenyl]-1H-indol-3-yl}-acetic acid methyl ester (0.3 g). LCMS: $R_T$ = 2.86 minutes, MS: 503 (M+Na).

Step 6. To a solution of {2-[4-chloro-3-(3-chloro-phenylmethanesulfonylamino)-phenyl]-1H-indol-3-yl}-acetic acid methyl ester (0.24 g) in 3:3:1 THF:MeOH:$H_2O$ (14 mL) is added lithium hydroxide (0.041 g). The solution is stirred at 80˚C overnight. An additional 2 equivalents of lithium hydroxide is added and heating is continued for 6 hrs until the reaction is complete. The solvent is removed under reduced pressure and 10% aqueous HCl is added to the residue. The mixture is extracted twice with EtOAc. The combined organic layers are dried ($MgSO_4$), filtered, evaporated onto silica gel and purified by flash silica gel chromatography (EtOAc/heptane) to afford {2-[4-chloro-3-(3-chloro-phenylmethanesulfonylamino)-phenyl}-1H-indol-3-yl}-acetic acid (186 mg). LCMS: $R_T$ = 2.53 minutes, MS: 489 (M+Na). $^1H$ NMR (300 MHz, DMSO) δ 3.78 (s, 2H), 4.67 (s, 2H), 7.07 (t, J = 7.2 Hz, 1H), 7.19 (t, J = 7.3 Hz, 1H), 7.40-7.46 (m, 4H), 7.51 (s, 1H), 7.57 (m, 2H), 7.70 (d, J = 8.2 Hz, 1H), 7.83 (s, 1H), 9.75 (s, 1H), 11.44 (s, 1H), 12.44 (s, 1H). IC50 = 12 nM

PHARMACOLOGICAL TESTING

The inhibitory effects of the compounds according to the invention are assessed in a human DP functional assay. A cAMP assay is employed using the human cell line LS174T, which expresses the endogenous DP receptor. The protocol is similar to that described previously (Wright DH, Ford-Hutchinson AW, Chadee K, Metters KM, The human prostanoid DP receptor stimulates mucin secretion in LS174T cells, Br J Pharmacol. 131(8):1537-45 (2000)).

Protocol for SPA cAMP Assay in Human LS174 T Cells

Materials

- PGD2 (Cayman Chemical Cat#12010)
- IBMX (Sigma Cat# 5879)
- cAMP SPA direct screening assay system (Amersham code RPA 559)
- 96-well cell plates (Wallac Cat# 1450-516)
- Wallac 1450 Microplate Trilux scintillation counter (PerkinElmer)
- Plate sealers
- Eppendorf tubes
- Dulbecco's Phosphate-Buffered Saline (PBS) (Invitrogen Cat#14040-133)
- Distilled water
- Vortex
- Magnetic stirrer and stirrer bars

Reagent Preparation:

All reagents should be allowed to equilibrate to room temperature before reconstitution.

1X assay buffer

Transfer the contents of the bottle to a 500 mL graduated cylinder by repeated washing with distilled water. Adjust the final volume to 500 mL with distilled water and mix thoroughly.

Lysis reagent 1 & 2

Dissolve each of the lysis reagents 1 and 2 in 200 mL assay buffer respectively. Leave at room temperature for 20 minutes to dissolve.

SPA anti-rabbit beads

Add 30 mL of lysis buffer 2 to the bottle. Gently shake the bottle for 5 minutes.

Antiserum

Add 15 mL of lysis buffer 2 to each vial, and gently mix until the contents are completely dissolved.

Tracer ($I^{125}$-cAMP)

Add 14 mL lysis buffer 2 to each vial and gently mix until the contents are completely dissolved.

Preparation of immunoreagent

1) Add equal volumes of tracer, antiserum and SPA anti-rabbit reagent to a bottle, ensuring that a sufficient volume of this mixture is prepared for the desired number of wells (150 μL/well).
2) Mix thoroughly.
3) This immunoreagent solution should be freshly prepared before each assay and not reused.

Standard

1) Add 1 mL lysis buffer 1 and gently mix until contents are completely dissolved.
2) The final solution contains cAMP at a concentration of 512 pmol/mL.
3) Label 7 polypropylene or polystyrene tubes, 0.2 pmol, 0.4 pmol, 0.8 pmol, 1.6 pmol, 3.2 pmol, 6.4 pmol and 12.8 pmol.
4) Pipette 500 μL of lysis buffer 1 into all the tubes.
5) Into the 12.8 pmol tube pipette 500 μL of stock standard (512 pmol/mL) and mix thoroughly. Transfer 500 μL from 12.8 pmol tube to the 6.4 pmol tube and mix thoroughly. Repeat this doubling dilution successively with the remaining tubes.
6) 50 μL aliquots in duplicate from each serial dilution and the stock standard will give rise to 8 standard levels of cAMP ranging from 0.2-25.6 pmol standard

Compound dilution buffer

Add 50 μL of 1 mM IBMX into 100 mL PBS to make a final concentration of 100 μM and sonicate at 30° C for 20 minutes.

PGD2 preparation

Dissolve 1 mg PGD2 (FW, 352.5) in 284 μL DMSO to make 10 mM stock solution and store at 20°C. Before each assay, it is freshly prepared. Add 3 μL of 10 mM stock solution to 20 mL DMSO, mix it thoroughly, and transfer 10 mL to 40 mL PBS.

Compound Dilution

Compound dilution is carried out in Biomex 2000 (Beckman) using Method 1_cAMP DP 11 points.

5 μL of each compound from the 10 mM stock compound plates is transferred to the wells of a 96-well plate respectively as below.

| | 1 | 2 | 3 | 4 | 5 | 6 | 7 | 8 | 9 | 10 | 11 | 12 |
|---|---|---|---|---|---|---|---|---|---|---|---|---|
| A | 1 | | | | | | | | | | | |
| B | 2 | | | | | | | | | | | |
| C | 3 | | | | | | | | | | | |
| D | 4 | | | | | | | | | | | |
| E | 5 | | | | | | | | | | | |
| F | 6 | | | | | | | | | | | |
| G | 7 | | | | | | | | | | | |
| H | reference | | | | | | | | | | | |

Fill the plate with 45 μL of DMSO except column 7 is filled with 28 μL DMSO. Pipette column 1 thoroughly, and transfer 12 μL into column 7 parallel. Perform 1:10 serial dilution from column 1 to column 6 and from column 7 to column 11 by transfer 5 μL to 45 μL DMSO to make following concentrations:

| First plate | Final concentration |
|---|---|
| Column 12 | 0 |
| Column 11 | 0.03 μM |
| Column 10 | 0.3 μM |
| Column 9 | 3 μM |
| Column 8 | 0.03 mM |
| Column 7 | 0.3 mM |
| Column 6 | 0.01 μM |
| Column 5 | 0.1 μM |
| Column 4 | 1 μM |
| Column 3 | 0.01 mM |
| Column 2 | 0.1 mM |
| Column 1 | 1 mM |

Fill a new 96-well plate with 247.5 μL of compound dilution buffer. Transfer 2.5 μL of serially diluted compounds from above plate to the new plate (1:100 dilution) as following:

| First plate | Second plate | Final concentration |
|---|---|---|
| Column 12 | Column 1 | 0 |
| Column 6 | Column 2 | 0.1 nM |
| Column 11 | Column 3 | 0.3 nM |
| Column 5 | Column 4 | 1 nM |
| Column 10 | Column 5 | 3 nM |
| Column 4 | Column 6 | 0.01 μM |

(continued)

| First plate | Second plate | Final concentration |
|---|---|---|
| Column 9 | Column 7 | 0.03 μM |
| Column 3 | Column 8 | 0.1 μM |
| Column 8 | Column 9 | 0.3 μM |
| Column 2 | Column 10 | 1 μM |
| Column 7 | Column 11 | 3 μM |
| Column 1 | Column 12 | 10 μM |

Cell Growth

1. LS174 T are always grown in MEM (ATCC Cat# 30-2003), 10% FBS (ATCC Cat# 30-2020) and additional 2 mM L-glutamine, at 37˚C and 5% $CO_2$.
2. Warm 0.05% Trypsin and Versine (Invitrogen Cat# 25300-054) at 37˚C water bath.
3. Remove growth medium from cells. Cells in T165 flask are washed twice with 4 mL Trypsin followed by incubation at 37˚C and 5% $CO_2$ for 3 minutes.
4. Add 10 mL of medium and pipette thoroughly to separate the cells and count the cells.
5. Bring the cell density to 2.25 x $10^5$ cells/ml and seed 200 μL cells/well (45,000 cells/well) in 96-well plates 1 day before the assay.

Assay Procedure

Day 1 Seed 45,000 cells/well in 200 μL medium in 96-well plates. Incubate the cell plate at 37˚ C, 5% $CO_2$ and 95% humidity overnight.

Day 2 1. Perform compound dilution.
2. Prepare assay buffer, lysis buffer 1 & 2, $PGD_2$ and standard.
3. Aspirate media from the cells and add 100 μL of compound solution using Zymark Sciclone-ALH/FD protocol cAMP DP.
4. Incubate the cells at 37˚C, 5% $CO_2$ and 95% humidity for 15 minutes.
5. Add 5 μL of 300 nM PGD2 (20X 15 nM final concentration) into each well using Zymark protocol cAMP DP PGD2, and incubate the cells at 37˚ C, 5% $CO_2$ and 95% humidity for additional 15 minutes.
6. Aspirate media from the cells and add 50 μL of lysis buffer 1 using Zymark protocol cAMP DP lysis, and incubate at room temperature with shaking for 30 minutes.
7. Add 150 μL immunoreagent to all wells (a total volume of 200 μL/well).
8. Seal the plates and shake for 2 minutes, put into the chamber of the Wallac microtitre plate μ scintillation counter for 16 hours.

Day 3 Count the amount of [$^{125}$I] cAMP for 2 minutes in 1450 Trilux scintillation counter.

Data Processing

Set up standard curve of cAMP versus CPM.

Table 1. Typical assay data for standard

| cAMP (pmol/mL) | CPM | | Average CPM |
|---|---|---|---|
| 0.2 | 5725 | 5769 | 5530 |
| 0.4 | 5367 | 5259 | 6317 |

(continued)

| cAMP (pmol/mL) | CPM | | Average CPM |
|---|---|---|---|
| 0.8 | 4695 | 4796 | 6507 |
| 1.6 | 4251 | 4178 | 6581 |
| 3.2 | 3434 | 3429 | 6601 |
| 6.4 | 2758 | 2716 | 6711 |
| 12.8 | 2094 | 2054 | 6680 |
| 25.6 | 1531 | 1573 | 6653 |

The cAMP concentrations (pmol/mL) of unknown samples are calculated from a standard curve of cAMP versus CPM. % inhibition is calculated using the following formula:

$$\%\ \text{Inhibition} = \frac{(\text{pmol of control - pmol of sample})\ \text{X}100}{\text{pmol of control (cells + PGD2 only)}}$$

The present invention may be embodied in other specific forms.

## Claims

1. A compound of formula (A),

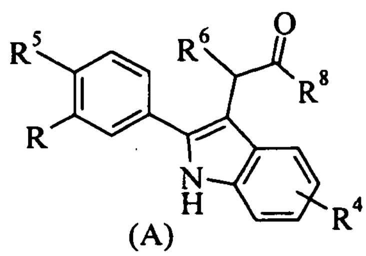

(A)

wherein:

R is $R^1CH_2SO_2$-, $R^2CH_2SO_2NH$-, or $R^3NHSO_2$-;
$R^1$ is phenyl optionally substituted with halo,
$R^2$ is phenyl substituted with halo,
$R^3$ is 2,6-dichloro-benzyl, 3,5-dichloro-benzyl, 2,4-dichloro-phenylethyl, 2-methoxy- phenylethyl, 3-methoxy-phenylethyl, 4-methoxy-phenylethyl, 2-trifluoromethyl- phenylethyl, phenylethyl or 3-phenyl-n-propyl,
$R^4$ is hydrogen,
$R^5$ is chloro,
$R^6$ is hydrogen, and
$R^8$ is hydroxy; or
R is cyclohexylaminosulfonyl,
$R^4$ is 4-chloro, 4-fluoro, 4-methyl or 7-chloro,
$R^5$ is chloro or ethyl,
$R^6$ is hydrogen or methyl, and
$R^8$ is hydroxy; or
R is cyclohexylaminosulfonyl,
$R^4$ is hydrogen,
$R^5$ is chloro,

$R^6$ is hydrogen,
$R^8$ is $-NHR^7$, and
$R^7$ is methyl, methylsulfonyl, ethylsulfonyl, haloalkylsulfonyl or tetrazolyl;

or a pharmaceutically acceptable salt, hydrate, or solvate thereof, a pharmaceutically acceptable ester prodrug thereof, or a pharmaceutically acceptable salt, hydrate or solvate of the ester prodrug.

**2.** The compound according to claim 1, which is a compound of formula (I):

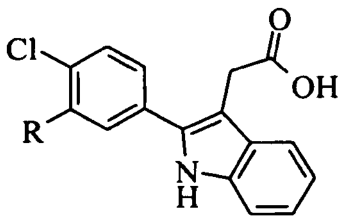

(I)

wherein:

R is $R^1CH_2SO_2$-, $R^2CH_2SO_2NH$-, or $R^3NHSO_2$-;
$R^1$ is phenyl optionally substituted with halo;
$R^2$ is phenyl substituted with halo; and
$R^3$ is 2,6-dichloro-benzyl, 3,5-dichloro-benzyl, 2,4-dichloro-phenylethyl, 2-methoxy- phenylethyl, 3-methoxy-phenylethyl, 4-methoxy-phenylethyl, 2-trifluoromethyl- phenylethyl, phenylethyl or 3-phenyl-n-propyl;

or a pharmaceutically acceptable salt, hydrate, or solvate thereof, a pharmaceutically acceptable ester prodrug thereof, or a pharmaceutically acceptable salt, hydrate or solvate of the ester prodrug.

**3.** The compound according to claim 2, wherein R is $R^3NHSO_2$-, or a pharmaceutically acceptable salt, hydrate, or solvate thereof, a pharmaceutically acceptable ester prodrug thereof, or a pharmaceutically acceptable salt, hydrate or solvate of the ester prodrug.

**4.** The compound according to claim 1, which is a compound of formula (II):

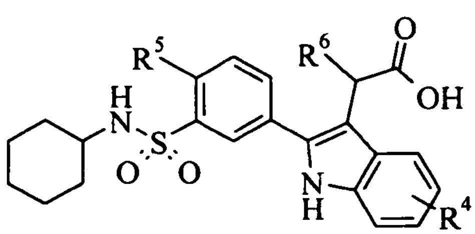

(II)

wherein:

$R^4$ is 4-chloro, 4-fluoro, 4-methyl or 7-chloro;
$R^5$ is chloro or ethyl; and
$R^6$ is hydrogen or methyl;

or a pharmaceutically acceptable salt, hydrate, or solvate thereof, a pharmaceutically acceptable ester prodrug thereof, or a pharmaceutically acceptable salt, hydrate or solvate of the ester prodrug.

**5.** The compound according to claim 4, wherein $R^5$ is chloro and $R^6$ is hydrogen, or a pharmaceutically acceptable

salt, hydrate, or solvate thereof, a pharmaceutically acceptable ester prodrug thereof, or a pharmaceutically acceptable salt, hydrate or solvate of the ester prodrug.

**6.** The compound according to claim 1, which is a compound of formula (III):

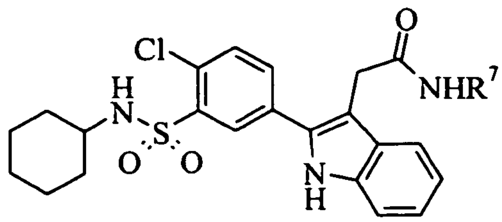

**(III)**

wherein:

$R^7$ is methyl, methylsulfonyl, ethylsulfonyl, haloalkylsulfonyl or tetrazolyl,
or a pharmaceutically acceptable salt, hydrate, or solvate thereof, a pharmaceutically acceptable ester prodrug thereof, or a pharmaceutically acceptable salt, hydrate or solvate of the ester prodrug.

**7.** The compound according to claim 1, which is selected from
{2-[4-Chloro-3-(2,6-dichloro-benzylsulfamoyl)-phenyl]-1H-indol-3-yl}-acetic acid,
{2-[4-Chloro-3-(3,5-dichloro-benzylsulfamoyl)-phenyl]-1H-indol-3-yl}-acetic acid,
(2-{4-Chloro-3-[2-(2,4-dichloro-phenyl)-ethylsulfamoyl]-phenyl}-1H-indol-3-yl)-acetic acid,
(2-{4-Chloro-3-[2-(2-methoxy-phenyl)-ethylsulfamoyl]-phenyl}-1H-indol-3-yl)-acetic acid,
(2-{4-Chloro-3-[2-(3-methoxy-phenyl)-ethylsulfamoyl]-phenyl}-1H-indol-3-yl)-acetic acid,
(2-{4-Chloro-3-[2-(4-methoxy-phenyl)-ethylsulfamoyl]-phenyl}-1H-indol-3-yl)-acetic acid, (2-{4-Chloro-3-[2-(2-trifluoromethoxy-phenyl)-ethylsulfamoyl]-phenyl}-1H-indol-3-yl)-acetic acid,
[2-(4-Chtoro-3-phenethytsutfamoyt-phenyt)-1H-indol-3-yl]-acetic acid,
{2-[4-Chloro-3-(3-phenyl-propylsulfamoyl)-phenyl]-1H-indol-3-yl}-acetic acid,
{2-[4-Chloro-3-(3-chloro-phenylmethanesulfolyl)-phenyl]-1H-indol-3-yl}-acetic acid,
{2-[4-Chloro-3-(3-chloro-phenylmethanesulfonylamino)-phenyl]-1H-indol-3-yl}-acetic acid,
[4-Chloro-2-(4-chloro-3-cyclohexylsulfamoyl-phenyl)-1H-indol-3-yl]-acetic acid,
[2-(4-Chloro-3-cyclohexylsulfamoyl-phenyl)-4-fluoro-1H-indol-3-yl]-acetic acid,
[2-(4-Chloro-3-cyclohexylsulfamoyl-phenyl)-4-methyl-1H-indol-3-yl]-acetic acid,
[7-Chloro-2-(4-chloro-3-cyclohexylsulfamoyl-phenyl)-1H-indol-3-yl]-acetic acid,
[2-(3-cyctohexytsutfamoyl-4-ethyl-phenyl)-1H-mdol-3-yl]-acetic acid,
2-[2-(4-Chloro-3-cyclohexylsulfamoyl-phenyl)-1H-indol-3-yl]-propionic acid,
2-[2-(4-Chloro-3-cyclohexylsulfamoyl-phenyl)-1H-indol-3-yl]-N-methyl-acetamide,
2-Chloro-N-cyclohexyl-5-[3-(2-methanesulfonylamino-2-oxo-ethyl)-1H-indol-2-yl]-benzenesulfonamide,
2-Chloro-N-cyclohexyl-5-[3-(2-ethanesulfonylamino-2-oxo-ethyl)-11H-indol-2-yl]-benzenesulfonamide,
2-Chloro-N-cyclohexyl-5-[3-(2-oxo-2-trifluoromethanesulfonylamino-ethyl)-1H-indol-2-yl]-benzenesulfonamide, or
2-[2-(4-Chloro-3-cyclohexylsulfamoyl-phenyl)-1H-indol-3-yl]-N-(1H-tetrazol-5-yl)-acetamide,
or a pharmaceutically acceptable salt, hydrate, or solvate thereof, a pharmaceutically acceptable ester prodrug thereof, or a pharmaceutically acceptable salt, hydrate or solvate of the ester prodrug.

**8.** The pharmaceutically acceptable salt of the compound according to claim 1 is potassium, [4-chloro-2-(4-chloro-3-cyclohexylsulfamoyl-phenyl)-1H-indol-3-yl]-acetate.

**9.** A pharmaceutical composition comprising a pharmaceutically effective amount of the compound according to claim 1, or a pharmaceutically acceptable salt, hydrate, or solvate thereof, a pharmaceutically acceptable ester prodrug thereof, or a pharmaceutically acceptable salt, hydrate or solvate of the ester prodrug, in admixture with a pharmaceutically acceptable carrier.

**10.** Use of the compound according to claim 1, or a pharmaceutically acceptable salt, hydrate, or solvate thereof, a pharmaceutically acceptable ester prodrug thereof, or a pharmaceutically acceptable salt, hydrate or solvate of the

ester prodrug for the manufacture of a medicament for treating an allergic disease, systemic mastocytosis, a disorder accompanied by systemic mast cell activation, anaphylaxis shock, bronchoconstriction, bronchitis, eczema, a diseases accompanied by itch, a disease which is generated secondarily as a result of behavior accompanied by itch, chronic obstructive pulmonary diseases, ischemic reperfusion injury, cerebrovascular accident, chronic rheumatoid arthritis, pleurisy, or ulcerative colitis.

**11.** Use according to claim 10, wherein the behavior accompanied by itch is scratching or beating.

**12.** Use according to claim 10, wherein the disease which is generated secondarily as a result of behavior accompanied by itch is cataract, retinal detachment, inflammation, infection or sleeping disorder.

**13.** Use according claim 10, wherein the allergic disease is allergic rhinitis, allergic conjunctivitis, atopic dermatitis, bronchial asthma, or food allergy.

**14.** Use according claim 10, wherein the diseases accompanied by itch is atopic dermatitis or urticaria.

**15.** A pharmaceutical composition comprising a pharmaceutically effective amount of a compound according to claim 1, a compound selected from the group consisting of an antihistamine, a leukotriene antagonist, a beta agonist, a PDE4 inhibitor, a TP antagonist and a CrTh2 antagonist, in admixture with a pharmaceutically acceptable carrier.

**16.** The pharmaceutical composition according to claim 15, wherein the antihistamine is fexofenadine, loratadine, desloratadine or cetirizine, the leukotriene antagonist is montelukast or zafirlukast, the beta agonist is albuterol, salbuterol or terbutaline, the PDE4 inhibitor is roflumilast or cilomilast, the TP antagonist is Ramatroban, and the CrTh2 antagonist is Ramatroban.

**17.** A compound according to claim 1, or a pharmaceutically acceptable salt, hydrate, or solvate thereof, a pharmaceutically acceptable ester prodrug thereof, or a pharmaceutically acceptable salt, hydrate or solvate of the ester prodrug for use in treatment of an allergic disease in particular allergic rhinitis, allergic conjunctivitis, atopic dermatitis, bronchial asthma, or food allergy, systemic mastocytosis, a disorder accompanied by systemic mast cell activation, anaphylaxis shock, bronchoconstriction, bronchitis, eczema, a disease accompanied by itch selected from atopic dermatitis or urticaria, a disease which is generated secondarily as a result of behavior accompanied by itch in particular cataract, retinal detachment, inflammation, infection or sleeping disorder, wherein the behavior accompanied by itch is scratching or beating, chronic obstructive pulmonary diseases, ischemic reperfusion injury, cerebrovascular accident, chronic rheumatoid arthritis, pleurisy, or ulcerative colitis.

## Patentansprüche

**1.** Verbindung der Formel (A),

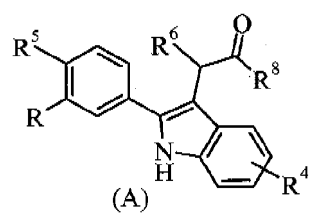

(A)

wobei:

R für $R^1CH_2SO_2$-, $R^2CH_2SO_2NH$- oder $R^3NHSO_2$- steht;
$R^1$ für Phenyl steht, das gegebenenfalls mit Halogen substituiert ist,
$R^2$ für Phenyl steht, das mit Halogen substituiert ist,
$R^3$ für 2,6-Dichlor-benzyl, 3,5-Dichlor-benzyl, 2,4-Dichlor-phenylethyl, 2-Methoxy-phenyl- ethyl, 3-Methoxy-phenylethyl, 4-Methoxy- phenylethyl, 2-Trifluormethyl-phenylethyl, Phenylethyl oder 3-Phenyl-n-propyl steht,
$R^4$ für Wasserstoff steht,
$R^5$ für Chlor steht,

$R^6$ für Wasserstoff steht, und
$R^8$ für Hydroxy steht; oder
R für Cyclohexylaminosulfonyl steht,
$R^4$ für 4-Chlor, 4-fluor, 4-Methyl oder 7-Chlor steht,
$R^5$ für Chlor oder Ethyl steht,
$R^6$ für Wasserstoff oder Methyl steht, und
$R^8$ für Hydroxy steht; oder
R für Cyclohexylaminosulfonyl steht,
$R^4$ für Wasserstoff steht;
$R^5$ für Chlor steht,
$R^6$ für Wasserstoff steht,
$R^8$ für $-NHR^7$ steht, und
$R^7$ für Methyl, Methylsulfonyl, Ethylsulfonyl, Halogenalkylsulfonyl oder Tetrazolyl steht;

oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**2.** Verbindung nach Anspruch 1, die eine Verbindung der Formel (I) ist:

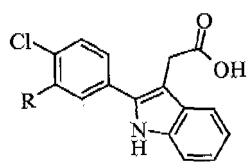

(I)

wobei:

R für $R^1CH_2SO_2$-, $R^2CH_2SO_2NH$- oder $R^3NHSO_2$- steht;
$R^1$ für Phenyl steht, das gegebenenfalls mit Halogen substituiert ist,
$R^2$ für Phenyl steht, das mit Halogen substituiert ist; und
$R^3$ für 2,6-Dichlor-benzyl, 3,5-Dichlor-benzyl, 2,4-Dichlor-phenylethyl, 2-Methoxy-phenyl- ethyl, 3-Methoxy-phenylethyl, 4-Methoxy- phenylethyl, 2-Trifluormethyl-phenylethyl, Phenylethyl oder 3-Phenyl-n-propyl steht;

oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**3.** Verbindung nach Anspruch 2, wobei R für $R^3NHSO_2$-steht, oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**4.** Verbindung nach Anspruch 1, die eine Verbindung der Formel (II) ist:

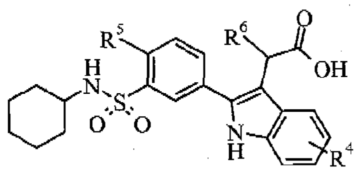

(II)

wobei:

$R^4$ für 4-Chlor, 4-Fluor, 4-Methyl oder 7-Chlor steht,
$R^5$ für Chlor oder Ethyl steht, und
$R^6$ für Wasserstoff oder Methyl steht;

oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**5.** Verbindung nach Anspruch 4, wobei $R^5$ für Chlor und $R^6$ für Wasserstoff steht, oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**6.** Verbindung nach Anspruch 1, die eine Verbindung der Formel (III) ist:

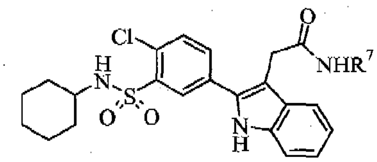

(III)

wobei:

$R^7$ für Methyl, Methylsulfonyl, Ethylsulfonyl, Halogenalkylsulfonyl oder Tetrazolyl steht;

oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester.

**7.** Verbindung nach Anspruch 1, die ausgewählt ist aus
{2-[4-Chlor-3-(2,6-dichlor-benzylsulfamoyl)-phenyl]-1H-indol-3-yl}-essigsäure,
{2-[4-Chlor-3-(3,5-dichlor-benzylsulfamoyl)-phenyl]-1H-indol-3-yl}-essigsäure,
(2-{4-Chlor-3-[2-(2,4-dichlor-phenyl)-ethylsulfamoyl]-phenyl}-1H-indol-3-yl)-essigsäure,
(2-{4-Chlor-3-[2-(2-methoxy-phenyl)-ethylsulfamoyl]-phenyl}-1H-indol-3-yl)-essigsäure,
(2-{4-Chlor-3-[2-(3-methoxy-phenyl)-ethylsulfamoyl]-phenyl}-1H-indol-3-yl)-essigsäure,
(2-{4-Chlor-3-[2-(4-methoxy-phenyl)-ethylsulfamoyl]-phenyl}-1H-indol-3-y1)-essigsäure,
(2-{4-Chlor-3-[2-(2-trifluormethoxy-phenyl)-ethylsulfamoyl]-phenyl}-1H-indcl-3-yl)-essigsäure,
[2-(4-Chlor-3-phenethylsulfamoyl-phenyl)-1H-indol-3-yl]-essigsäure,
{2-[4-Chlor-3-(3-phenyl-propylsulfamoyl)-phenyl]-1H-indol-3-yl}-essigsäure,
{2-[4-Chlor-3-(3-chlor-phenylmethansulfonyl)-phenyl]-1H-indol-3-yl}-essigsäure,
{2-[4-Chlor-3-(3-chlor-phenylmethansulfonylamino)-phanyl]-1H-indol-3-yl}-essigsäure,
[4-Chlor-2-(4-chlor-3-cyclohemylsulfamoyl-phenyl)-1H-indol-3-yl]-essigsäure,
[2-(4-Chlor-3-cyclohexylsulfamoyl-phenyl)-4-fluor-1H-indol-3-yl]-essigsäure,
[2-(4-Chlor-3-cyclohexylsulfamoyl-phenyl)-4-methyl-1H-indol-3-yl]-essigsäure,
[7-Chlor-2-(4-chlor-3-cyclohexylsulfamoyl-phenyl)-1H-indol-3-yl]-essigsäure,
[2-(3-Cyclohexylsulfamoyl-4-ethyl-phenyl)-1H-indol-3-yl]-essigsäure,
2-[2-(4-chlor-3-cyclohexylsulfamoyl-phenyl)-1H-indol-3-yl]-propionsäure,
2-[2-(4-Chlor-3-cyclohexylsulfamoyl-phenyl)-1H-indol-3-yl]-N-methyl-acetamid,
2-Chlor-N-cyclohexyl-5-[3-(2-methansulfonylamino-2-oxo-ethyl)-1H-indol-2-yl]-benzolsulfonamid,
2-Chlor-N-cyclohexyl-5-[3-(2-ethansulfonylamino-2-oxo-ethyl)-1H-indol-2-yl]-benzolsulfonamid,
2-Chlor-N-cyclohexyl-5-[3-(2-cxo-2-trifluormethansulfonylamino-ethyl)-1H-indol-2-yl]-benzolsulfonamid, oder
2-[2-(4-Chlor-3-cyclohexylsulfamoyl-phenyl)-1H-indol-3-yl]-N-(1H-tetrazol-5-yl)-acetamid;
oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat von dem Prodrug-Ester,

**8.** Pharmazeutisch verträgliches Salz der Verbindung nach Anspruch 1 ist Kalium[4-chlor-2-(4-Chlor-3-cyclohexylsulfamoyl-phenyl)-1H-indol-3-yl]-acetat.

**9.** Pharmazeutische Zusammensetzung, die eine pharmazeutisch wirksame Menge von der Verbindung nach Anspruch 1 oder ein pharmazeutisch verträgliches Salz, Hydrat oder Solvat davon, einen pharmazeutisch verträglichen Prodrug-Ester davon oder ein pharmazeutisch verträgliches Salz, Hydrat oder Solvat von dem Prodrug-Ester im Gemisch mit einem pharmazeutisch verträglichen Träger umfasst.

**10.** Verwendung der Verbindung nach Anspruch 1 oder eines pharmazeutisch verträglichen Salzes, Hydrates oder Solvates davon, eines pharmazeutisch verträglichen, Prodrug-Esters davon oder eines pharmazeutisch verträgliches Salzes, Hydrates oder Solvates von dem Prodrug-Ester für die Herstellung von einem Medikament zur Behandlung von einer allergischen Erkrankung, einer systemischen Mastozytose, einer Erkrankung, die durch eine systemische Aktivierung von Mastzellen begleitet ist, von anaphylaktischem Schock, Bronchokonstriktion, Bronchitis, Hautausschlag, von einer Krankheit, die mit Juckreiz einhergeht, von einer Erkrankung, die sekundär als Folge des Verhaltens entsteht, das mit Juckreiz einhergeht, von chronisch obstruktiven Lungenerkrankungen, von ischämischen Reperfusionsschäden, Schlaganfall, chronischer rheumatoider Arthritis, Brustfellentzündung oder von chronisch-entzündlichen Darmerkrankungen.

**11.** Verwendung nach Anspruch 10, wobei das Verhalten, das mit Juckreiz einhergeht, Kratzen oder Schlagen ist.

**12.** Verwendung nach Anspruch 10, wobei die Erkrankung, die sekundär als eine Folge des Verhaltens, das mit Juckreiz einhergeht, entsteht, Katarakt, Netzhautablösung, Entzündung, Infektion oder Schlafstörung ist.

**13.** Verwendung nach Anspruch 10, wobei die allergische Erkrankung ein allergischer Schnupfen, allergische Bindehautentzündung, atopische Dermatitis, Bronchialasthma oder Nahrungsmittelallergie ist.

**14.** Verwendung nach Anspruch 10, wobei die Erkrankungen, die mit Juckreiz einhergehen, atopische Dermatitis oder Nesselsucht sind.

**15.** Pharmazeutische Zusammensetzung, die eine pharmazeutisch wirksame Menge von einer Verbindung nach Anspruch 1, eine Verbindung, die ausgewählt ist aus der Gruppe, die aus einem Antihistaminikum, einem Leukotrien-Antagonisten, einem Beta-Agonisten, einem PDE4-Inhibitor, einem TP-Antagonisten und einem CrTh2-Antagonisten besteht, im Gemisch mit einem pharmazeutisch verträglichen Träger umfasst.

**16.** Pharmazeutische Zusammensetzung nach Anspruch 15, wobei das Antihistaminikum Fexofenadin, Loratadin, Desloratadin oder Cetirizin ist, der Leukotrien-Antagonist Montelukast oder Zafirlukast ist, der Beta-Agonist Albuterol, Salbuterol oder Terbutalin ist, der PDE4-Inhibitor Roflumilast oder Cilomilast ist, der TP-Antagonist Ramatroban ist und der CrTh2-Antagonist Ramatroban ist.

**17.** Verbindung nach Anspruch 1 oder ein pharmazeutisch verträgliches Salz, Hydrat oder Solvat davon, ein pharmazeutisch verträglicher Prodrug-Ester davon oder ein pharmazeutisch verträgliches Salz, Hydrat oder Solvat des Prodrug-Esters zur Verwendung für die Behandlung von einer allergischen Erkrankung, insbesondere von allergischem Schnupfen, allergischer Bindehautentzündung, von atopischer Dermatitis, von Bronchialasthma oder Nahrungsmittelallergie, systemischer Mastozytose, einer Erkrankung, die durch eine systemische Aktivierung von Mastzellen begleitet ist, von anaphylaktischem Schock, Bronchokonstriktion, Bronchitis, Hautausschlag, von einer Krankheit, die mit Juckreiz einhergeht, ausgewählt aus atopischer Dermatitis oder Nesselsucht, einer Erkrankung, die sekundär als Folge des Verhaltens entsteht, das mit Juckreiz einhergeht, insbesondere Katarakt, Netzhautablösung, Entzündung, Infektion oder Schlafstörung, wobei das Verhalten, das mit Juckreiz einhergeht, Kratzen oder Schlagen ist, von chronisch obstruktiven Lungenerkrankungen, von ischämischen Reperfusionsschäden, Schlaganfall, chronischer rheumatoider Arthritis, von Brustfellentzündung oder von chronisch-entzündlichen Darmerkrankungen.

## Revendications

**1.** Composé de formule (A),

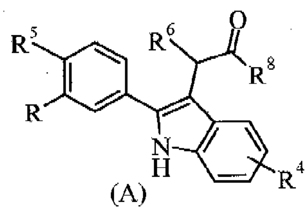

(A)

dans laquelle :

R est $R^1CH_2SO_2$-, $R^2CH_2SO_2NH$- ou $R^3NHSO_2$- ;
$R^1$ est phényle éventuellement substitué par halogéno,
$R^2$ est phényle substitué par halogéno,
$R^3$ est 2,6-dichloro-benzyle, 3,5-dichloro- benzyle, 2,4-dichloro-phényléthyle, 2- méthoxy-phényléthyle, 3-méthoxy- phényléthyle, 4-méthoxy-phényléthyle, 2- trifluorométhyl-phényléthyle, phényléthyle ou 3-phényl-n-propyle,
$R^4$ est hydrogène,
$R^5$ est chloro,
$R^6$ est hydrogène, et
$R^8$ est hydroxy ; ou
R est cyclohexylaminosulfonyle,
$R^4$ est 4-chloro, 4-fluoro, 4-méthyle ou 7-chloro,
$R^5$ est chloro ou éthyle,
$R^6$ est hydrogène ou méthyle, et
$R^8$ est hydroxy ; ou
R est cyclohexylaminosulfonyle,
$R^4$ est hydrogène,
$R^5$ est chloro,
$R^6$ est hydrogène,
$R^8$ est $-NHR^7$, et
$R^7$ est méthyle, méthylsulfonyle, éthylsulfonyle, halogénoalkylsulfonyle ou tétrazolyle ;

ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**2.** Composé selon la revendication 1, **caractérisé en ce qu'**il s'agit d'un composé de formulé (I) :

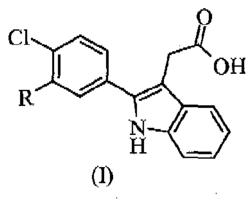

(I)

dans laquelle :

R est $R^1CH_2SO_2$-; $R^2CH_2SO_2NH$- ou $R^3NHSO_2$- ;
$R^1$ est phényle éventuellement substitué par halogéno ;
$R^2$ est phényle substitué par halogéno ; et
$R^3$ est 2,6-dichloro-benzyle, 3,5-dichloro-benzyle, 2,4-dichloro-phényléthyle, 2-méthoxy- phényléthyle, 3-méthoxy-phényléthyle, 4- méthoxy-phényléthyle, 2-trifluorométhyl- phényléthyle, phényléthyle ou 3-phényl-n-propyle ;

ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**3.** Composé selon la revendication 2, **caractérisé en ce que** R est $R^3NHSO_2$-, ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**4.** Composé selon la revendication 1, **caractérisé en ce qu'**il s'agit d'un composé de formule (II) :

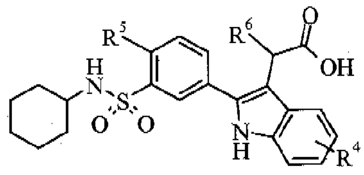

(II)

dans laquelle :

$R^4$ est 4-chloro, 4-fluoro, 4-méthyle ou 7-chloro,
$R^5$ est chloro ou éthyle ; et
$R^6$ est hydrogène ou méthyle ;

ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**5.** Composé selon la revendication 4, **caractérisé en ce que** $R^5$ est chloro et $R^6$ est hydrogène, ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**6.** Composé selon la revendication 1, **caractérisé en ce qu'**il s'agit d'un composé de formule (III) :

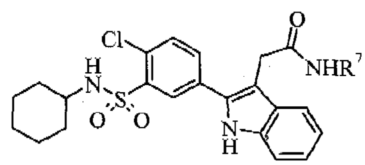

(III)

dans laquelle :

$R^7$ est méthyle, méthylsulfonyle, éthylsulfonyle, halogénoalkylsulfonyle ou tétrazolyle ;

ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester.

**7.** Composé selon la revendication 1, **caractérisé en ce qu'**il est choisi parmi
l'acide {2-[4-chloro-3-(2,6-dichloro-benzylsulfamoyl)-phényl]-1H-indol-3-yl}-acétique,
l'acide {2-[4-chloro-3-(3,5-dichloro-benzylsulfamoyl)-phényl]-1H-indol-3-yl}-acétique,
l'acide (2-{4-chloro-3-[2-(2,4-dichloro-phényl)-éthylsulfamoyl]-phényl}-1H-indol-3-yl)-acétique,
l'acide (2-{4-chloro-3-[2-(2-méthoxy-phényl)-éthylsulfamoyl]-phényl}-1H-indol-3-yl)-acétique,
l'acide (2-{4-chloro-3-[2-(3-méthoxy-phényl)-éthylsulfamoyl]-phényl}-1H-indol-3-yl)-acétique,
l'acide (2-{4-chloro-3-[2-(4-méthoxy-phényl)-éthylsulfamoyl]-phënyl}-1H-indol-3-yl)-acétique,
l'acide (2-{4-chloro-3-[2-(2-trifluorométhoxy-phényl)-éthylsulfamoyl]-phényl}-1H-indol-3-yl)-acétique,
l'acide [2-(4-chloro-3-phénéthylsulfamoyl-phényl)-1H-indol-3-yl]-acétique,
l'acide {2-[4-chloro-3-(3-phényl-pronylsulfamoyl)-phényl]-1H-indol-3-yl}-acétique,
l'acide {2-[4-chloro-3-(3-chloro-phénylméthanesulfonyl)-phényl]-1H-indol-3-yl}-acétique,
l'acide {2-[4-chloro-3-(3-chloro-phénylméthanesulfonylamino)-phényl]-1H-indol-3-yl}-acétique,
l'acide [4-chloro-2-(4-chloro-3-cyclohexylsulfamoyl-phényl)-1H-indol-3-yl]-acétique,
l'acide [2-(4-chloro-3-cyclohexylsulfamoyl-phényl)-4-fluoro-1H-indol-3-yl]-acétique,
l'acide [2-(4-chloro-3-cyclohexylsulfamoyl-phényl)-4-méthyl-1H-indol-3-yl]-acétique,
l'acide [7-chloro-2-(4-chloro-3-cyclohexylsulfamoyl-phényl)-1H-indol-3-yl]-acétique,
l'acide [2-(3-cyclohexylsulfamoyl-4-éthyl-phényl)-1H-indol-3-yl]-acétique,
l'acide 2-[2-(4-chloro-3-cyclohexylsulfamoyl-phényl)-1H-indol-3-yl]-propionique,
le 2-[2-(4-chloro-3-cyclohexylsulfamoyl-phényl)-1H-indol-3-yl]-N-méthyl-acétamide,

le 2-chloro-N-cyclohexyl-5-[3-(2-méthanesulfonylamino-2-oxo-éthyl)-1H-indol-2-yl]-benzènesulfonamide,
le 2-chloro-N-cyclohexyl-5-[3-(2-éthanesulfonylaminc-2-oxo-éthyl)-1H-indol-2-yl]-benzènesulfonamide,
le 2-chloro-N-cyclohexyl-5-[3-(2-oxo-2-trifluorométhanesulfonylamino-éthyl)-1H-indol-2-yl]-benzènesulfonamide, ou
le 2-[2-(4-chloro-3-cyclohexylsulfamoyl-phényl)-1H-indol-3-yl]-N-(1H-tétrazol-5-yl)-acétamide ;
ou un sel, hydrate ou solvate pharmaceutiquement acceptable de ceux-ci, une prodrogue ester pharmaceutiquement acceptable de ceux-ci, ou un sel, hydrate ou solvate pharmaceutiquement acceptable de la prodrogue ester.

**8.** Sel pharmaceutiquement acceptable du composé selon la revendication 1, **caractérisé en ce qu'**il s'agit du [4-chloro-2-(4-chloro-3-cyclohexylsulfamoyl-phényl)-1H-indol-3-yl]-acétate de potassium.

**9.** Composition pharmaceutique comprenant une quantité pharmaceutiquement efficace du composé selon la revendication 1, ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester, en mélange avec un support pharmaceutiquement acceptable.

**10.** Utilisation du composé selon la revendication 1, ou d'un sel, hydrate ou solvate pharmaceutiquement acceptable de celui-ci, d'une prodrogue ester pharmaceutiquement acceptable de celui-ci, ou d'un sel, hydrate ou solvate pharmaceutiquement acceptable de la prodrogue ester, pour la fabrication d'un médicament destiné au traitement d'une maladie allergique, d'une mastocytose systémique, d'un trouble accompagné de l'activation systémique de cellules mastocytaires, du choc anaphylactique, de la bronchoconstriction, de la bronchite, de l'eczéma, d'une maladie accompagnée de démangeaison, d'une maladie qui est provoquée de façon secondaire en conséquence d'un comportement accompagné de démangeaison, des bronchopneumopathies chroniques obstructives, d'une lésion d'ischémie-repérfusion, de l'accident cérébrovasculaire, de la polyarthrite rhumatoïde chronique, de la pleurésie ou de la colite ulcéreuse.

**11.** Utilisation selon la revendication 10, **caractérisée en ce que** le comportement accompagné de démangeaison est l'égratignure ou les coups.

**12.** Utilisation selon la revendication 10, **caractérisée en ce que** la maladie qui est provoquée de façon secondaire en conséquence d'un comportement accompagné de démangeaison est la cataracte, le décollement rétinien, l'inflammation, l'infection ou le trouble du sommeil.

**13.** Utilisation selon la revendication 10, **caractérisée en ce que** la maladie allergique est la rhinite allergique, la conjonctivite allergique, la dermatite atopique, l'asthme bronchique ou l'allergie alimentaire.

**14.** Utilisation selon la revendication 10, **caractérisée en ce que** la maladie accompagnée de démangeaison est la dermatite atopique ou l'urticaire.

**15.** Composition pharmaceutique comprenant une quantité pharmaceutiquement efficace d'un composé selon la revendication 1, d'un composé choisi dans le groupe constitué d'un antihistaminique, d'un antagoniste de leucotriène, d'un bêta-agoniste, d'un inhibiteur de PDE4, d'un antagoniste de TP et d'un antagoniste de CrTh2, en mélange avec un support pharmaceutiquement acceptable.

**16.** Composition pharmaceutique selon la revendication 15, **caractérisée en se que** l'antihistaminique est la fexofénadine, la loratadine, la desloratadine ou la cétirizine, l'antagoniste de leucotriène est le montélukast ou le zafirlukast, le bêta-agoniste est l'albutérol, le salbutérol ou la terbutaline, l'inhibiteur de PDE4 est le roflumilast ou le cilomilast, l'antagoniste de TP est le ramatroban, et l'antagoniste de CrTh2 est le ramatroban.

**17.** Composé selon la revendication 1, ou un sel, hydrate ou solvate pharmaceutiquement acceptable de celui-ci, une prodrogue ester pharmaceutiquement acceptable de celui-ci, ou un sel, hydrate ou solvate pharmaceutiquement acceptable de la prodrogue ester, destiné à être utilisé dans le traitement d'une maladie allergique, en particulier de la rhinite allergique, de la conjonctivite allergique, de la dermatite atopique, de l'asthme bronchique, d'une allergie alimentaire, d'une mastocytose systémique, d'un trouble accompagné de l'activation systémique de cellules mastocytaires, du choc anaphylactique, de la bronchoconstriction, de la bronchite, de l'eczéma, d'une maladie accompagnée de démangeaison choisie parmi la dermatite atopique ou l'urticaire, d'une maladie qui est provoquée de façon secondaire en conséquence d'un comportement accompagné de démangeaison, en particulier la cataracte, le décollement rétinien, l'inflammation, l'infection ou le trouble du sommeil, le comportement accompagné de démangeaison étant l'égratignure ou les coups, des bronchopneumopathies chroniques obstructives, d'une lésion d'ischémie-repérfusion, d'un accident cérébrovasculaire, de la polyarthrite rhumatoïde chronique, de la pleurésie, ou de la colite ulcéreuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006081343 A
- WO 0178697 A2


### Non-patent literature cited in the description


- **Lewis, RA ; Soter NA ; Diamond PT ; Austen KF ; Oates JA ; Roberts LJ II.** prostaglandin D2 generation after activation of rat and human mast cells with anti-IgE. *J.Immunol.,* 1982, vol. 129, 1627-1631
- **Brightling CE ; Bradding P ; Pavord ID ; Wardlaw AJ.** New Insights into the role of the mast cell in asthma. *Clin Exp Allergy,* 2003, vol. 33, 550-556
- **Holgate S ; Lackie P ; Wilson S ; Roche W ; Davies D.** Bronchial Epithelium as a Key Regulator of Airway Allergen Sensitzation and Remodeling in Asthma. *Am J Respir Crit Care Med.,* 2000, vol. 162, 113-117
- **Matsuoka T ; Hirata M ; Tanaka H ; Takahashi Y ; Murata T ; Kabashima K ; Sugimoto Y ; Kobayashi T ; Ushikubi F ; Aze Y.** Prostaglandin D2 as a mediator of allergic asthma. *Science,* 2000, vol. 287, 2013-2017
- **Doyle WJ ; Bochm S ; Skoner DP.** Physiologic responses to intranasal dose-response challenges with histamine, methacholine, bradykinin, and prostaglandin in adult volunteers with and without nasal allergy. *J Allergy Clin Immunol.,* 1990, vol. 86, 924-35
- **Arimura A ; Yasui K ; Kishino J ; Asanuma F ; Hasegawa H ; Kakudo S ; Ohtani M ; Arita H.** Prevention of allergic inflammation by a novel prostaglandin receptor antagonist, S-5751. *J Pharmacol Exp Ther.,* 2001, vol. 298 (2), 411-9
- **Jones, T. R. ; Savoie, C. ; Robichaud, A. ; Sturino, C. ; Scheigetz, J. ; Lachance, N. ; Roy, B. ; Boyd, M. ; Abraham, W.** Studies with a DP receptor antagonist in sheep and guinea pig models of allergic rhinitis. *Am. J. Resp. Crit. Care Med.,* 2003, vol. 167, A218
- **Torisu K ; Kobayashi K ; Iwahashi M ; Nakai Y ; Onoda T ; Nagase T ; Sugimoto I ; Okada Y ; Matsumoto R ; Nanbu F.** Discovery of a new class of potent, selective, and orally active prostaglandin D2 receptor antagonists. *Bioorg. & Med Chem.,* 2004, vol. 12, 5361-5378
- Design of Prodrugs. Elsevier, 1985
- Methods in Enzymology. Academic Press, 1985, vol. 42, 309-396
- A Textbook of Drug Design and Development
- Design and Applications of Prodrugs. 1991, 113-191
- **H. Bundgaard.** *Advanced Drug Delivery Reviews,* 1992, vol. 8, 1-38
- *J. Pharm. Sci.,* 1988, vol. 77, 285
- **N. Nakeya et al.** *Chem. Pharm. Bull.,* 1984, vol. 32, 692
- **T. Higuchi ; V. Stella.** Pro-drugs as Novel Delivery Systems. A.C.S. Symposium Series, vol. 14
- Bioreversible Carriers in Drug Design. American Pharmaceutical Association and Pergamon Press, 1987
- *J. Med. Chem.,* 2004, vol. 47 (10), 1-12
- **S.M. Berge et al.** Pharmaceutical Salts. *J. Pharm. Sci.,* 1977, vol. 66, 1-19
- Remington's Pharmaceutical Sciences. Mack Publishing Co,
- **R.C. Larock.** Comprehensive Organic Transformations. VCH publishers, 1989
- **Wright DH ; Ford-Hutchinson AW ; Chadee K ; Metters KM.** The human prostanoid DP receptor stimulates mucin secretion in LS174T cells. *Br J Pharmacol.,* 2000, vol. 131 (8), 1537-45